# EUROPÄISCHE PATENTSCHRIFT

(11) **EP 0 564 928 B1**
(45) Veröffentlichungstag und Bekanntmachung des Hinweises auf die Patenterteilung: **15.01.1997**
(21) Anmeldenummer: 93105072.8
(22) Anmeldetag: 27.03.1993
(51) Int. Cl.: C07C 251/48, C07C 69/738, C07C 251/40, C07C 251/80, C07C 233/11, C07D 295/192, C07C 255/57, C07C 235/34, C07D 295/18, A01N 37/42, A01N 37/50

(54) **Derivate der Phenylessigsäure, ihre Herstellung und Verwendung als Schädlingsbekämpfungsmittel**
Phenylacetic acid derivatives, their preparation and use as pesticides
Dérivés de l'acide phénylacétique, leur préparation et leur utilisation comme pesticides

(30) Priorität: 04.04.1992 DE 4211384
(43) Veröffentlichungstag der Anmeldung: 13.10.1993
(73) Patentinhaber: BASF Aktiengesellschaft, 67063 Ludwigshafen (DE)
(72) Erfinder: Doetzer, Reinhard, Dr., W-8500 Nuernberg (DE); Sauter, Hubert, Dr., W-6800 Mannheim 1 (DE); Wingert, Horst, Dr., W-6800 Mannheim 1 (DE); Kirstgen, Reinhard, Dr., W-6730 Neustadt (DE); Harreus, Albrecht, Dr., W-6700 Ludwigshafen (DE); Lorenz, Gisela, Dr., W-6730 Neustadt (DE); Ammermann, Eberhard, Dr., W-6148 Heppenheim (DE)

(56) Entgegenhaltungen:
- EP-A- 0 253 213
- EP-A- 0 254 426
- EP-A- 0 354 571
- EP-A- 0 463 513

## Beschreibung

Die vorliegende Erfindung betrifft neue Phenylessigsäurederivate mit fungizider, insektizider und akarizider Wirkung, diese Verbindungen enthaltende Fungizide und Verfahren zur Bekämpfung von Pilzen.

Es ist bekannt (z.B. aus EP-A 178 826, EP-A 244 077, EP-A 253 213 und EP-A 280 185), Ester der Acrylsäure, Crotonsäure oder Methoxyiminoessigsäure als Fungizide zu verwenden. Die Wirkung dieser Verbindungen ist jedoch in vielen Fällen unbefriedigend.

Es wurde nun gefunden, daß neue Phenylessigsäurederivate der allgemeinen Formel I eine ausgezeichnete fungizide Aktivität aufweisen und darüber hinaus insektizid und akarizid wirksam sind.

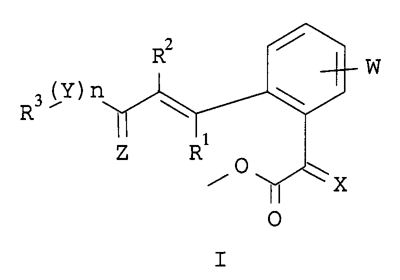

In Formel I haben der Index und die variablen Substituenten folgende Bedeutungen:
- n: bedeutet 0 oder 1,
- W: kann jede beliebige freie Position am Benzolring einnehmen und bedeutet
Wasserstoff,
Halogen wie Fluor, Chlor, Brom oder Jod,
gegebenenfalls substituiertes C₁-C₄-Alkyl wie Methyl, Ethyl, n-Propyl, i-Propyl, n-Butyl, i-Butyl, s-Butyl oder t-Butyl
gegebenenfalls substituiertes C₁-C₄-Alkoxy wie Methoxy, Ethoxy, n-Propyloxy, i-Propyloxy, n-Butyloxy, i-Butyloxy, s-Butyloxy und t-Butyloxy;
bevorzugt bedeutet W Wasserstoff, Chlor, Methyl oder Methoxy, besonders bevorzugt Wasserstoff
- X: bedeutet eine Gruppe CHOCH₃, NOCH₃, CHCH₃ oder CHC₂H₅
- Y: bedeutet Sauerstoff, Schwefel oder eine Gruppe NR⁴, -O-NR⁴-, -NR-⁴-O- oder NR⁴-NR⁵, bevorzugt Sauerstoff, Schwefel oder NR⁴, besonders bevorzugt Sauerstoff oder NR⁴
- Z: bedeutet Sauerstoff, eine Gruppe NR⁶, NOR⁷ oder N-NR⁸R⁹ sowie falls
n = 1 bedeutet, auch Schwefel, bevorzugt Sauerstoff oder NOR⁷ oder
N-NR⁸R⁹, besonders bevorzugt Sauerstoff oder NOR⁷
- R¹: bedeutet Wasserstoff oder gegebenenfalls substituiertes C₁-C₄- Alkyl wie vorstehend genannt, bevorzugt Wasserstoff oder Methyl, besonders bevorzugt Wasserstoff
- R²: bedeutet Wasserstoff,
gegebenenfalls substituiertes C₁-C₄-Alkyl wie vorstehend genannt,
gegebenenfalls substituiertes C₃-C₆-Cycloalkyl wie Cyclopropyl, Cyclobutyl, Cyclopentyl und Cyclohexyl, gegebenenfalls substituiertes und/oder kondensiertes Aryl wie Phenyl, Naphthyl, Anthryl und Phenanthryl, gegebenenfalls substituiertes und/oder kondensiertes Heteroaryl wie 2-Pyridyl, 3-Pyridyl, 4-Pyridyl, 2-Chinolinyl, 1-Isochinolinyl, Pyrazinyl, 2-Pyrimidinyl, 4-Pyrimidinyl, 2-Chinoxalinyl, 1-Pyrrolyl, 2-Pyrrolyl, 3-Pyrrolyl, 2-Furyl, 3-Thienyl, 3-Pyrazolyl, 5-Pyrazolyl, 1-Imidazolyl, 4-Imidazolyl, 1,2,4-Triazol-3-yl, 2-Oxazolyl, 4-Oxazolyl, 5-Oxazolyl, 3-Isoxazolyl, 2-Thiazolyl, 4-Isothiazolyl, 1,3,4-Oxadiazolyl, 1,3,4-Thiadiazolyl, 2-Indolyl, 3-Indolyl, 2-Benzothiazolyl, 9-Carbazolyl und 2-Pteridinyl
C₁-C₄-Perfluoroalkyl wie Trifluormethyl, Pentafluorethyl, Heptafluoro-i-Propyl oder Nonafluoro-n-butyl, oder eine Gruppe CO-V und ist von R¹ unabhängig.

Bevorzugt bedeutet R² Wasserstoff, C₁-C₄-Alkyl, oder eine Gruppe CO-V, besonders bevorzugt Wasserstoff.
- R³: bedeutet Wasserstoff,
gegebenenfalls substituiertes C₁-C₄Alkyl wie vorstehend genannt,
gegebenenfalls substituiertes C₂-C₄-Alkenyl wie Ethenyl, Propen-1-yl, Propen-2-yl, Propen-3-yl, 2-Buten-1-yl oder 2-Methylpropen-1-yl,
gegebenenfalls substituiertes C₃-C₆-Cycloalkyl wie vorstehend genannt,
gegebenenfalls substituiertes 3- bis 6-gliedriges Heterocyclyl wie Oxiranyl, Azetidinyl, Tetrahydrofuran-2-yl, Pyrrolidin-1-yl oder Thiopyran-4-yl,
gegebenenfalls substituiertes und/oder kondensiertes Aryl wie vorstehend genannt,
gegebenenfalls substituiertes und/oder kondensiertes Heteroaryl wie vorstehend genannt,
gegebenenfalls substituiertes Aryl-C₁-C₄-Alkyl wie Benzyl, 1-Phenylethyl, 2-Phenylethyl, 4-Phenyl-n-butyl,
2-Phenyl-2-Methylprop-1-yl und Naphthylmethyl,
gegebenenfalls substituiertes Heteroaryl-C₁-C₄-Alkyl wie (2-Pyridyl)methyl, 2-(3-Furyl)-ethyl, 3-(5-Isoxazolyl)propyl und 1-Indolylmethyl
gegebenenfalls substituiertes Aryl-C₂-C₄-Alkenyl wie 1-Phenylethenyl und 1-(3-Naphthyl)-1-propen-3-yl, oder gegebenenfalls substituiertes Heteroaryl-C₂-C₄-Alkenyl wie 2-(4-Pyridyl)-ethenyl oder 1-(3-Thienyl)-ethenyl,
sowie, falls n = 0 bedeutet auch Cyano, C₁-C₄-Perfluoroalkyl und
gegebenenfalls substituiertes C₂-C₄-Alkinyl wie Ethinyl, Propin-1-yl, Propin-3-yl, 1-Butin-4-yl und 2-Butin-1-yl und falls n = 1 bedeutet und wenn Y Sauerstoff bedeutet, auch gegebenenfalls substituiertes Trialkylsilyl wie Trimethylsilyl, Triethylsilyl, tert.-Butyl-dimethylsilyl und Di-tert.-butyl-methylsilyl

Bevorzugt bedeutet R³ Wasserstoff, gegebenenfalls substituiertes C₁-C₄-Alkyl, gegebenenfalls substituiertes und/oder kondensiertes Aryl, gegebenenfalls substituiertes und/oder kondensiertes Heteroaryl oder gegebenenfalls substituiertes Aryl-C₁-C₄-Alkyl,
besonders bevorzugt Wasserstoff, C₁-C₄-Alkyl, gegebenenfalls substituiertes Phenyl und gegebenenfalls substituiertes Benzyl
- R⁴ und R⁵: bedeuten Wasserstoff,
gegebenenfalls substituiertes C₁-C₄-Alkyl wie vorstehend genannt,
gegebenenfalls substituiertes C₃-C₆-Cycloalkyl wie vorstehend genannt,
und sind unabhängig voneinander;
bevorzugt bedeuten R⁴ und R⁵ Wasserstoff, Methyl oder Ethyl
- R⁶: bedeutet gegebenenfalls substituiertes C₁-C₄-Alkyl wie vorstehend genannt, oder
gegebenenfalls substituiertes und/oder kondensiertes Aryl oder Heteroaryl wie vorstehend genannt;
bevorzugt bedeutet R⁶ substituiertes Phenyl
- R⁷ und R⁸: bedeuten Wasserstoff,
gegebenenfalls substituiertes C₁-C₄-Alkyl wie vorstehend genannt,
gegebenenfalls substituiertes C₂-C₄-Alkenyl wie vorstehend genannt,
gegebenenfalls substituiertes C₂-C₄-Alkinyl wie vorstehend genannt,
gegebenenfalls substituiertes C₃-C₆-Cycloalkyl wie vorstehend genannt,
gegebenenfalls substituiertes und/oder kondensiertes Aryl wie vorstehend genannt,
gegebenenfalls substituiertes und/oder kondensiertes Heteroaryl wie vorstehend genannt,
gegebenenfalls substituiertes Aryl-C₁-C₄-Alkyl wie vorstehend genannt,
gegebenenfalls substituiertes Heteroaryl-C₁-C₄-Alkyl wie vorstehend genannt,
oder eine Gruppe COR¹⁰

Bevorzugt bedeutet R⁷ Wasserstoff, gegebenenfalls substituiertes C₁-C₄-Alkyl, gegebenenfalls substituiertes C₂-C₄-Alkenyl, gegebenenfalls substituiertes Aryl-C₁-C₄-Alkyl oder gegebenenfalls substituiertes Heteroaryl-C₁-C₄-Alkyl, besonders bevorzugt C₁-C₄-Alkyl oder gegebenenfalls substituiertes Benzyl, und
R⁸ bedeutet bevorzugt Wasserstoff, gegebenenfalls substituiertes C₁-C₄-Alkyl oder gegebenenfalls substituiertes Aryl, besonders bevorzugt gegebenenfalls substituiertes Phenyl
- R⁹: hat die gleichen - allgemeinen sowie auch die bevorzugten - Bedeutungen wie R⁴ und ist von R⁸ unabhängig
- R¹⁰: bedeutet gegebenenfalls substituiertes C₁-C₄-Alkyl wie vorstehend genannt,
gegebenenfalls substituiertes C₃-C₆-Cycloalkyl wie vorstehend genannt,
gegebenenfalls substituiertes Aryl wie vorstehend genannt, oder
gegebenenfalls substituiertes Heteroaryl wie vorstehend genannt;
bevorzugt bedeutet R¹⁰ gegebenenfalls substituiertes C₁-C₄-Alkyl oder gegebenenfalls substituiertes Aryl, besonders bevorzugt Methyl oder gegebebenfalls substituiertes Phenyl
- V: bedeutet gegebenenfalls substituiertes C₁-C₄-Alkyl wie vorstehend genannt,
gegebenenfalls substituiertes Aryl wie vorstehend genannt,
gegebenenfalls substituiertes Heteroaryl wie vorstehend genannt,
Hydroxyl,
gegebenenfalls substituiertes C₁-C₄-Alkoxy wie vorstehend genannt,
gegebenenfalls substituiertes Allyloxy,
gegebenenfalls substituiertes Benzyloxy, oder
gegebenenfalls substituiertes Trimethylsilyloxy

Die mit "gegebenenfalls substituiert" gekennzeichneten Reste können, in beliebiger Position, einen oder mehrere, bevorzugt einen bis drei, Substituenten tragen, die gleich oder verschieden sind. Diese Substituenten bedeuten beispielsweise:

Wasserstoff, Halogen, Cyano, Nitro, Alkyl, Alkenyl, Alkinyl, Cycloalkyl, Aryl, Heteroalkyl, Heterocyclyl, Haloalkyl, Hydroxy, Alkoxy, Alkenyloxy, Alkinyloxy, Cycloalkyloxy, Aryloxy, Heteroaryloxy, Heterocyclyloxy, Haloalkoxy, Mercapto, Alkylthio, Alkenylthio, Alkinylthio, Cycloalkylthio, Arylthio, Heteroarylthio, Heterocyclylthio, Haloalkylthio, Amino, Alkylamino, Alkenylamino, Alkinylamino, Cycloalkylamino, Arylamino, Heteroarylamino, Heterocyclylamino, Dialkylamino, N-Alkyl-Arylamino, N-Alkyl-Heteroarylamino, Formyl, Alkylcarbonyl, Alkenylcarbonyl, Alkinylcarbonyl, Cycloalkylcarbonyl, Arylcarbonyl, Heteroarylcarbonyl, Heterocyclylcarbonyl, Carboxyl, Alkoxycarbonyl, Alkenyloxycarbonyl, Alkinyloxycarbonyl, Cycloalkyloxycarbonyl, Aryloxycarbonyl, Heteroaryloxycarbonyl, Heterocyclyloxycarbonyl, Formyloxy, Alkylcarbonyloxy, Alkenylcarbonyloxy, Alkinylcarbonyloxy, Cycloalkylcarbonyloxy, Arylcarbonyloxy, Heteroarylcarbonyloxy, Heterocyclylcarbonyloxy, Aminocarbonyl, Alkylaminocarbonyl, Alkenylaminocarbonyl, Alkinylaminocarbonyl, Cycloalkylaminocarbonyl, Arylaminocarbonyl, Heteroarylaminocarbonyl, Heterocyclylaminocarbonyl, Dialkylaminocarbonyl, N-Alkyl-Arylaminocarbonyl, N-Alkyl-Heteroarylaminocarbonyl, Hydroxylaminocarbonyl, Alkoxyaminocarbonyl, Alkenyloxyaminocarbonyl, Alkinylaminocarbonyl, Formamido, Alkylcarbonylamino, Alkenylcarbonylamino, Alkinylcarbonylamino, Cycloalkylcarbonylamino, Arylcarbonylamino, Heteroarylcarbonylamino, Heterocyclylcarbonylamino, Arylcarbonyl-N-Alkylamino, Heteroarylcarbonyl-N-Alkylamino, Aminothiocarbonyl, Alkylaminothiocarbonyl, Dialkylaminothiocarbonyl, Alkylsulfoxyl, Arylsulfoxyl, Heteroarylsulfoxyl, Alkylsulfonyl, Arylsulfonyl, Heteroarylsulfonyl, Hydroxysulfinyl, Alkoxysulfinyl, Hydroxysulfonyl, Alkoxysulfonyl, Alkylsulfinyloxy, Arylsulfinyloxy, Heteroarylsulfinyloxy, Alkylsulfonyloxy, Arylsulfonyloxy, Heteroarylsulfonyloxy, Dialkoxyphosphinyl, Bis-(Aryloxy)-Phosphinyl, Trialkylsilyl, Trialkylsilyloxy, oder ein Fragment der Formel R'-O-N=CR'', in der R' und R'' unabhängig voneinander Wasserstoff, Alkyl, Alkenyl oder Alkinyl bedeuten sowie, falls R' nicht Wasserstoff bedeutet, R'' auch Alkoxy bedeuten kann.

In den oben aufgeführten Substituenten haben Halogen und die organischen Teilstrukturen z.B. Alkyl, jeweils die vorstehend genannten Bedeutungen. Die Substituenten können ihrerseits wieder mit einem oder mehreren der oben aufgeführten Reste substituiert sein.

Die neuen Verbindungen der Formel I können bei der Herstellung als Gemische von Stereoisomeren (E/Z-Isomere an C=C oder C=N-Doppelbindungen, Diastereomere, Enantiomere) anfallen, die in üblicher Weise, z.B. durch Kristallisation oder Chromatographie, in ihre Einzelkomponenten getrennt werden können. Sowohl die einzelnen Isomeren wie auch ihre Gemische können als Fungizide, Insektizide oder Akarizide eingesetzt werden und werden alle von der Erfindung erfaßt. Die fungizide Wirkung wird bevorzugt.

Bevorzugt betrifft die Erfindung neue Verbindungen der allgemeinen Formel II, 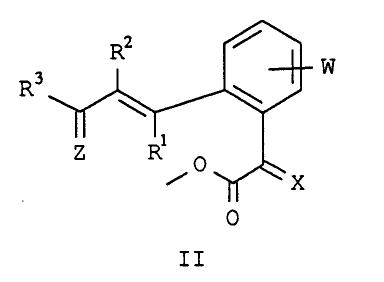
in der die variablen Substituenten folgende Bedeutungen haben:
- W: kann jede beliebige freie Position am Benzolring einnehmen und bedeutet Wasserstoff, Chlor, Methyl oder Methoxy, bevorzugt Wasserstoff
- X: bedeutet eine Gruppe CHOCH₃, NOCH₃, CHCH₃ oder CHC₂H₅
- Z: bedeutet Sauerstoff, eine Gruppe NR⁶, NOR⁷ oder N-NR⁸R⁹, bevorzugt Sauerstoff oder NOR⁷
- R¹ und R²: bedeuten Wasserstoff oder C₁-C₄-Alkyl wie vorstehend genannt, bevorzugt Wasserstoff und sind unabhängig voneinander
- R³: bedeutet Wasserstoff,
gegebenenfalls substituiertes C₁-C₄-Alkyl, gegebenenfalls substituiertes C₂-C₄-Alkenyl, gegebenenfalls substituiertes C₂-C₄-Alkinyl, gegebenenfalls substituiertes C₃-C₆-Cycloalkyl, gegebenenfalls substituiertes 3- bis 6-gliedriges Heterocyclyl,
gegebenenfalls substituiertes und/oder kondensiertes Aryl,
gegebenenfalls substituiertes und/oder kondensiertes Heteroaryl,
gegebenenfalls substituiertes Aryl-C₁-C₄-Alkyl, gegebenenfalls substituiertes Heteroaryl-C₁-C₄-Alkyl gegebenenfalls substituiertes Aryl-C₂-C₄-Alkenyl oder gegebenenfalls substituiertes Heteroaryl-C₂-C₄-Alkenyl, jeweils wie vorstehend genannt;
bevorzugt bedeutet R³ Wasserstoff, gegebenenfalls substituiertes C₁-C₄-Alkyl, gegebenenfalls substituiertes Aryl, gegebenenfalls substituiertes Heteroaryl, besonders bevorzugt C₁-C₄-Alkyl oder gegebenen-falls substituiertes Phenyl,
- R⁴: bedeutet Wasserstoff,
gegebenenfalls substituiertes C₁-C₄-Alkyl oder gegebenenfalls substituiertes C₃-C₆-Cycloalkyl, jeweils wie vorstehend genannt
bevorzugt bedeutet R⁴ Wasserstoff, Methyl oder Ethyl
- R⁶: bedeutet gegebenenfalls substituiertes C₁-C₄-Alkyl oder gegebenenfalls substituiertes und/oder kondensiertes Aryl oder Heteroaryl, jeweils wie vorstehend genannt;
bevorzugt bedeutet R⁶ gegebenenfalls substituiertes Phenyl
- R⁷ und R⁸: bedeuten Wasserstoff,
gegebenenfalls substituiertes C₁-C₄-Alkyl, gegebenenfalls substituiertes C₂-C₄-Alkenyl, gegebenenfalls substituiertes C₂-C₄-Alkinyl gegebenenfalls substituiertes C₃-C₆-Cycloalkyl, gegebenenfalls substituiertes und/oder konden-siertes Aryl,
gegebenenfalls substituiertes und/oder kondensiertes Heteroaryl,
gegebenenfalls substituiertes Aryl-C₁-C₄-Alkyl, gegebenenfalls substituiertes Heteroaryl-C₁-C₄-Alkyl, jeweils wie vorstehend genannt,
oder eine Gruppe COR¹⁰;
wobei R⁷ bevorzugt Wasserstoff, gegebenenfalls substituiertes C₁-C₄-Alkyl, gegebenenfalls substituiertes C₂-C₄-Alkenyl, gegebenenfalls substituiertes Aryl-C₁-C₄-Alkyl, besonders bevorzugt C₁-C₄-Alkyl oder gegebenenfalls substituiertes Benzyl und
R⁸ bevorzugt gegebenenfalls substituiertes C₁-C₄-Alkyl oder gegebe-nenfalls substituiertes Aryl-C₁-C₄-Alkyl, besonders bevorzugt C₁-C₄-Alkyl oder gegebenenfalls substituiertes Benzyl bedeutet
R⁹ hat die gleichen - allgemeinen sowie bevorzugten - Bedeutungen wie R⁴ und ist von R⁸ unabhängig
- R¹⁰: bedeutet gegebenenfalls substituiertes C₁-C₄-Alkyl, gegebenenfalls substituiertes C₃-C₆-Cycloalkyl, gegebenenfalls substituiertes Aryl, oder gegebenenfalls substituiertes Heteroaryl, jeweils wie vorstehend genannt;
bevorzugt bedeutet R¹⁰ C₁-C₄-Alkyl oder gegebenenfalls substituiertes Phenyl.

Außerdem betrifft die Erfindung bevorzugt neue Verbindungen der allgemeinen Formel III, 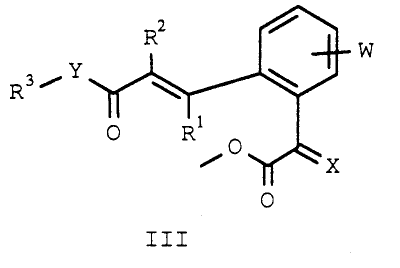
in der die variablen Substituenten folgende Bedeutungen haben:
- W: kann jede beliebige freie Position am Benzolring einnehmen und bedeutet Wasserstoff, Chlor, Methyl oder Methoxy, bevorzugt Wasserstoff
- X: bedeutet eine Gruppe CHOCH₃, NOCH₃, CHCH₃ oder CHC₂H₅
- Y: bedeutet Sauerstoff, Schwefel oder eine Gruppe NR⁴, bevorzugt Sauerstoff oder NR⁴
- R¹: bedeutet Wasserstoff oder C₁-C₄-Alkyl, bevorzugt Wasserstoff
- R²: bedeutet Wasserstoff, C₁-C₄-Alkyl oder eine Gruppe CO-V, bevorzugt Wasserstoff
- R³: bedeutet Wasserstoff,
gegebenenfalls substituiertes C₁-C₄-Alkyl, gegebenenfalls substituiertes C₁-C₄-Alkenyl, gegebenenfalls substituiertes und/oder kondensiertes Aryl,
gegebenenfalls substituiertes und/oder kondensiertes Heteroaryl,
gegebenenfalls substituiertes Aryl-C₁-C₄-Alkyl, oder gegebenenfalls substituiertes Heteroaryl-C₁-C₄-Alkyl, sowie, falls Y Sauerstoff bedeutet, auch Trialkylsilyl, jeweils wie vorstehend genannt;
bevorzugt bedeutet R³ C₁-C₄-Alkyl, gegebenenfalls substituiertes Phenyl oder gegebenenfalls substituiertes Benzyl
- R⁴: bedeutet Wasserstoff,
gegebenenfalls substituiertes C₁-C₄-Alkyl oder gegebenenfalls substituiertes C₃-C₆-Cycloalkyl, jeweils wie vorstehend genannt;
bevorzugt bedeutet R⁴ Wasserstoff, Methyl oder Ethyl
- V: bedeutet Hydroxyl, C₁-C₄-Alkoxy, Allyloxy, Trialkylsilyloxy oder gegebenenfalls substituiertes Benzyloxy, jeweils wie vorstehend genannt.

Besonders bevorzugt betrifft die Erfindung, in einer Hinsicht, neue Verbindungen der allgemeinen Formel IV, 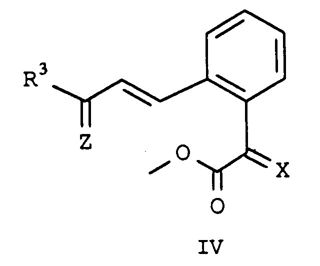
in der die variablen Substituenten folgende Bedeutungen haben:
- X: bedeutet eine Gruppe CHOCH₃, NOCH₃, CHCH₃ oder CHC₂H₅
- Z: bedeutet Sauerstoff oder eine Gruppe NOR⁷
- R³: bedeutet Wasserstoff,
gegebenenfalls substituiertes C₁-C₄-Alkyl, gegebenenfalls substituiertes C₂-C₄-Alkenyl, gegebenenfalls substituiertes und/oder kondensiertes Aryl, oder
gegebenenfalls substituiertes und/oder kondensiertes Heteroaryl, jeweils wie vorstehend genannt;
bevorzugt bedeutet R³ Wasserstoff, C₁-C₄-Alkyl oder gegebenenfalls substituiertes Aryl, besonders bevorzugt Wasserstoff, Methyl oder gegebenenfalls substituiertes Phenyl
- R⁷: bedeutet Wasserstoff,
gegebenenfalls substituiertes C₁-C₄-Alkyl, gegebenenfalls substituiertes C₂-C₄-Alkenyl, gegebenenfalls substituiertes Aryl-C₁-C₄-Alkyl, bevorzugt gegebenenfalls substituiertes Benzyl gegebenenfalls substituiertes Heteroaryl-C₁-C₄-Alkyl, bevorzugt mit fünfgliedrigem Heteroaryl substituiertes Methyl, jeweils wie vorstehend genannt, oder eine Gruppe COR¹⁰
- R¹⁰: bedeutet gegebenenfalls substituiertes C₁-C₄-Alkyl, gegebenenfalls substituiertes Aryl, oder gegebenenfalls substituiertes Heteroaryl, jeweils wie vorstehend genannt;
bevorzugt bedeutet R¹⁰ C₁-C₄-Alkyl oder gegebenenfalls substituiertes Phenyl.

Weiterhin betrifft die Erfindung, besonders bevorzugt neue Verbindungen der allgemeinen Formel V, 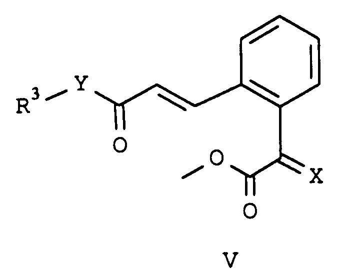
in der die variablen Substituenten folgende Bedeutungen haben:
- X: bedeutet eine Gruppe CHOCH₃, NOCH₃, CHCH₃ oder CHC₂H₅
- Y: bedeutet Sauerstoff oder eine Gruppe NR⁴
- R³: bedeutet gegebenenfalls substituiertes C₁-C₄-Alkyl, gegebenenfalls substituiertes und/oder kondensiertes Aryl,
gegebenenfalls substituiertes und/oder kondensiertes Heteroaryl,
gegebenenfalls substituiertes Aryl-C₁-C₄-Alkyl, oder gegebenenfalls substituiertes Heteroaryl-C₁-C₄-Alkyl, jeweils wie vorstehend genannt
- R⁴: bedeutet Wasserstoff oder C₁-C₄-Alkyl, bevorzugt Wasserstoff, Methyl oder Ethyl.

Die erfindungsgemäßen Verbindungen können beispielsweise auf den in den nachfolgenden Syntheseschemata skizzierten Wegen hergestellt werden. In allen Schemata bedeutet A - falls keine Einschränkungen speziell angegeben sind - eine Abkürzung für die in Anspruch 1 definierte Gruppierung R³-(Y)ₙ-; R bedeutet jeweils einen aliphatischen, aromatischen oder heterocyclischen Rest, R' kann darüber hinaus auch für Wasserstoff stehen. B repräsentiert eine der Gruppen 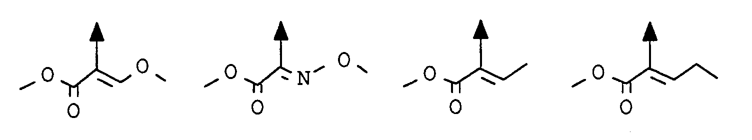
oder ein Fragment, aus dem sich eine dieser Gruppen aufbauen läßt. 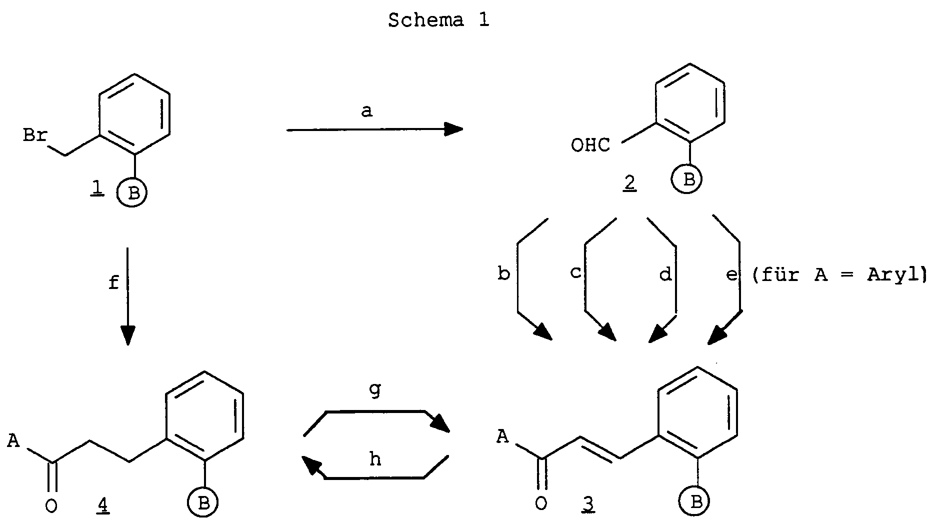
**a**, N-Methylmorpholin-N-Oxid, ΔT
**b**, A-COCH₃, Base oder Säure, ggf. wasserentziehendes Mittel
**c**, z.B. A-CO-CH₂-COOtBu, Piperidin/p-TsOH, dann CF₃COOH, dann DT
**d**, A-CO-CH=PR₃ oder A-CO-CH₂P(=O)Z₂ (Z = Ar, O-Alkyl)/Base
**e**, LiCH₂PO(OR)₂, A-CN
**f**, A-CO-CH₂-CO-A, NaOMe
**g**, H₂, Pd/C
**h**, PdCl₂(PhCN)₂, AgOSO₂CF₃ oder Sn(OSO₂CF₃)₂, N-Methylmorpholin

- DT =: erhöhte Temperatur, p-TsOH = p-Toluolsulfonsäure.

Schema I zeigt Synthesewege für die Carbonylverbindungen vom Typ 3 bzw. 4 (A = H, Alkyl, Aryl, Hetaryl, Organyloxy, Organylthio oder Mono- oder Diorganylamino) auf. Die ab-ungesättigten Verbindungen 3 lassen sich vorteilhaft aus Aldehyden 2 synthetisieren, was durch Aldol- [Org.Synth. Coll.Vol.**I** (1941),77] oder Knoevenagel-Kondensation mit anschließender Abspaltung einer Carbonyl- oder Carboxylfunktion [Acta Chem.Scand. **17**(1963),2316], wie auch durch Wittig[J.Chem.Soc. **1961**,1266] oder Horner-Wadsworth-Emmons-Olefinierung [Review: Org.React. **25**(1977),73] erreicht werden kann. Chalkone (d.h. A = Aryl) können auch in einer Eintopfreaktion durch Umsetzung von Lithiomethan-phosphonsäureestern mit aromatischen Nitrilen und den Aldehyden 2 [Synthesis **1991**,213] aufgebaut werden.

Die Aldehyde des Typs 2 sind nach der in EP 393428 beschriebenen Methode aus den Benzylbromiden 1 [Herstellung s. EP 226917, 337211 und 363818] darstellbar. Die für die Kondensationsreaktionen benötigten Ketone, Ester und 1,3-Dicarbonylverbindungen sind allgemein bekannte Bausteine der organischen Synthese; die carbonylsubstituierten Wittig- und Horner-Reagentien sind entweder bekannt oder nach Literaturmethoden aufbaubar [Ang.Chem. **72**(1960),572 und **73**(1961),27; Chem.Ber. **95**(1962),1513; Org.Synth. Coll.Vol.**VI** (1988),448].

Die gesättigten Carbonylverbindungen 4 lassen sich aus den Benzylbromiden 1 durch Umsetzung mit b-Dicarbonylverbindungen unter Ab-spaltung einer der beiden Carbonylgruppen darstellen [J.Org.Chem. **30** (1965),3321]; sie können andrerseits aus den ungesättigten Analogen 3, z.B. durch Hydrierung [Org.Synth. Coll.Vol.**I** (1941),101; J.Am. Chem.Soc. **111**(1989),1063] synthetisiert werden. Umgekehrt ist auch die Darstellung carbonylsubstituierter Alkene 3 aus den analogen Alkanen 4 möglich [Chem.Lett. **1983**,1207].

Die in Schema I mit **a**,**b**,**d**,**f** und **g** bezeichneten Methoden sind für die Darstellung der zu den Verbindungen 2, 3 und 4 analogen Substanzen, in denen R¹ und R² (s. Anspruch 1, 7 oder 8) nicht Wasserstoff bedeuten, ebenfalls geeignet. 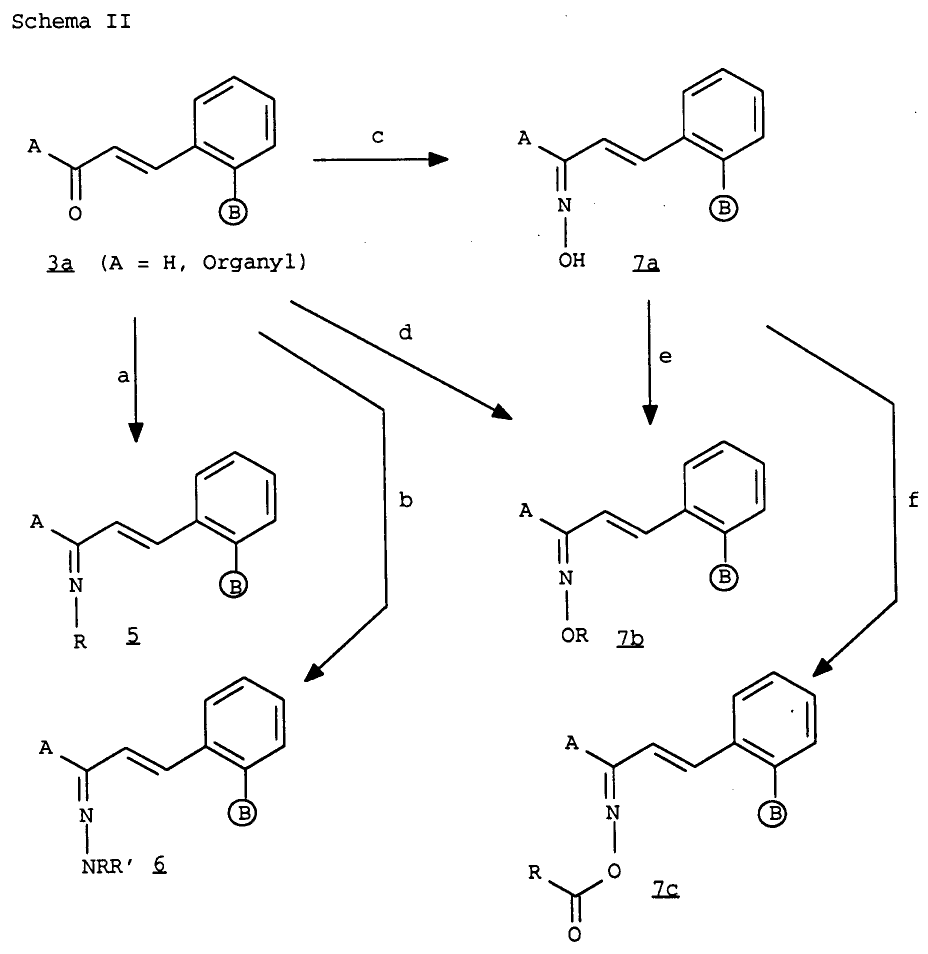
**a**, RNH₂, H⁺, Wasserentzug (Schlepperdestillation, Molekularsieb)
**b** RR'N-NH₂ x HCl bzw. x H₂SO₄, DT
**c**, H₂NOH x HCl, DT und/oder
**d**, H₂NOR x HCl, DT und/oder Hilfsbase (z.B. Pyridin)
**e**, R-Br, Base (z.B. NaOCH₃, K₂CO₃)
**f**, R-COCl, Pyridin

Schema II zeigt Möglichkeiten auf, wie die Aldehyde und Ketone des Typs 3a (A = H, Organyl) nach Standardmethoden in die N-funktionellen Derivate uberführt werden können. So sind Imine 5 [Bull.Soc. Chim.Belg. **81**(1972),643], Hydrazone 6 [J.Am.Chem.Soc. **90**(1968),6821] und Oxime [Helv.Chim.Acta **60**(1977),2294] 7a sowie Oximether 7b [J. Org.Chem. **38**(1973),3749] durch Kondensation der Carbonylverbindungen 3a mit Aminen, Hydrazinen und Hydroxyl- bzw. Alkoxyaminen zugänglich. Für die Darstellung der Oximether 7b kann eine zweistufige Synthese - Umsetzung von 3a zum freien Oxim 7a, gefolgt von O-Alkylierung [Helv.Chim.Acta **60**(1977),2294] - vorteilhaft sein. Oximester 7c können ebenfalls aus den Oximen 7a durch Umsetzung mit Säurechlorid/Pyridin gewonnen werden [J.Org.Chem. **33**(1968),150].

Die in Schema II beschriebenen Reaktionen sind für die Darstellung analoger Verbindungen mit R¹ und/oder R² · H gleichfalls geeignet. 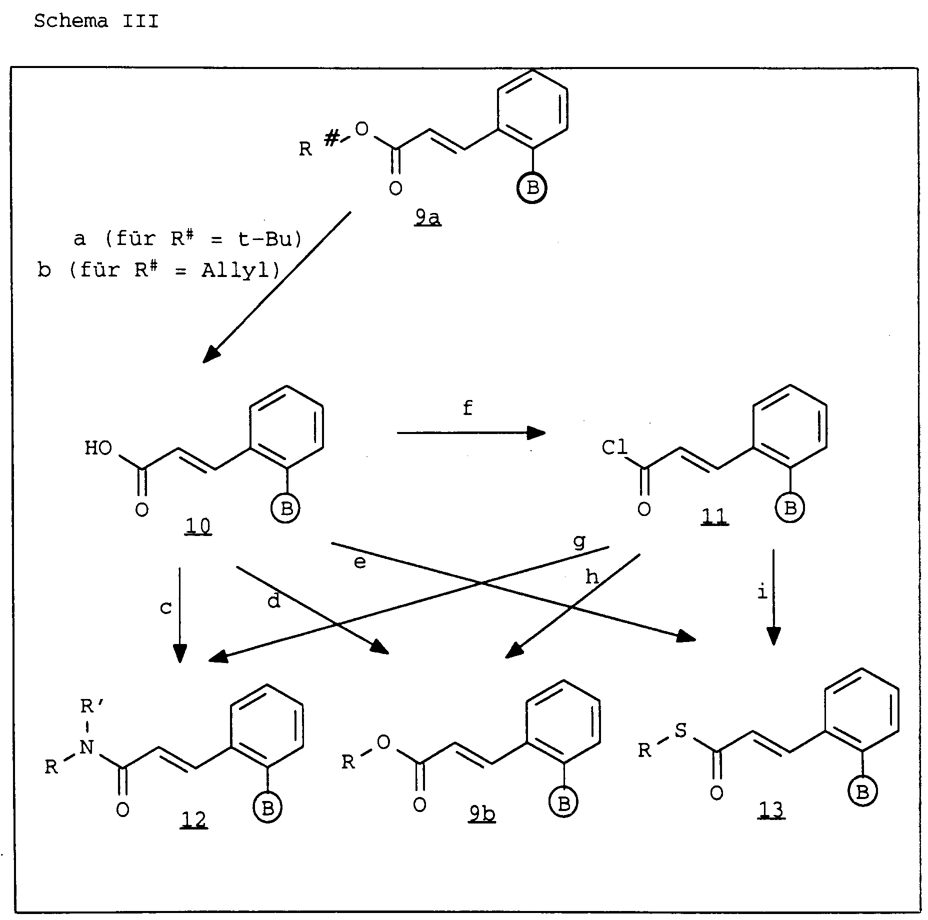
**a**, CF₃COOH, DT
**b**, PdCl₂ oder Pd(OOCCH₃)₂, PPh₃
**c**, RR'NH, carborylaktivierendes Agens (z.B. Di-(2-Pyridyl)-disulfid oder Ph₂P(=O)Cl)
**d**, R-Br, Base (z.B. K₂CO₃)
**e**, RSH, carboxylaktivierendes Agens (z.B. Dicyclohexyl-carbodiimid)
**f**, Chlorierungsmittel (z.B. SOCl₂), DT
**g**, RR'NH; ggf. + Pyridin, N(C₂H₅)₃
**h**, ROH, Pyridin, N(C₂H₅)₃
**i**, RSH, Pyridin, N(C₂H₅)₃

Die Synthese von Zimtsäurederivaten des Typs 3 (A = OR, SR, NRR') ist in Schema III erläutert. Sie verläuft vorteilhaft über solche, gemäß Schema I herstellbaren, Carbonsäureester 9a (entsprechend Typ 3 mit A = OR) die unter nicht-basischen Bedingungen zur Säure 10 verseifbar sind, z.B. tert.-Butylester [im sauren Medium, J.Am.Chem. Soc. **99**(1977),2353] oder Allylester [mit Palladiumverbindungen, Tetr.Lett. **1979**,613]. Die Carbonsäuren 10 lassen sich nach bekannten Methoden unter anderem zu mono- oder disubstituierten Amiden 12 [Tetr.Lett. **1970**,1901; Synthesis **1980**,385], anderen Estern 9b [EP 310954 und Synthesis **1975**,805] oder Thioestern 13 [J.Chem.Soc.(C) **1966**,540] umsetzen. Die Darstellung solcher Derivate kann auch auf dem allgemein bekannten Weg über das Säurechlorid 11 (durch Umsetzung der Carbonsäure 10 mit einem Chlorierungsmittel wie Thionylchlorid, Phosphortrichlorid, Phosphoroxychlorid oder Phosgen zugänglich) durch nucleophilen Ersatz des Chloratoms in 11 erfolgen.

Die in Schema III beschriebenen Reaktionen sind für die Darstellung analoger Verbindungen mit R¹ und/oder R² · H gleichfalls geeignet.

Die in Schema II und III aufgezeigten Reaktionswege eignen sich in gleicher Weise für die Umwandlung der gesättigten Carbonylverbindungen 4 in die entsprechenden Derivate. Auch eine Umwandlung der in den beiden Schemata beschriebenen (ab-ungesättigten) Produkte in ihre gesättigten Analoga ist unter geeigneten Hydrierungs- oder Reduktionsbedingungen möglich.

### Herstellungsbeispiele für ausgewählte Verbindungen

### Beispiel 1

### 2-Methoxyimino-2-{2-[3-oxoprop-(E)1-enyl]-phenyl}-essigsäuremethyl-ester (Verbindung Nr. 77 aus Tabelle I)

26,4 g (120 mmol) 2-Methoxyimino-2-(2-formyl-phenyl)-essigsäure-methylester (Darstellung s. EP 393428, S.3) und 45,6 g Triphenyl-phosphoranyliden-acetaldehyd werden in 500 ml Toluol unter Stickstoff 20 h bei 60°C gerührt. Nach dem Erkalten wird eingeengt, der Rückstand in tert.Butyl-methylether/n-Hexan aufgenommen, vom Unlöslichen (Triphenylphosphinoxid) abgesaugt, das Filtrat eingeengt, mit Diethylether versetzt und der Kolben ins Eisbad gestellt. Hierbei ausfallendes Ph₃PO wird ebenfalls abgesaugt und das Filtrat eingeengt. Nach Säulenchromatographie (Cyclohexan/Essigester 95:5 bis 90:10) erhält man 23,8 g (80 %) der Titelverbindung als gelbliche Kristalle vom Smp. 90-94°C. Das Produkt ist mit ca. 10% der vinylogen Verbindung 2-Methoxyimino-2-{2-[5-oxopenta-(E)1, (E)3-di-enyl]-phenyl}-essigsäuremethylester verunreinigt.

¹H-NMR (CDCl₃, d-Skala): 3,88 und 4,04 (2 s, je 3 H, COOCH₃ und NOCH₃); 6,70 (dd, 1 H, CH=CH-CHO), 7,1 - 7,7 (m, zus. 5 H, Aromatenprotonen und CH=CH-CHO); 9,66 ( d, 1 H, CHO)

### Beispiel 2

### 2-Methoxyimino-2-{2-[3-(2-chlorprop-2-enyloxyimino-prop-(E)1-enyl]-phenyl}-essigsäuremethylester (Verbindung Nr. 57 aus Tabelle VI)

3,7 g (15 mmol) 2-Methoxyimino-2-{2-[3-oxoprop-(E)1-enyl]-phenyl}-essigsäuremethylester (Beispiel 1) und 2,3 g (15 mmol) 2-Chlorprop-2-enyloxyamin-hydrochlorid werden in 200 ml Tetrahydrofuran (THF) 1 h am Rückfluß gekocht. Man läßt erkalten, wäscht 2 x mit gesättigter Kochsalzlösung, trocknet über Magnesiumsulfat und engt ein. Nach Reinigung durch Säulenchromatographie (Cyclohexan/Essigester 9:1, KG 60) erhält man die Titelverbindung (gelbes Öl, 2,9 g / 58 %) als 4:1-Gemisch der anti- und syn-isomeren Oximether.

¹H-NMR (CDCl₃, d-Skala), anti-Isomer: 3,88 und 4,04 (2 s, je 3 H, COOCH₃ und NOCH₃); 4,76 (s, 2 H, H₂C=CCl-CH₂O); 5,42 und 5,48 (2 s, je 1 H, H₂C=CCl); 6,60 und 6,82 (2 d, je 1 H, CH=CH-C=N), 7,1 - 7,8 m, 4 H, Aromatenprotonen); 7,92 (d 1 H, CH=N)

### Beispiel 3

### 2-{2-[3-oxobut-(E)1-enyl]-phenyl}-3-methoxyprop-(E)2-ensäu-remethylester (Verbindung Nr. 2 aus Tabelle I)

29 g (130 mmol) (2-Formylphenyl)-3-methoxyprop-(E)2-ensäuremethylester (Darstellung s. EP 393428, S.3) und 3,9 g (120 mmol) Triphenylphosphoranylidenaceton werden in 500 ml Toluol unter Stickstoff 14 h bei 65°C gerührt. Man läßt erkalten, engt ein, nimmt den Rückstand in Diethylether auf, saugt den Feststoff (Hauptmenge Ph₃PO) ab und zieht das Solvens im Vakuum ab. Säulenchromatographie liefert die Titelverbindung (21,4 g / 69 %) als bräunliches Öl, rein genug für weitere Umsetzungen. Durch Umkristallisation aus Ether/Pentan wird kristallines Produkt (Smp. 69-71°C) erhalten.

¹H-NMR (CDCl₃, d-Skala): 2,32 (s, 3 H, CH₃-C=O); 3,71 und 3,81 (2 s, je 3 H, COOCH₃ und CH-OCH₃); 6,70 (d, 1 H, CH=CH-C=O), 7,2 - 7,5 (m, zus. 3 H) und 7,72 (d, 1 H; Aromatenprotonen); 7,53 ( d, 1 H, CH=CH-C=O); 7,68 (s, 1 H, CH-OCH₃)

### Beispiel 4

### 2-{2-[3-methoxyiminobut-(E)1-enyl]-phenyl}-3-methoxyprop-(E)2-en-säuremethylester (Verbindung Nr. 22 aus Tabelle VI)

4,0 g (15 mmol) 2-{2-[3-oxobut-(E)1-enyl]-phenyl}-3-methoxyprop-(E)2-ensäuremethylester (Beispiel 3) und 1,3 g (15 mmol) Methoxyamin-hydrochlorid werden in 100 ml Methanol 1 h am Rückfluß gekocht. Nach dem Erkalten wird das Methanol abgezogen, der Rückstand in Diethylether aufgenommen, mit Wasser gewaschen, über Magnesiumsulfat getrocknet und eingeengt. Die Titelverbindung wird durch Säulenchromatographie (Cyclohexan/Essigester 95:5) gereinigt und in ihre C=N-Doppelbindungsisomeren getrennt (weniger polar: anti-Oximether, Öl, 1,4 g; polarer: syn-Oximether, Öl, 0,5 g; zusammen 1,9 g / 44 %)

¹H-NMR (CDCl₃, d-Skala), anti-Isomer: 2,00 (s, 3 H, CH₃-C=N); 3,70, 3,81 und 3,94 (3 s, je 3 H, COOCH₃ CH-OCH₃ und NOCH₃); 6,81 (AA'-System, 2 H, CH=CH-C=O), 7,15 - 7,5 (m, zus. 3 H) und 7,67 (d, 1 H; Aromatenprotonen); 7,60 (s, 1 H, CH-OCH₃)

### Beispiel 5

### 2-Methoxyimino-2-{2-[3-oxobut-(E)1-enyl]-phenyl}-essigsäuremethylester (Verbindung Nr. 78 aus Tabelle I)

50 g (226 mmol) 2-Methoxyimino-2-(2-formyl-phenyl)-essigsäuremethylester (EP 393428, S.3) und 72 g (227 mmol) Triphenylphosphoranyliden-aceton werden in 1000 ml Toluol unter Stickstoff 5 h bei 65°C gerührt. Man läßt abkühlen, zieht das Solvens im RV ab und erhält die Titelverbindung (46,9 g / 80 %) durch Umkristallisation aus Isopropanol als gelbliche Kristalle, Smp. 115-117°C.

¹H-NMR (CDCl₃, d-Skala): 2,35 (s, 3 H, CH₃-C=O); 3,92 und 4,04 (2 s, je 3 H, COOCH₃ und NOCH₃); 6,69 (d, 1 H, CH=CH-C=O); 7,26 (mc, 1 H), 7,47 (mc, 2 H) und 7,75 (mc, 1 H; Aromatenprotonen); 7,31 ( d, 1 H, CH=CH-C=O)

### Beispiel 6

### 2-Methoxyimino-2-{2-[3-hydroxyiminobut-(E)1-enyl]-phenyl}-essigsäuremethylester (Verbindung Nr. 71 aus Tabelle VI)

10,4 g (40 mmol) 2-Methoxyimino-2-{2-[3-oxobut-(E)1-enyl]-phenyl}-essigsäuremethylester (Beispiel 5) und 3,1 g (44 mmol) Hydroxylamin-hydrochlorid werden in 200 ml Methanol 1 h am Rückfluß gekocht. Nach dem Erkalten wird das Reaktionsgemisch eingeengt und der Rückstand mit Diethylether digeriert und der Feststoff abgesaugt. Nach Säulenchromatographie erhält man die Titelverbindung als getrennte C=N-Doppelbindungsisomere (weniger polar: syn-Oxim, 2,5 g, farblose Kristalle, Smp. 127-130°C; polarer: anti-Oxim, 5,0 g, Smp. 136-138°C; zusammen 7,5 g / 68 %).

¹H-NMR (CDCl₃, d-Skala): syn-Isomer, 2,07 (s, 3 H, CH₃-C=N); 3,98 und 4,06 (2 s, je 3 H, COOCH₃ und NOCH₃); 6,72 (d, 1 H, CH=CH-C=N);7,00 (mc, 1 H), 7,3 - 7,5 (m, 2 H) und 7,80 (mc, 1 H; Aromatenprotonen), 7,54 (d, 1 H, CH=CH-C=N); 8,45 (br, 1 H, N-OH) / anti-Isomer,2,08 (s, 3 H, CH₃-C=N); 3,90 und 4,05 (2 s, je 3 H, COOCH₃ und NOCH₃); 6,73 (d, 1 H, CH=CH-C=N); 7,20 (mc, 1 H), 7,43 (mc, 2 H) und 7,79 (mc, 1 H; Aromatenprotonen), 7,53 (d, 1 H, CH=CH-C=N); 8,45 (br, 1 H, N-OH)

### Beispiel 7

### 2-Methoxyimino-2-{2-[3-(anti)-2,6-difluorbenzyloxyiminobut-(E)1-en-yl]-phenyl}-essigsäuremethylester (Verbindung Nr. 107 aus Tabelle VII)

4,0 g (15,2 mmol) 2-Methoxyimino-2-{2-[3-(anti)-hydroxyiminobut-(E)1-enyl]-phenyl}-essigsäuremethylester (Beispiel 6) werden in 60 ml Dimethylformamid bei +5°C 0,9 g (15,2 mmol) gepulvertes Kaliumhydroxid zugegeben und bei dieser Temperatur eine Lösung von 3,1 g (15,2 mmol) 2,6-Difluorbenzylbromid in 20 ml Dimethylformamid zugetropft. Man rührt noch 1 h bei +5°C, gießt das Reaktionsgemisch in Eiswasser, extrahiert 3 x mit Diethylether, trocknet über Magnesium-sulfat und engt ein. Chromatographie (Cyclohexan/Essigester 9:1, KG 60) liefert die Titelverbindung (1,2 g / 20 %) als farblose Kristal-le , Smp. 120-122°C.

¹H-NMR (CDCl₃, d-Skala): 1,98 (s, 3 H, CH₃-C=N); 3,87 und 4,01 (2 s, je 3 H, COOCH₃ und NOCH₃); 5,24 (s, 2 H, ArCH₂-O); 6,60 und 6,80 (2 d, je 1 H, CH=CH-C=N), 6,93 (mc, 2 H), 7,1 - 7,5 (m, 6 H) und 7,70 (mc, 1 H; Aromatenprotonen

### Beispiel 8

### 2-Methoxyimino-2-{2-[3-Benzyloxyiminobut-(E)1-enyl]-phenyl}-essig-säuremethylester (Verbindung Nr. 83 aus Tabelle VI)

2,0 g (7,6 mmol) 2-Methoxyimino-2-{2-[3-oxobut-(E)1-enyl]-phenyl}-essigsäuremethylester (Beispiel 5) und 1,2 g (7,6 mmol) Benzyloxyamin werden in 60 ml Methanol gelöst, 1 ml konzentrierte Salzsäure zugegeben und die Reaktionsmischung 1 h am Rückfluß gekocht. Man läßt erkalten, engt ein, nimmt den Rückstand in Diethylether auf, wäscht mit Wasser, trocknet über Magnesiumsulfat und zieht das Solvens ab. Nach Säulenchromatographie (Cyclohexan/Essigester 95:5 bis 90:10) erhält man die Titelverbindung als getrennte C=N-Doppelbindungsisomere (weniger polar: anti-Oximether, 1,1 g, Öl; polarer: syn-Oximether, 0,6 g, Öl; zusammen 1,7 g / 61 %).

¹H-NMR (CDCl₃, d-Skala), anti-Isomer: 2,04 (s, 3 H, CH₃-C=N); 3,88 und 4,03 (2 s, je 3 H, COOCH₃) und NOCH₃)); 5,24 (s, 2 H, Ar-CH₂-O); 6,62 und 6,82 (2 d, je 1 H, CH=CH-C=N); 7,17 (mc, 1 H), 7,3 -7,5 (m, 7 H) und 7,69 (mc, 1 H; Aromatenprotonen)

### Beispiel 9

### 2-Methoxyimino-2-{2-[3-(anti)-phenylhydrazonobut-(E)1-enyl]-phenyl}-essigsäuremethylester (Verbindung Nr. 68 aus Tabelle VIII)

2,6 g (10 mmol) 2-Methoxyimino-2-{2-[3-oxobut-(E)1-enyl]-phenyl}-essigsäuremethylester (Beispiel 5 ) und 1,08 g (10 mmol) Phenylhydrazin werden in 50 ml Methanol 4 h unter Rückfluß gekocht. Man läßt abkühlen, zieht das Lösungsmittel ab und reinigt das Produkt durch Säulenchromatographie (Cyclohexan/Essigester 80:20, KG 60). Man erhält als einziges definiertes Produkt das anti-Hydrazon (Öl, 1,5 g / 43 %), gelbe Kristalle aus Diethylether (Smp. 155-158°C).

¹H-NMR (CDCl₃, d-Skala): 1,99 (s, 3 H, CH₃-C=N); 3,86 und 4,05 (2 s, je 3 H, COOCH₃ und NOCH₃); 6,48 (d, 1 H, CH=CH-C=N); 6,86 (t, 1 H), 7,1 - 7,5 (m, 7 H) und 7,74 (d, 1 H; Aromatenprotonen); 7,02 ( d, 1 H, CH=CH-C=N)

### Beispiel 10

### 2-Methoxyimino-2-{2-[3-oxo-3-(4-methoxyphenyl)-prop-(E)1-enyl]-phe-nyl}-essigsäuremethylester (Verbindung Nr. 57 aus Tabelle II)

7,7 g (35 mmol) 2-Methoxyimino-2-(2-formyl-phenyl)-essigsäuremethylester (EP 393428, S.3) und 15,5 g (38 mmol) Triphenylphosphoranyliden-4-methoxy-acetophenon werden in 300 ml Toluol 2 h unter Rückfluß gekocht. Nach dem Erkalten wird eingeengt und der Rückstand säulenchromatographiert (Cyclohexan/Essigester 95:5 bis 85:15, KG 60). Man erhält die Titelverbindung (9,7 g / 69 %) als fast farblose Kristalle (Smp. 137-141°C).

¹H-NMR (CDCl₃, d-Skala): 3,86, 3,91 und 4,10 (3 s, je 3 H, COOCH₃, NOCH₃ und Ar-OCH₃); 7,00 und 8,02 (2 d, je 2 H, Protonen im p-disubstituierten Aromaten); 7,2 - 7,9 (m, 6 H, Protonen im o-disubstituierten Aromaten und CH=CH-C=O)

### Beispiel 11

### 2-Methoxyimino-2-{2-[3-methoxyimino-3-(4-methoxyphenyl)-prop-(E)1-enyl]-phenyl}-essigsäuremethylester (Verbindung Nr. 97 aus Tabelle VI)

3,5 g (10 mmol) 2-Methoxyimino-2-{2-[3-oxo-3-(4-methoxyphenyl)-prop-(E)1-enyl]-phenyl}-essigsäuremethylester (Beispiel 10) und 1,0 g (12 mmol) Methoxyamin-hydrochlorid werden 2 h unter Rückfluß gekocht. Man läßt auf Raumtemperatur (20°C, RT) kommen und zieht das Methanol ab. Säulenchromatographie (Cyclohexan/Essigester 9:1, KG 60) liefert die Titelverbindung (anti/syn-Isomerengemisch, 0,9 g / 24 %) als bräunliches Öl, kristallisierbar aus Pentan/Diethylether (Smp. 100-104°C).

¹H-NMR (CDCl₃, d-Skala) : 3,80, 3,82, 3,94 und 4,01 (4 s, je 3 H, COOCH₃, beide NOCH₃ und Ar-OCH₃ im anti-Isomer); 3,78, 3,83, 3,90 und 3,92 (4 s, je 3 H, COOCH₃, beide NOCH₃ und Ar-OCH₃ im syn-Iso-mer); 6,30 (d, 1 H, CH=CH-C=N im syn-Isomer); 6,60 (d, 1 H, CH=CH-C=N im anti-Isomer); 6,85 - 7,9 (m, 9 H, Aromatenprotonen und CH= CH-C=N in beiden Isomeren)

### Beispiel 12

### 2-Methoxyimino-2-{2-[2-tert.-butoxycarbonyl-(E)-ethenyl]-phenyl}-essigsäuremethylester (Verbindung Nr. 112 aus Tabelle I)

17,6 g (80 mmol) 2-Methoxyimino-2-(2-formyl-phenyl)-essigsäure-methylester (EP 393428, S.3) und 33 g (88 mmol) Triphenylphosphoranylidenessigsäure-tert.-butylester werden in 500 ml Toluol unter Stickstoff 1 h bei 60°C gerührt. Man läßt abkühlen, zieht das Solvens ab, versetzt den Rückstand mit Diethylether und stellt ins Eisbad. Die Hauptmenge an Triphenylphosphinoxid fällt aus und wird abgesaugt. Das Filtrat wird eingeengt und der Rückstand säulenchromatographisch gereinigt (Cyclohexan/Essigester 95:5 bis 93:7). Man erhält die Titelverbindung (24 g / 94 %) als bräunliches Öl.

¹H-NMR (CDCl₃, d-Skala): 1,53 (s, 9 H, (CH₃)₃CO); 3,85 und 4,03 (2 s, je 3 H, COOCH₃ und NOCH₃); 6,33 (d, 1 H, CH=CH-C=O); 7,1 - 7,75 (m, 5 H, Aromatenprotonen und CH=CH-C=O)

### Beispiel 13

### 2-Methoxyimino-2-{2-[2-carboxyl-(E)-ethenyl]-phenyl}-essigsäure-methylester (Verbindung Nr. 109 aus Tabelle I)

Eine Lösung von 18 g (48 mmol) 2-Methoxyimino-2-{2-[2-tert.-butoxy-carbonyl-(E)-ethenyl]-phenyl}-essigsäuremethylester (Beispiel 12) in 100 ml THF wird mit 20 ml konzentrierter Salzsäure 45 min bei 65°C gerührt. Man läßt abkühlen, zieht das Solvens ab und verreibt den verbliebenen Kristallbrei mit Diisopropylether/Pentan; der Feststoff wird abgesaugt und getrocknet. Man erhält die Titelverbindung (12 g / 81 %) als beigefarbene Kristalle (Smp. 178-182°C).

¹H-NMR (CDCl₃, d-Skala): 3,93 und 4,09 (2 s, je 3 H, COOCH₃ und NOCH₃); 6,48, d, 1 H, CH=CH-C=O); 7,23 (mc, 1 H), 7,50 (mc, 2 H) und 7,78 (mc, 1 H; Aromatenprotonen); 7,59 (d, 1 H, CH=CH-C=O); 10,8 (br, 1 H, COOH)

### Beispiel 14

### 2-Methoxyimino-2-{2-[2-(2,6-difluorbenzylorycarbonyl)-(E)-ethenyl]-phenyl}-essigsäuremethylester (Verbindung Nr. 37 aus Tabelle III)

3,4 g (13 mmol) 2-Methoxyimino-2-{2-[2-carboxyl-(E)-ethenyl]-phenyl}-essigsäuremethylester (Beispiel 13) werden in 50 ml Dimethyl-formamid gelöst, auf +5°C abgekühlt und 0,75 g (13 mmol) pulverisiertes Kaliumhydroxid zugesetzt. Bei der gleichen Temperatur wird eine Lösung von 2,7 g (13 mmol) 2,6-Difluorbenzylbromid in 15 ml Dimethylformamid zugetropft, dann läßt man die Reaktionsmischung auf RT kommen und rührt noch 1 h weiter. Das Reaktionsgemisch wird in Eiswasser eingerührt, 3 x mit Diethylether extrahiert, die vereinigten Extrakte mit Wasser gewaschen, getrocknet und eingeengt. Aus dem öligen Rückstand erhält man durch Verreiben mit Diisopropylether die Titelverbindung (3,2 g / 63 %) in kristalliner Form (Smp. 108-110°C).

¹H-NMR (CDCl₃, d-Skala): 3,91 und 4,06 (2 s, je 3 H, COOCH₃ und NOCH₃) 5,31 (s, 2 H, Ar-CH₂-O); 6,42 (d, 1 H, CH=CH-C=O); 6,95 (mc, 2 H) und 7,1 - 7,8 (m, 6 H; Aromatenprotonen und CH=CH-C=O)

### Beispiel 15

### 2-Methoxyimino-2-{2-[3-oxo-3-phenyl)-propyl]-phenyl}-essigsäure-methylester (Verbindung Nr. 50 aus Tabelle X)

5,0 g (17,5 mmol) 2-Methoxyimino-2-(2-brommethyl-phenyl)-essigsäure-methylester (EP 363818, S.3) und 3,9 g (17,5 mmol) Dibenzoylmethan werden in 50 ml Methanol gelöst und mit 2,7 g (19,3 mmol) Kaliumcarbonat und einer Spatelspitze Kaliumjodid versetzt. Man rührt 90 min unter Rückfluß, läßt abkühlen und zieht das Lösungsmittel ab. Der Rückstand wird mit Wasser versetzt, 2 x mit Methylenchlorid extrahiert, und die vereinigten Extrakte über Magnesiumsulfat getrock-net und eingeengt. Flash-Chromatographie (n-Hexan/Essigester 90:10 bis 75:25) liefert die Titelverbindung (1,4 g / 25 %) als tiefrotes Öl.

IR (KBr, ∼ in cm⁻¹) : 1722, 1689, 1218, 1205, 1067, 1049, 1015, 957, 748, 689

Beispiele für im Hinblick auf ihre Wirkung als Schädlingsbekämpfungsmittel bevorzugte Verbindungen sind in den nachstehenden Tabellen gegeben.

Die Doppelbindung C=X ist in den nachstehenden Tabellen, wenn nicht anders angegeben, E- (bzw. anti-)konfiguriert, ebenso eine zwischen den in der allgemeinen Formel I mit R¹ und R² substituierten Kohlenstoffatomen liegende Doppelbindung. Wurde in Verbindungen mit einer C=N-Doppelbindung in der Seitenkette (d. h. Z = N-R⁶, N-OR⁷ oder N-NR⁸R⁹) nur ein Stereoisomeres isoliert, oder wurden die Isomeren getrennt, ist die Konfiguration in einer Spalte "Isomer" durch den Buchstaben a (für anti) bzw. s (für syn) angegeben. Nichtaufgetrennte Gemische der beiden Formen erscheinen in dieser Spalte mit Prozentangaben: a(70) und s(30) bedeutet: Gemisch aus 70 % anti-und 30 % syn-Isomer.

Zur Charakterisierung nicht kristallin erhaltener Verbindungen wurde die chemische Verschiebung (Solvens CDCl₃, Standard TMS. δ-Skala) von Protonen herangezogen, die den Resten R¹ und R² entsprechen.

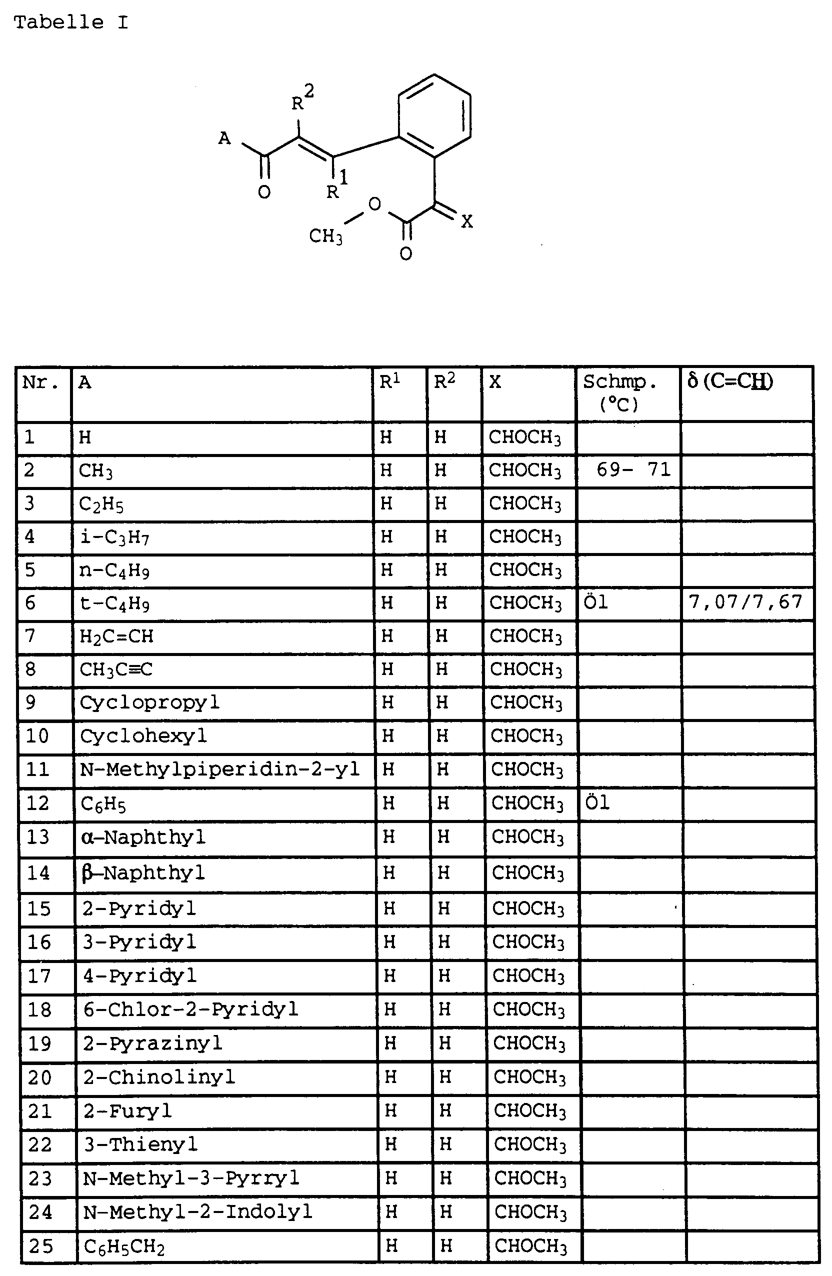

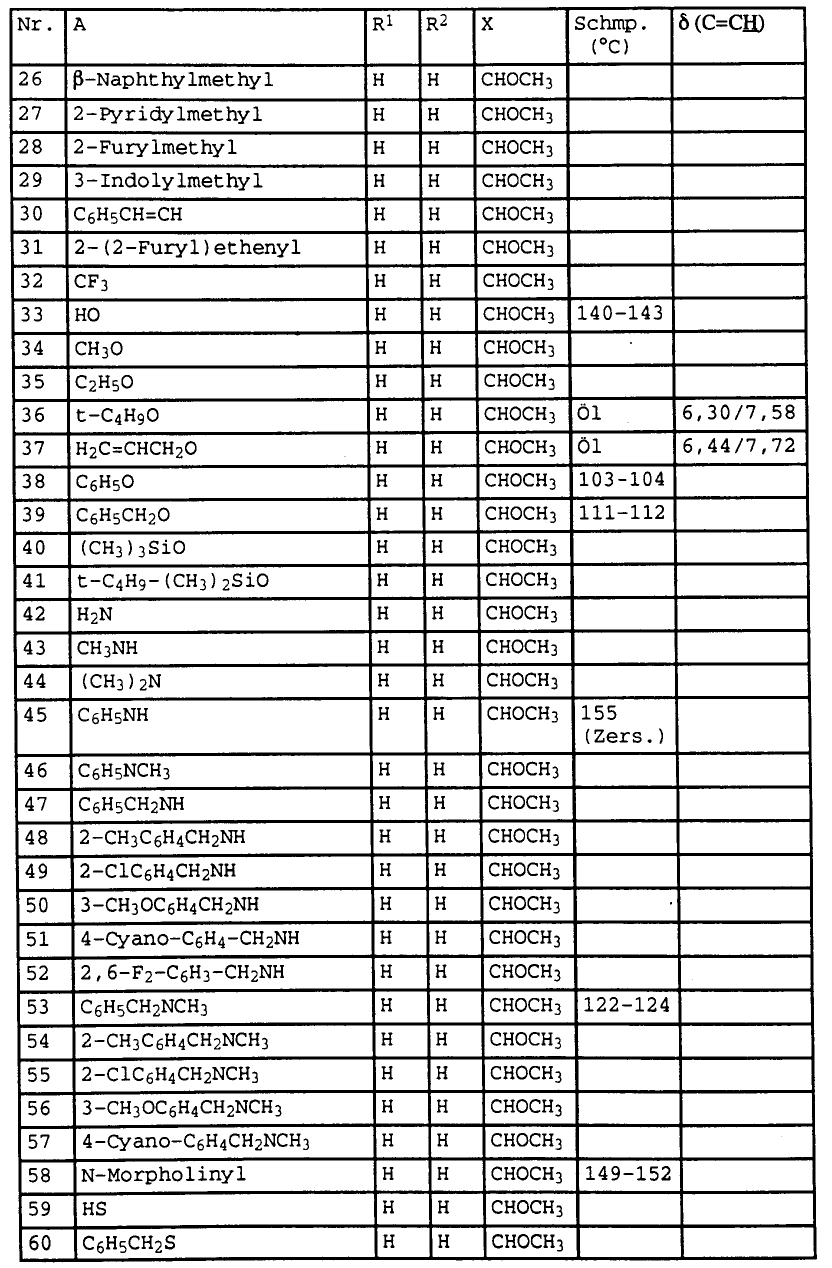

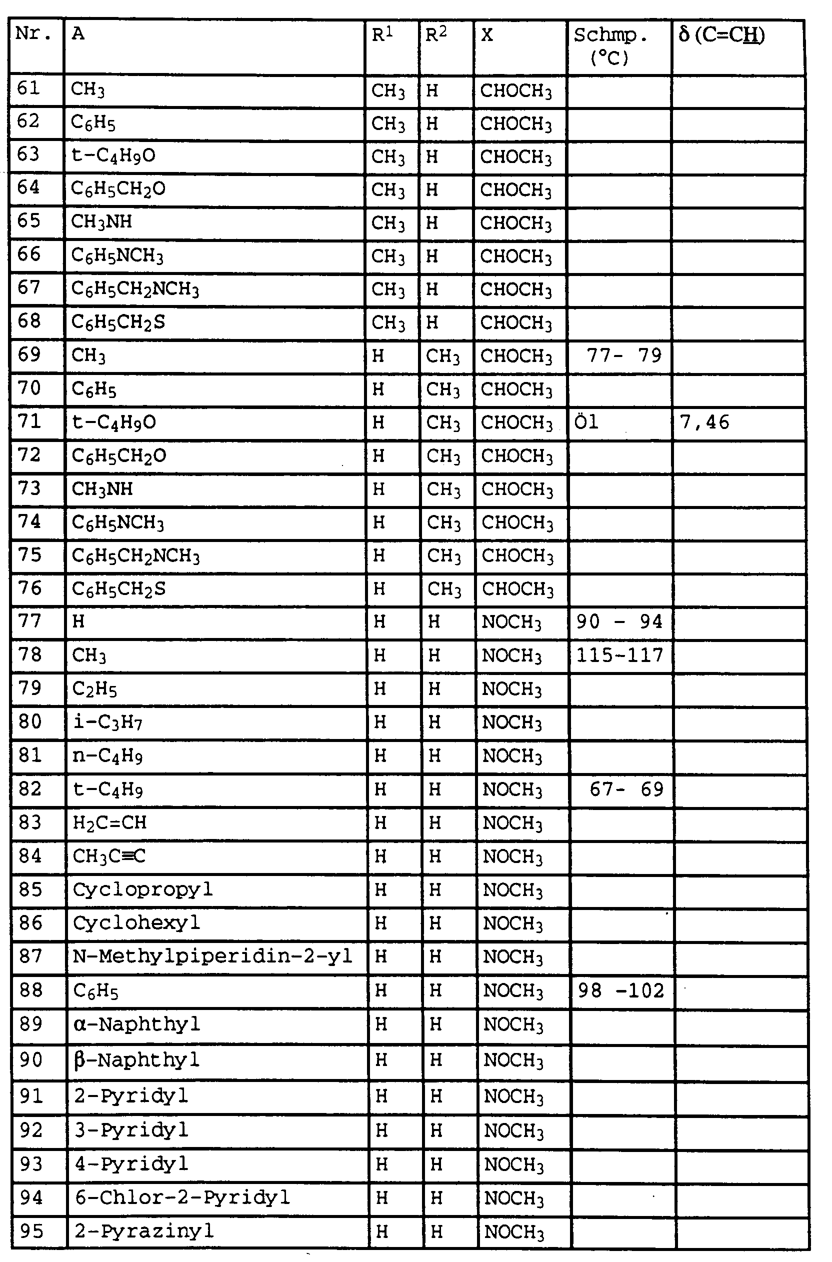

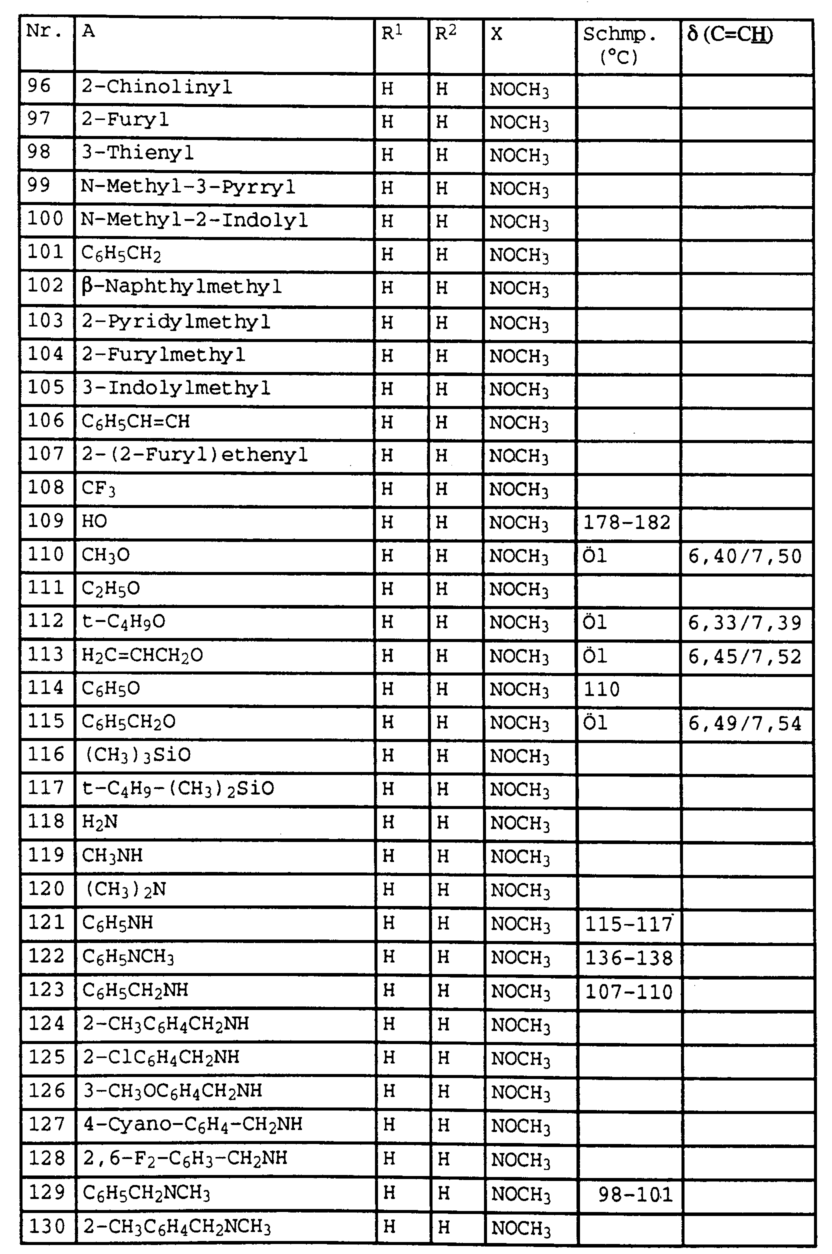

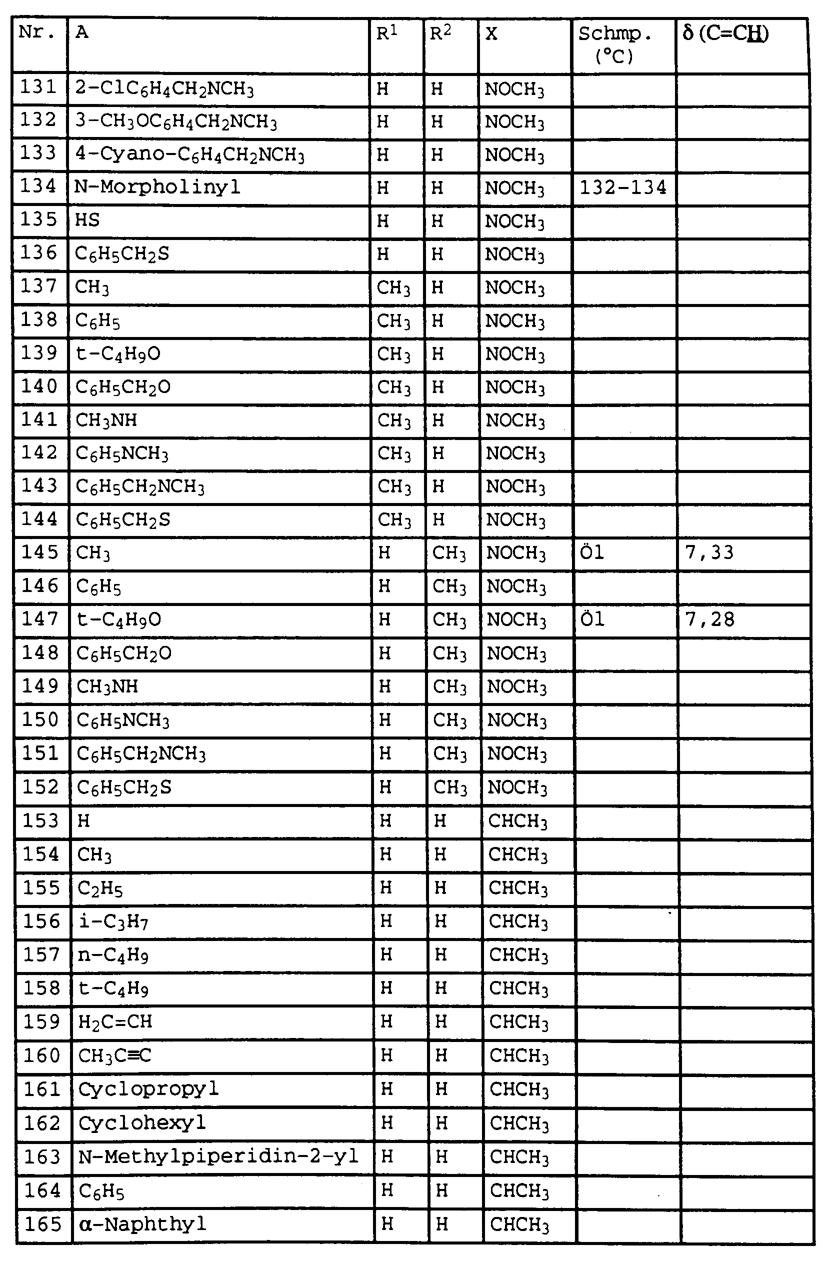

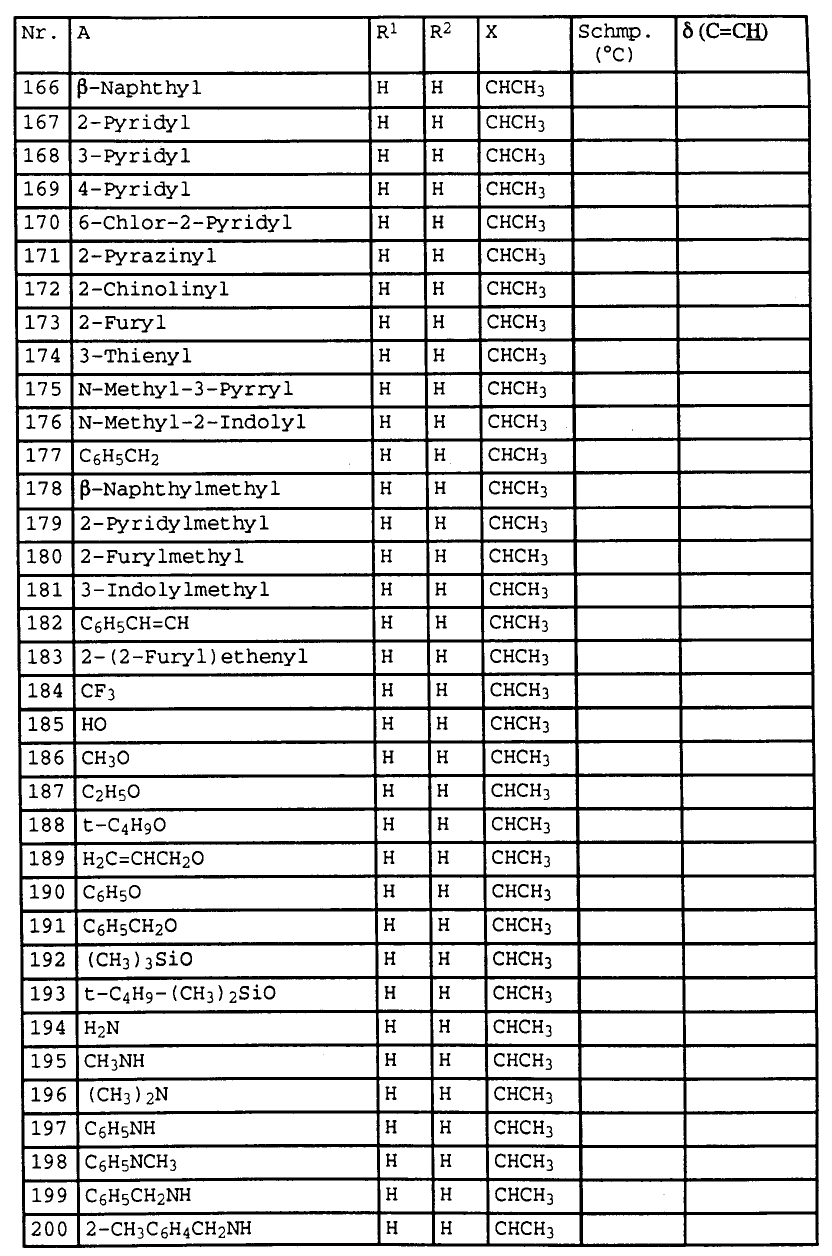

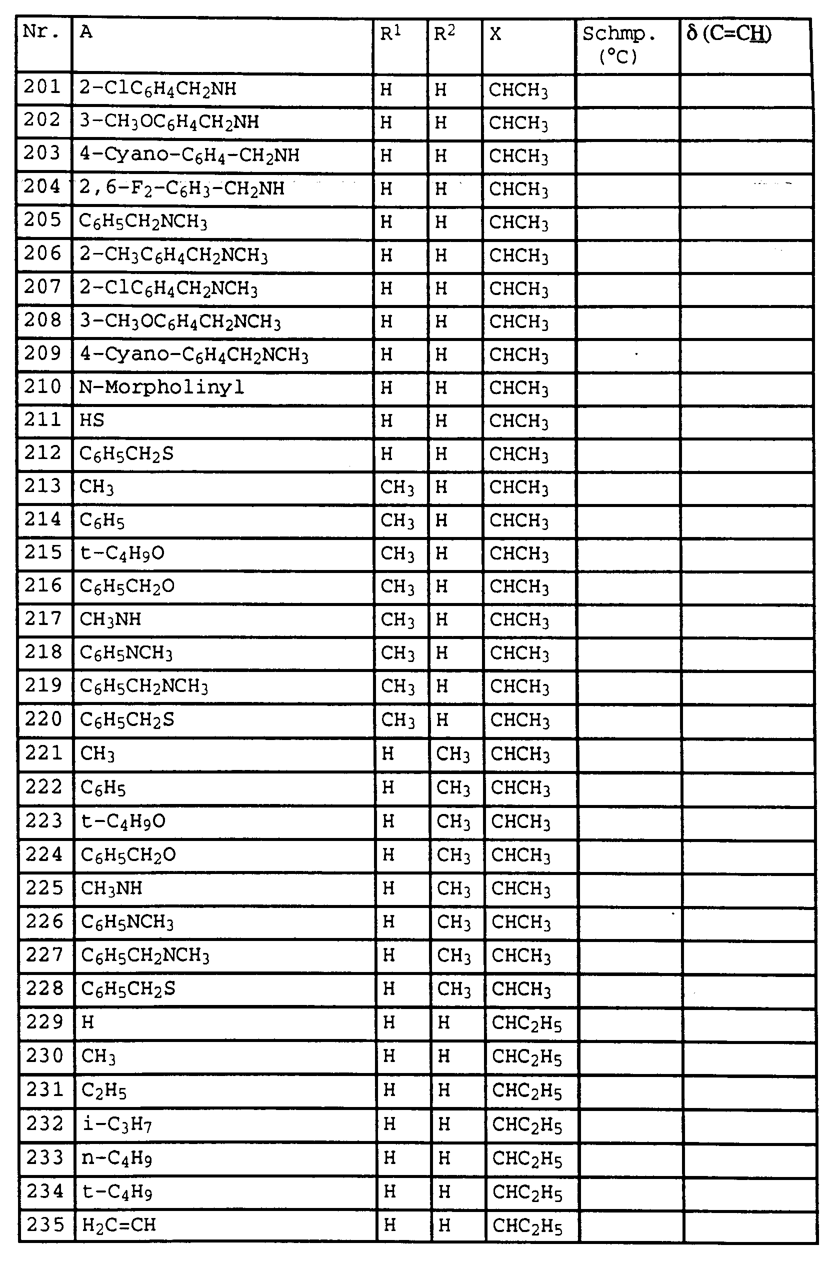

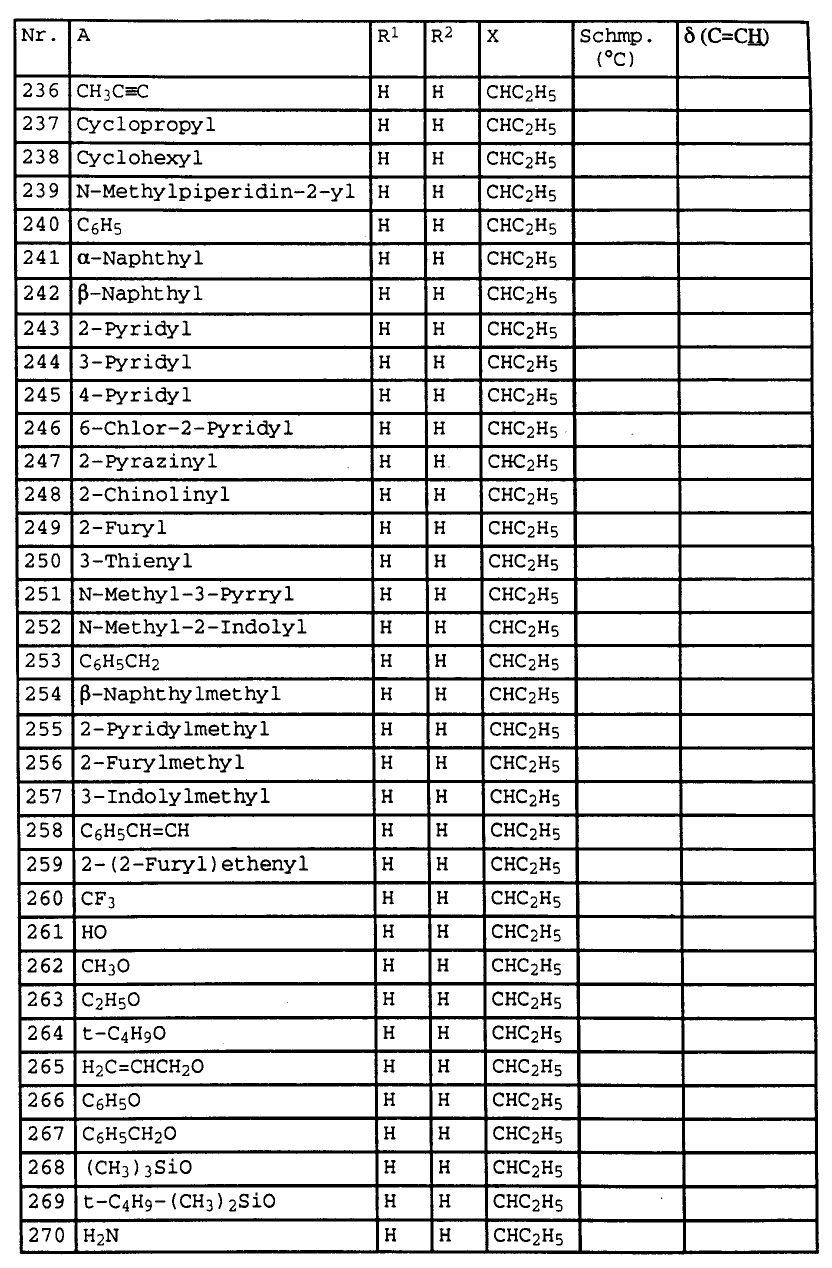

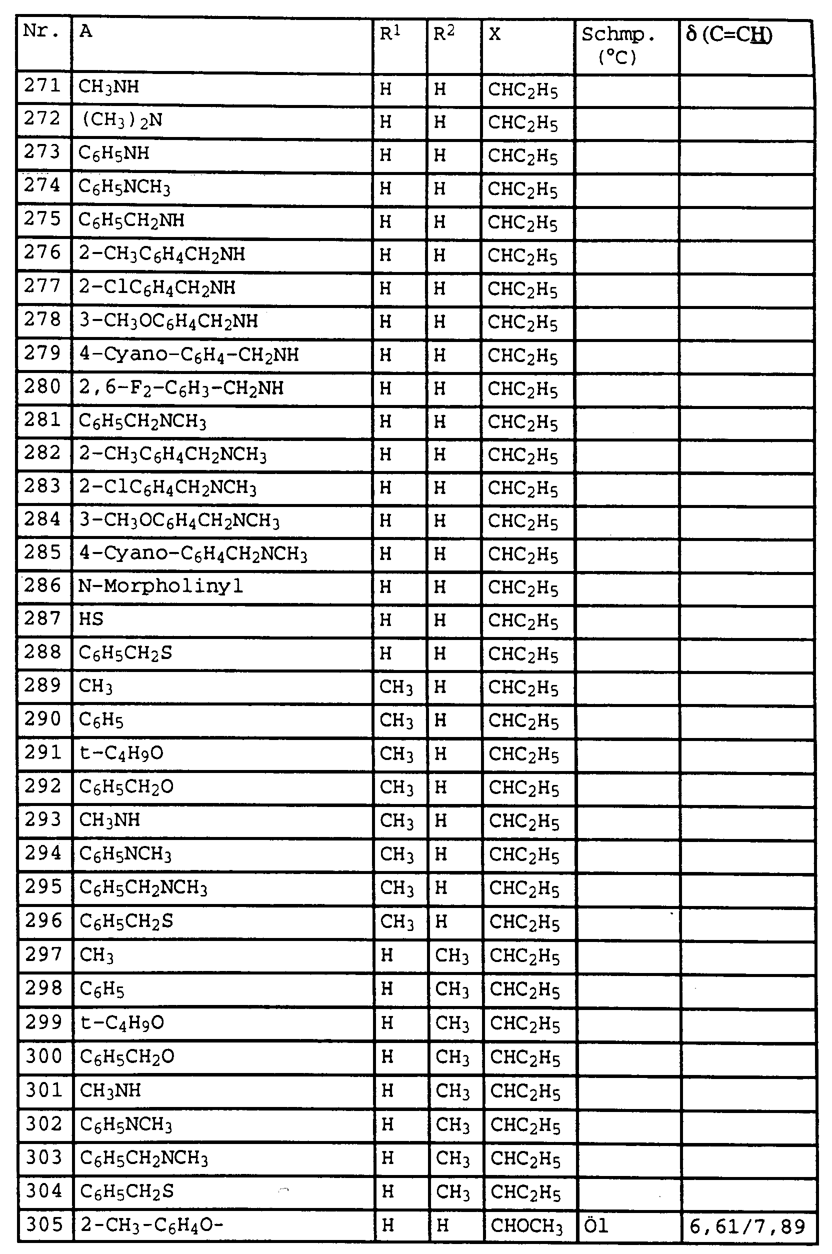

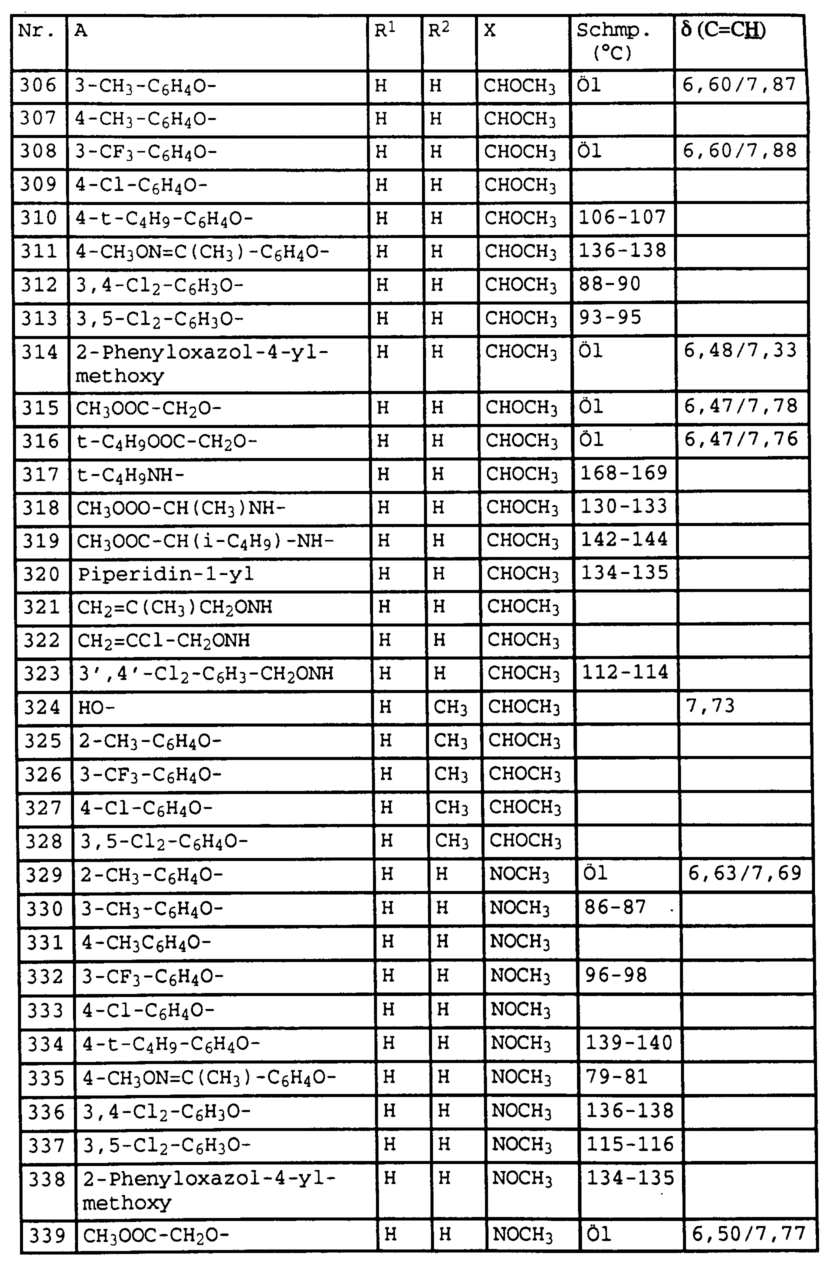

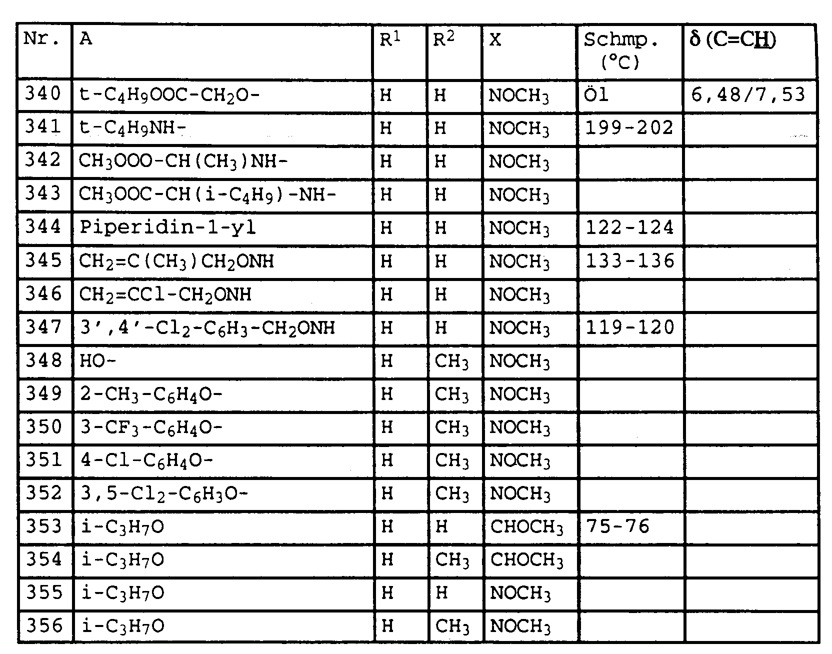

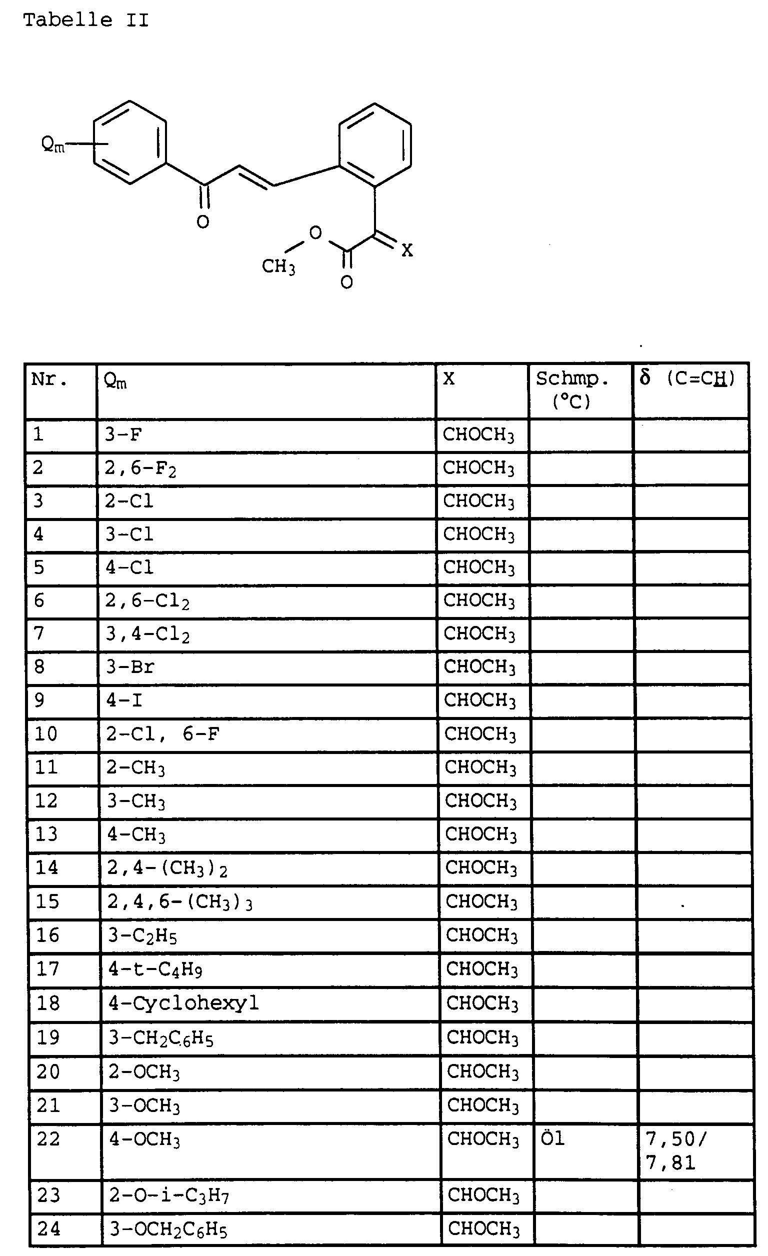

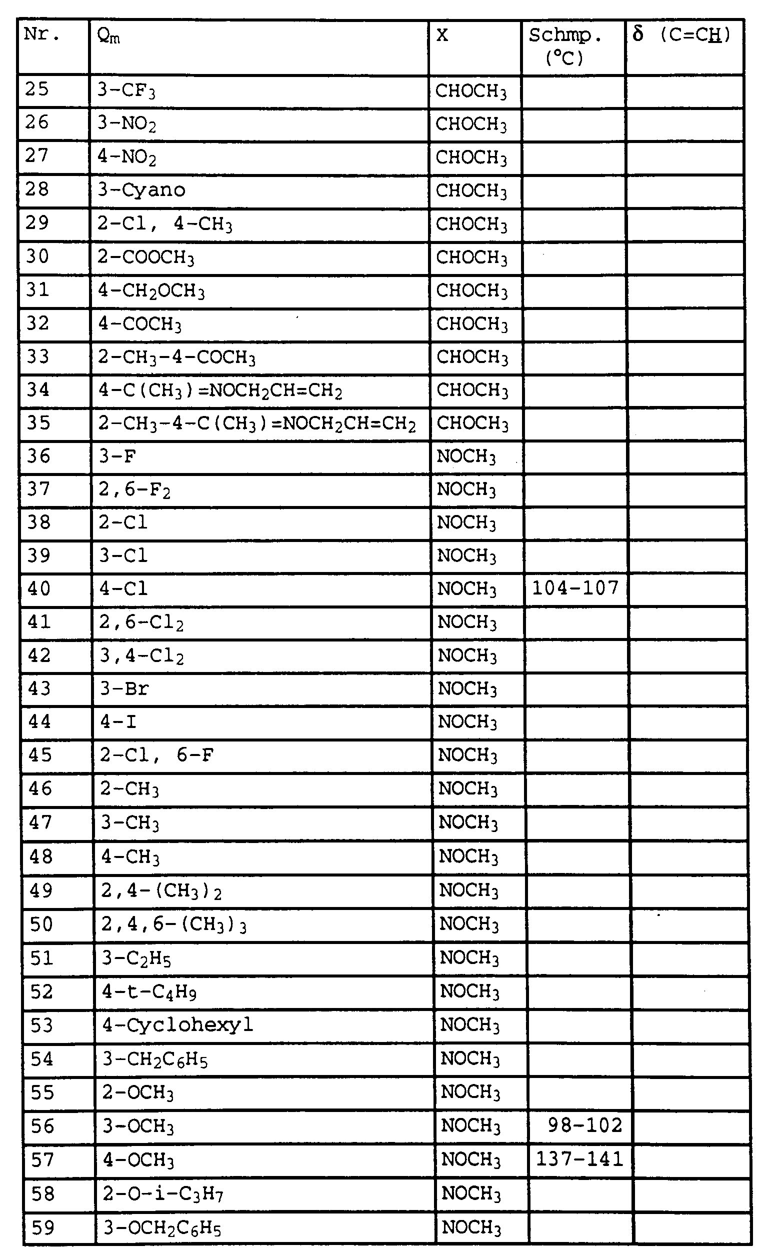

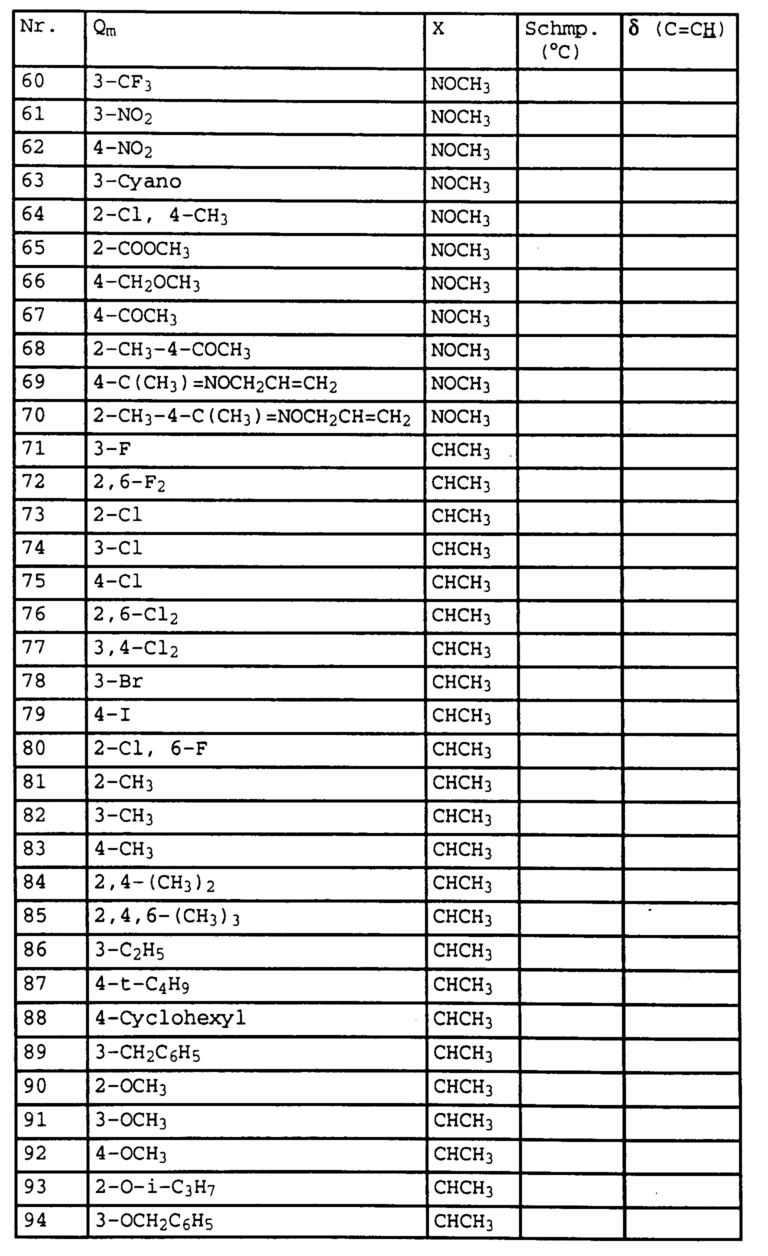

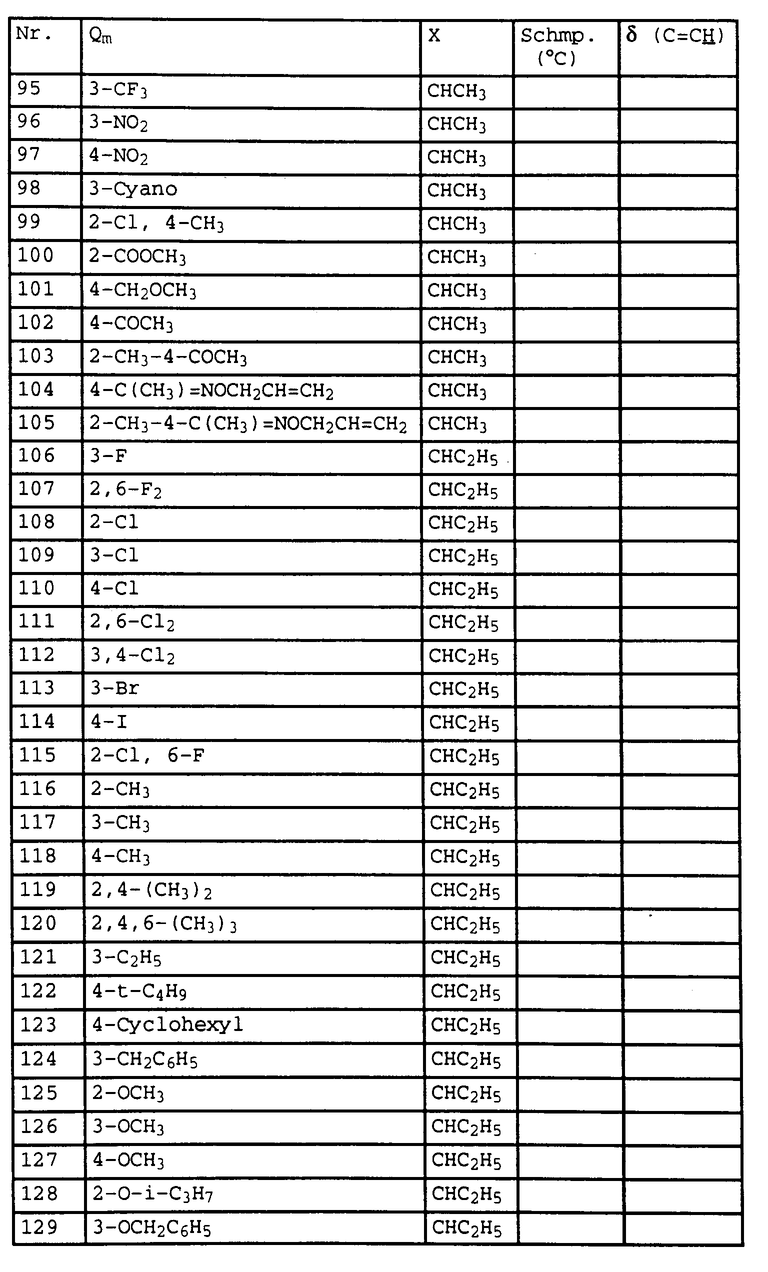

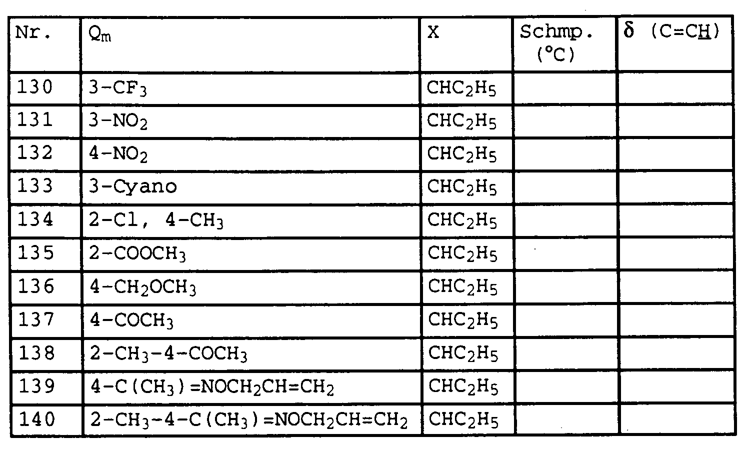

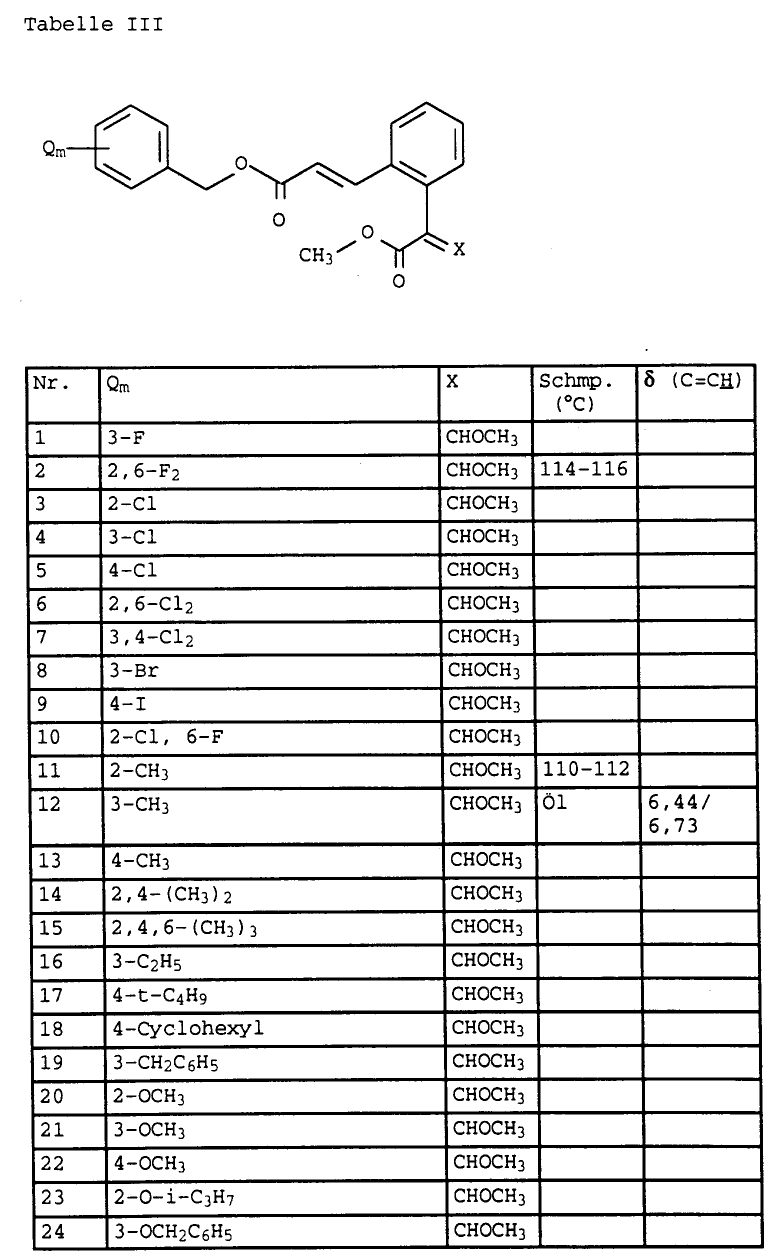

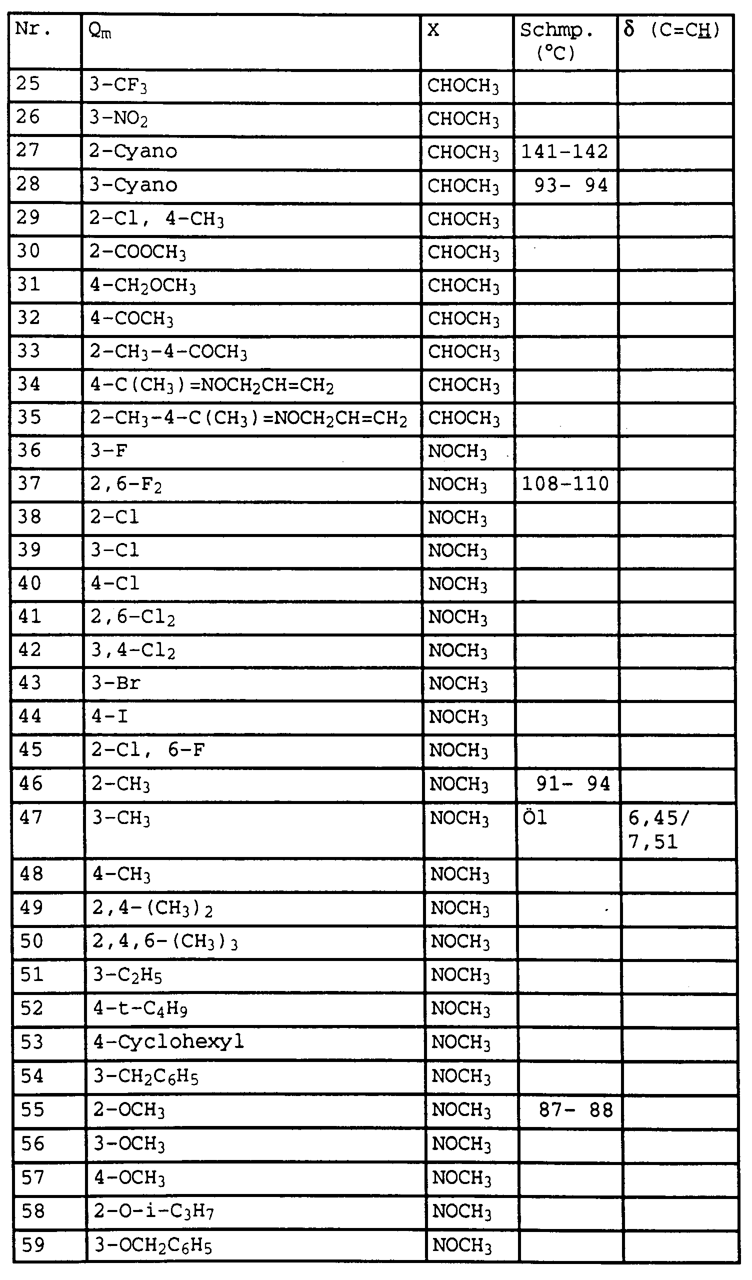

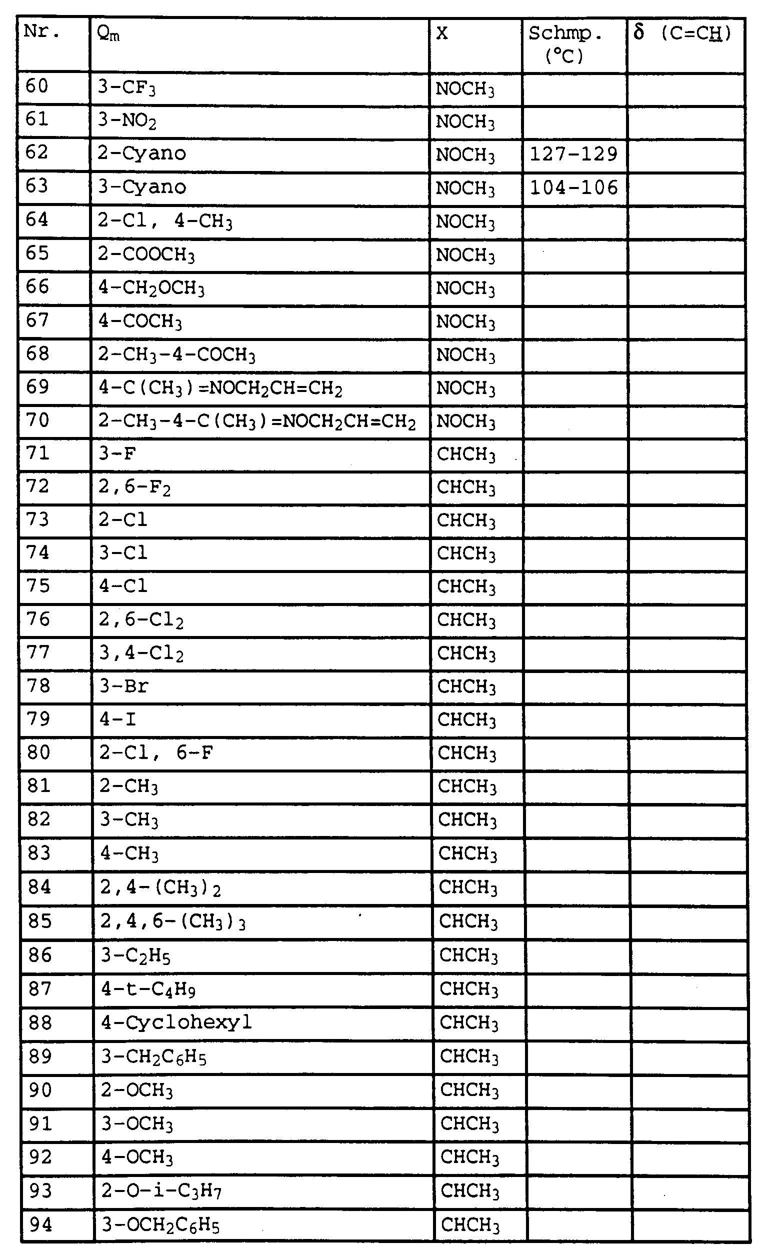

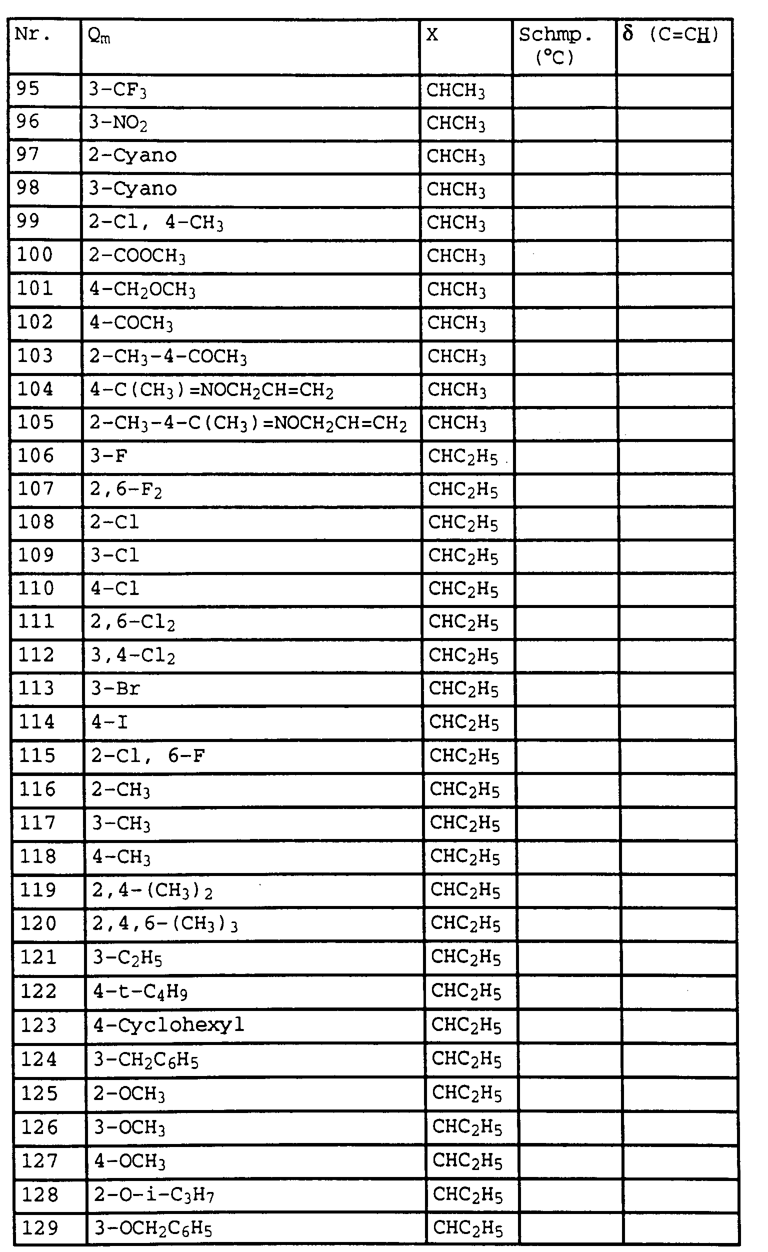

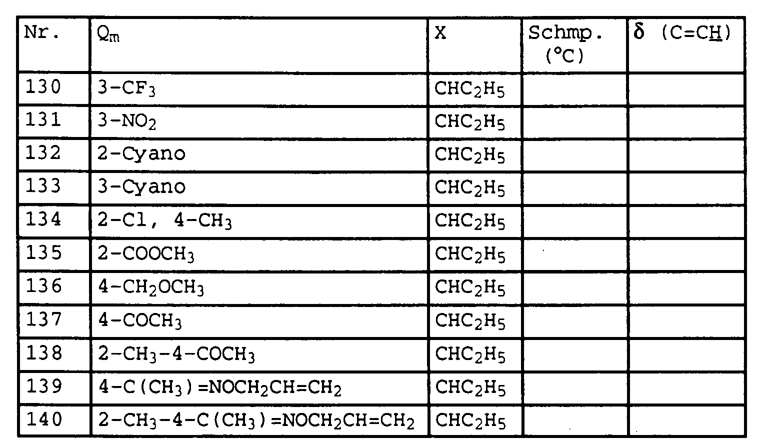

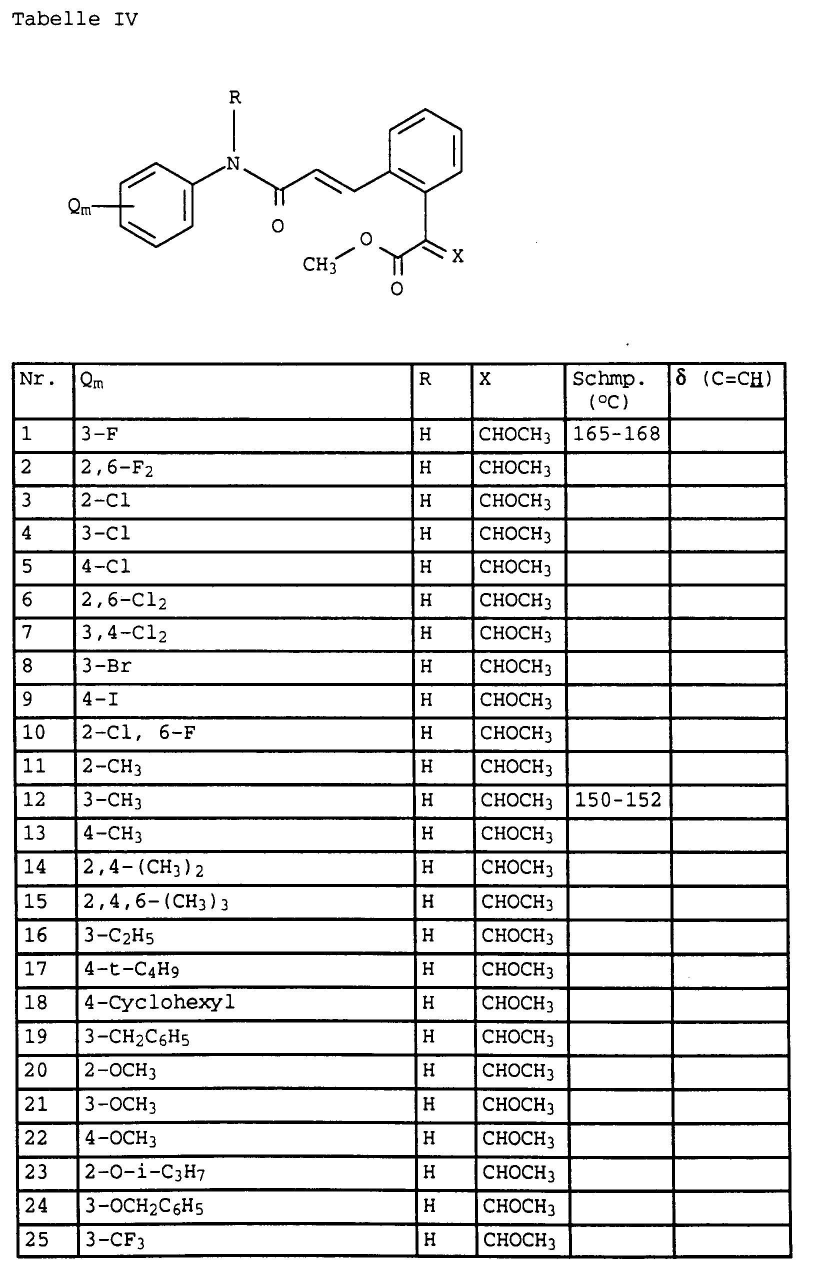

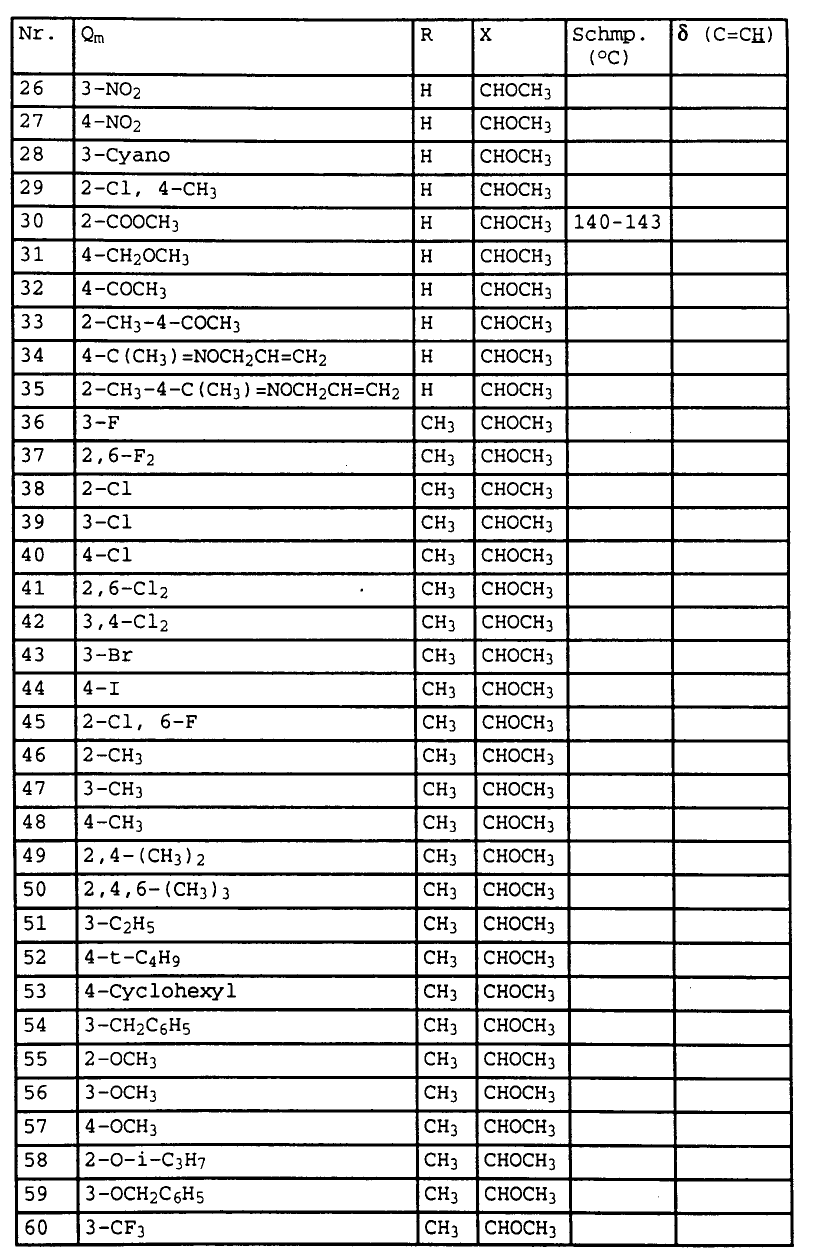

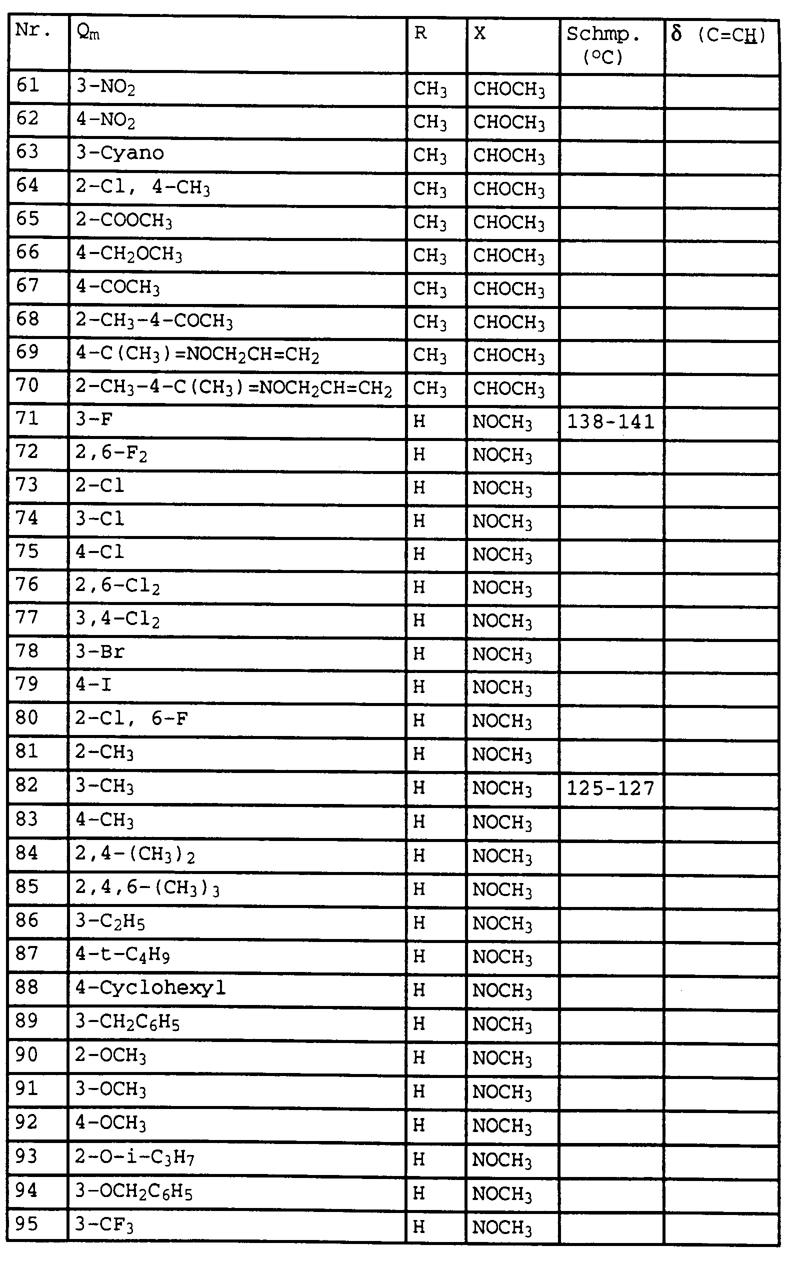

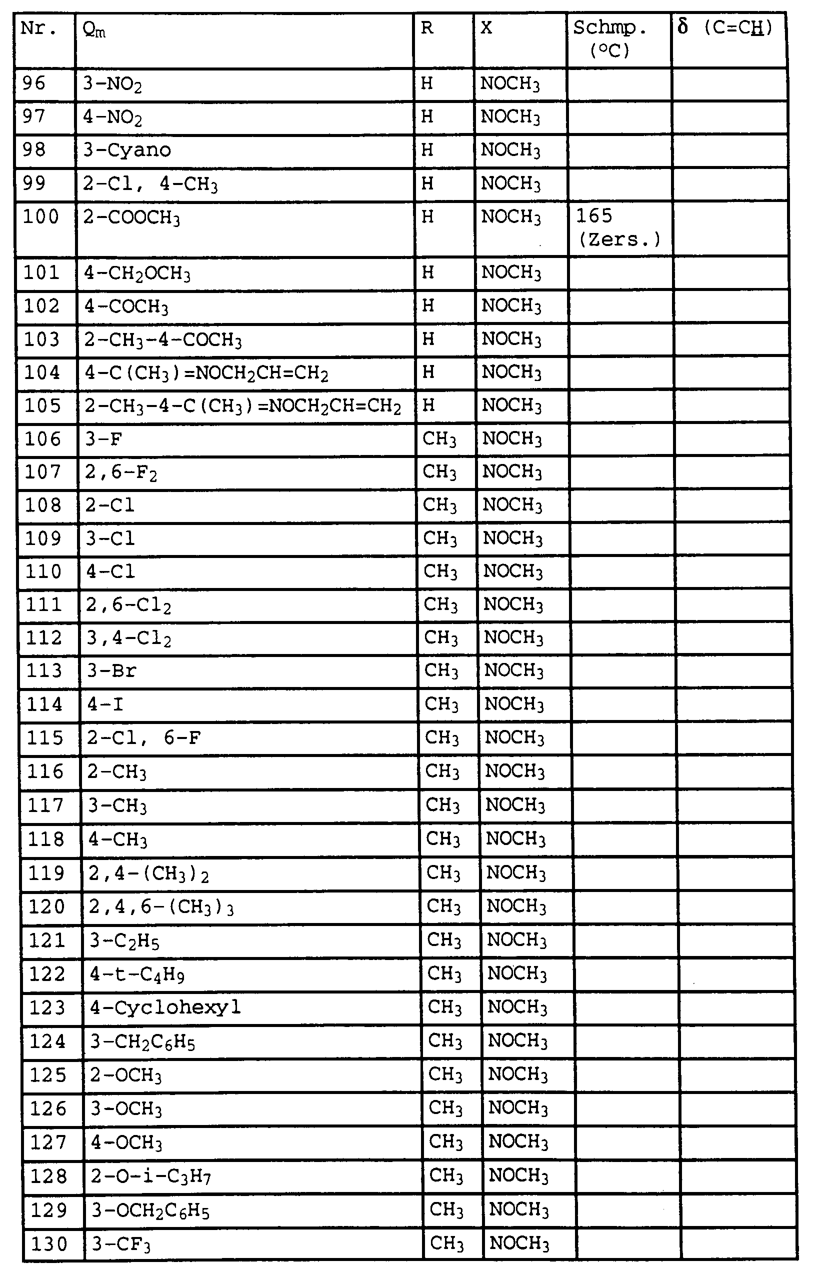

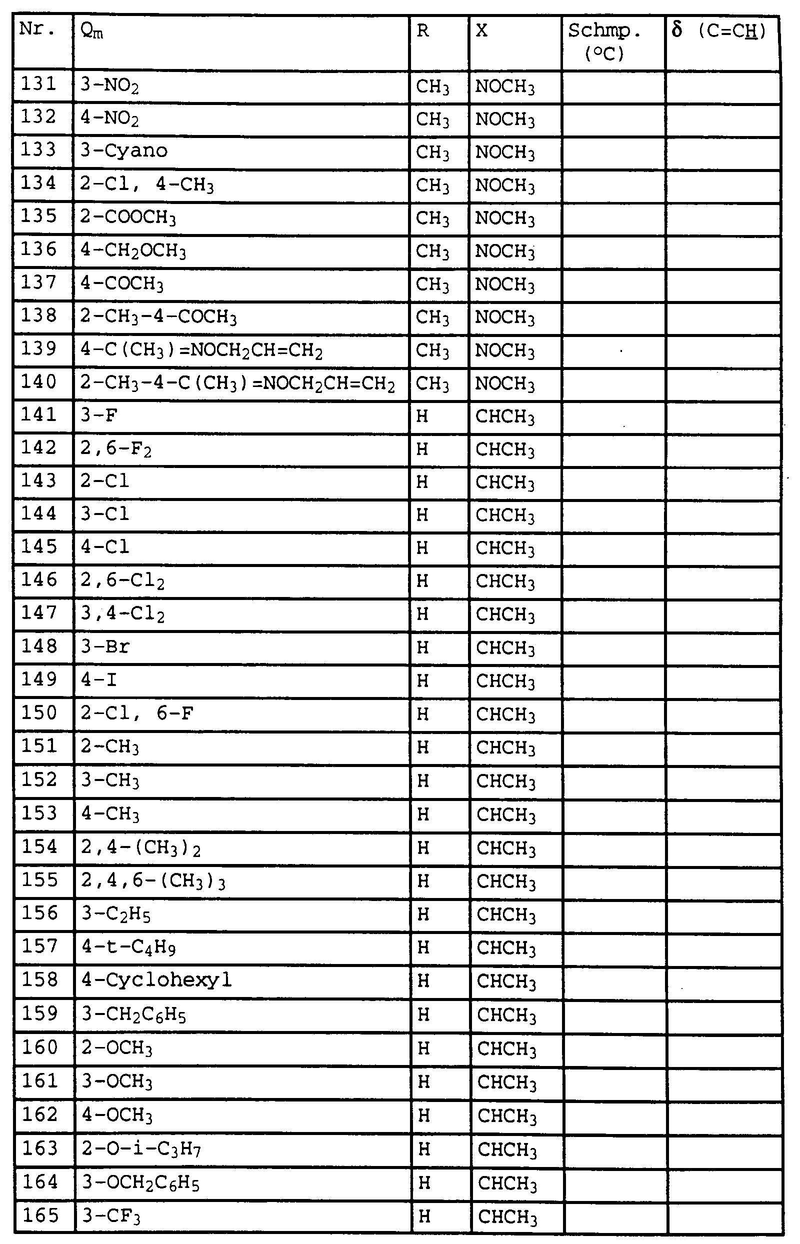

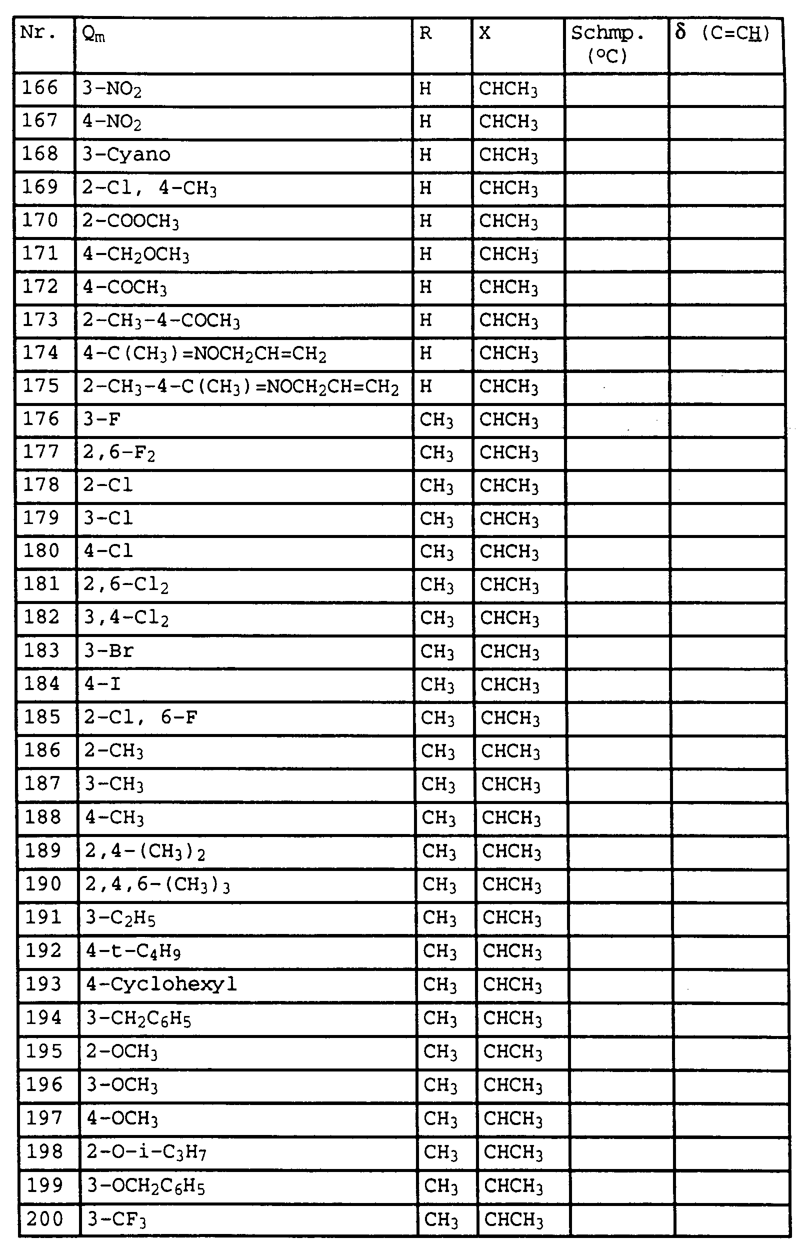

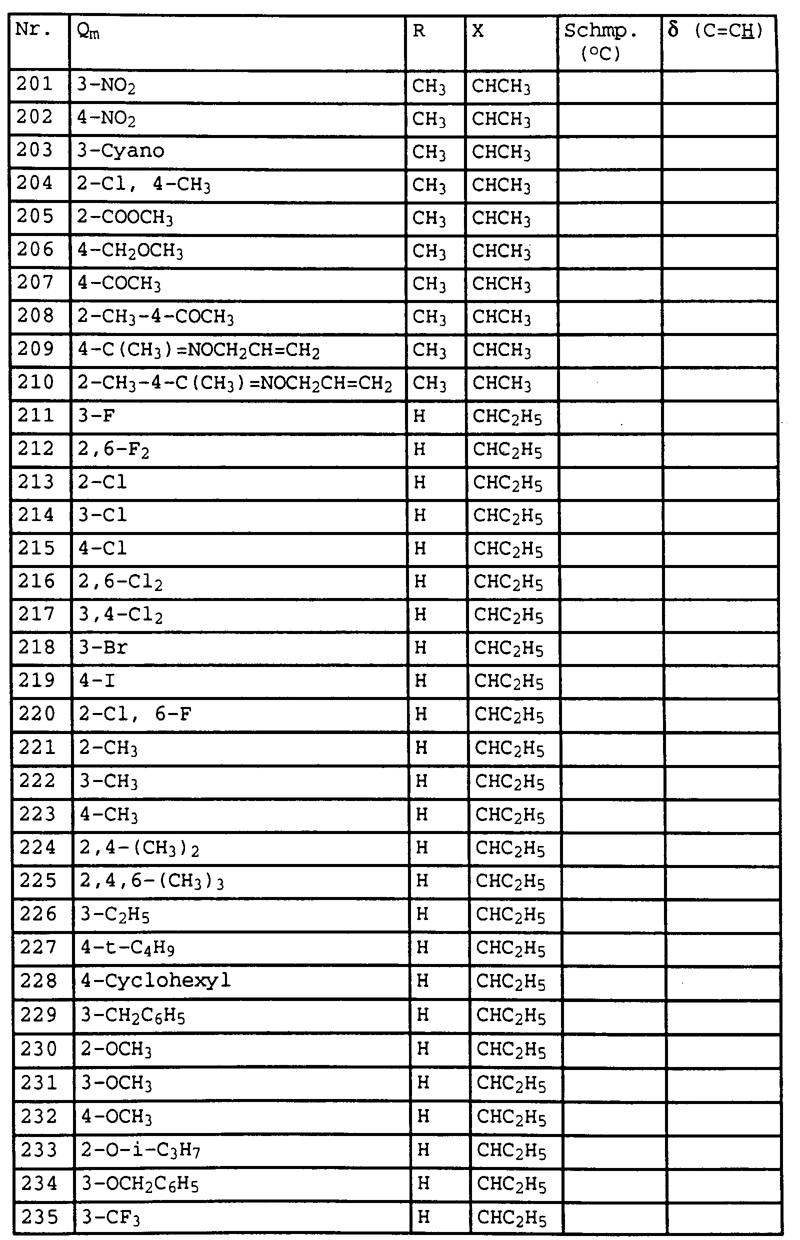

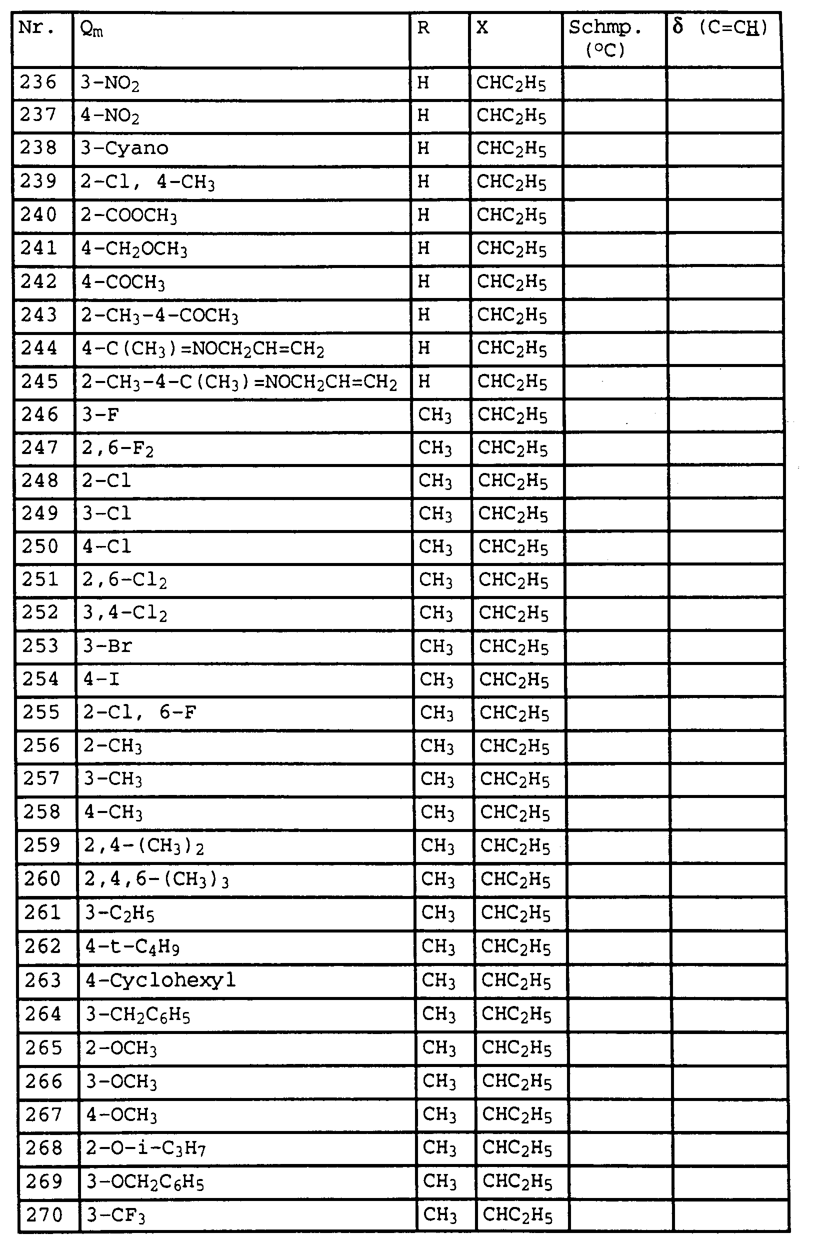

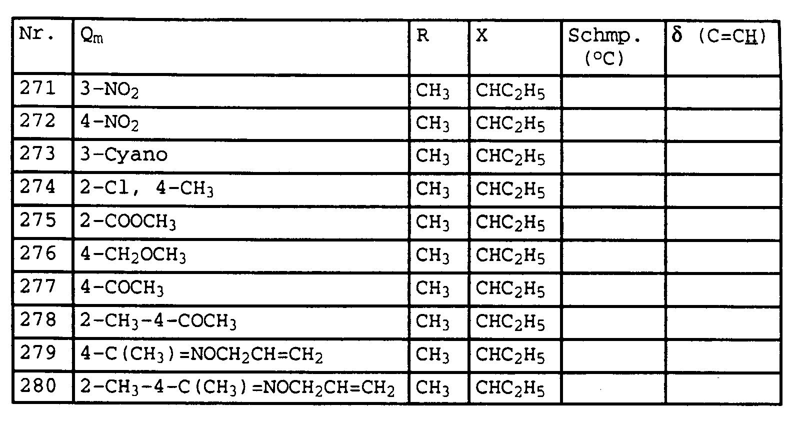

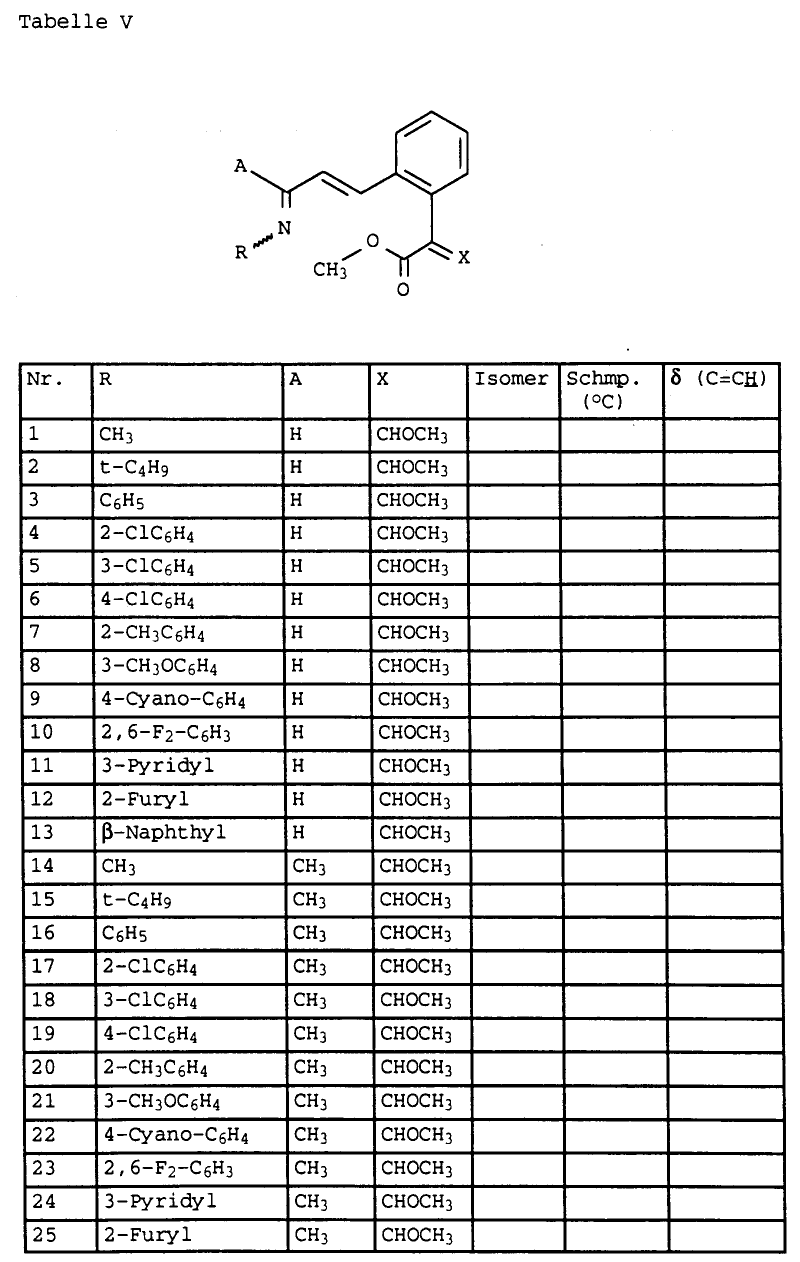

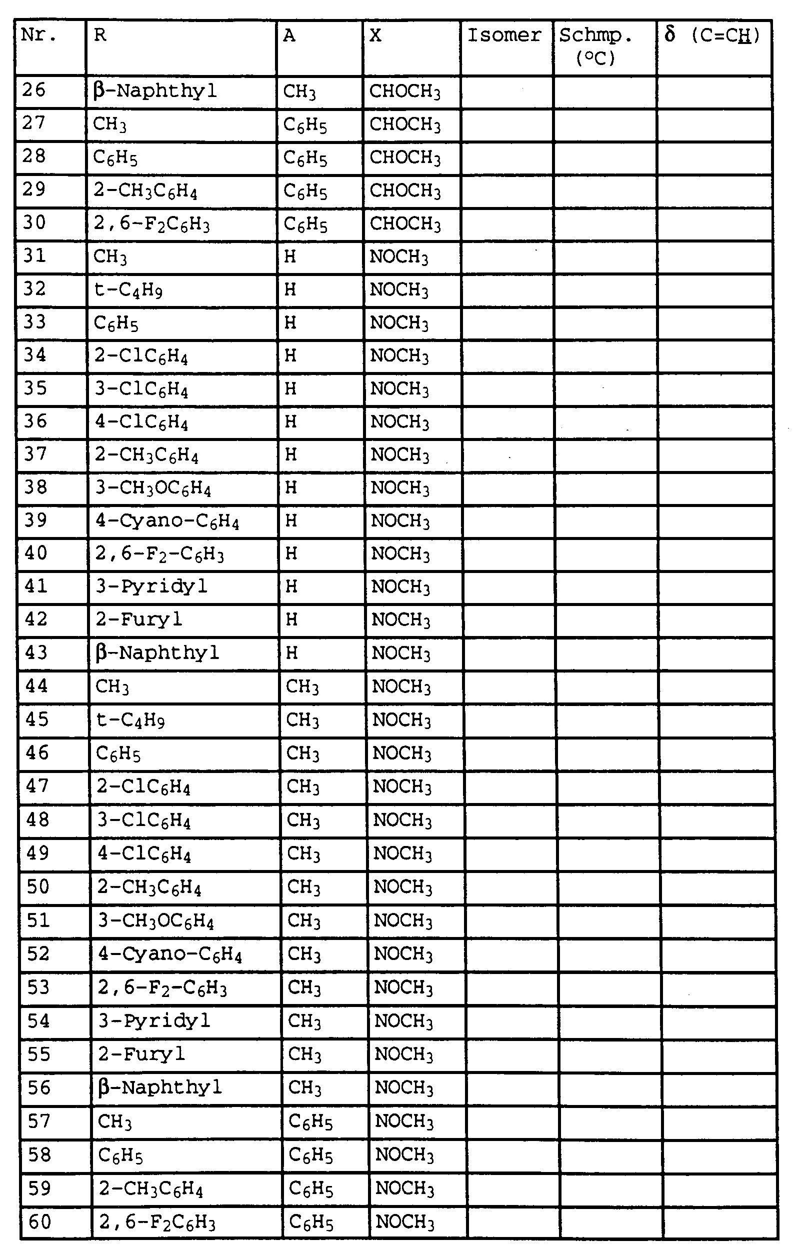

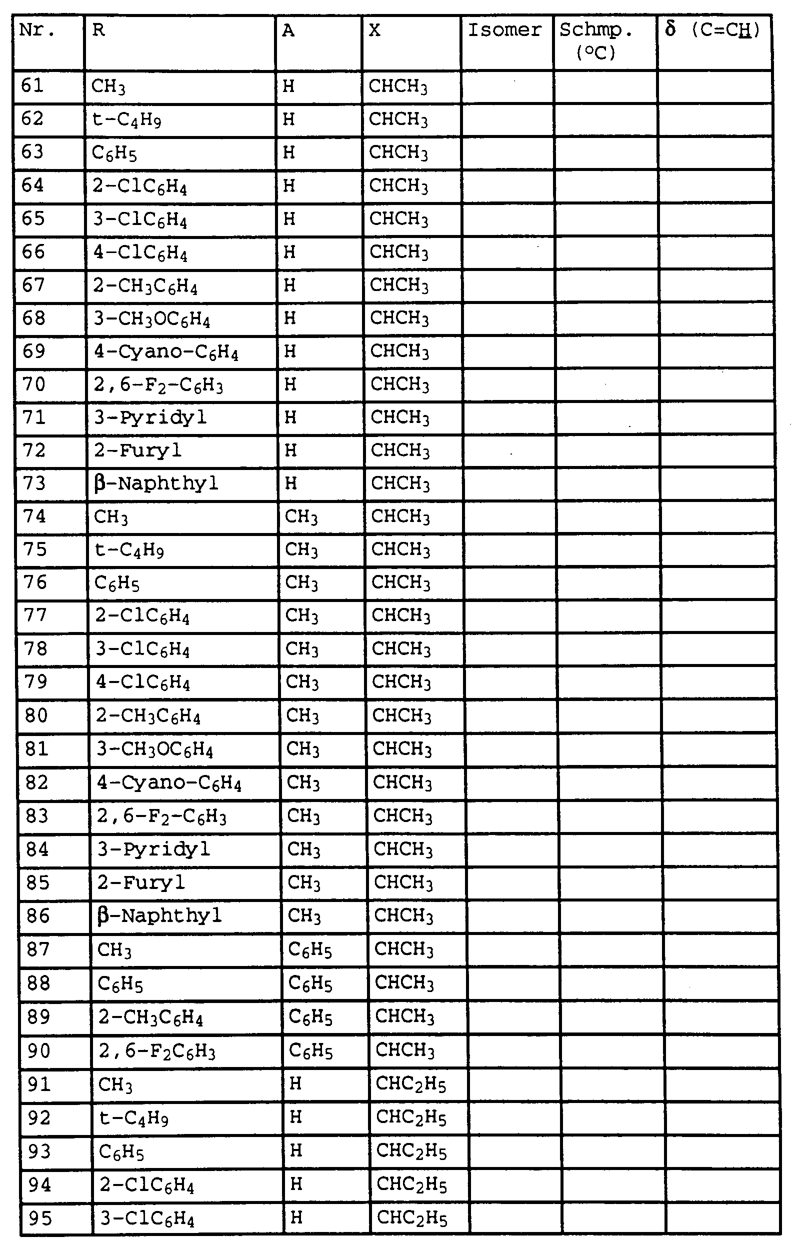

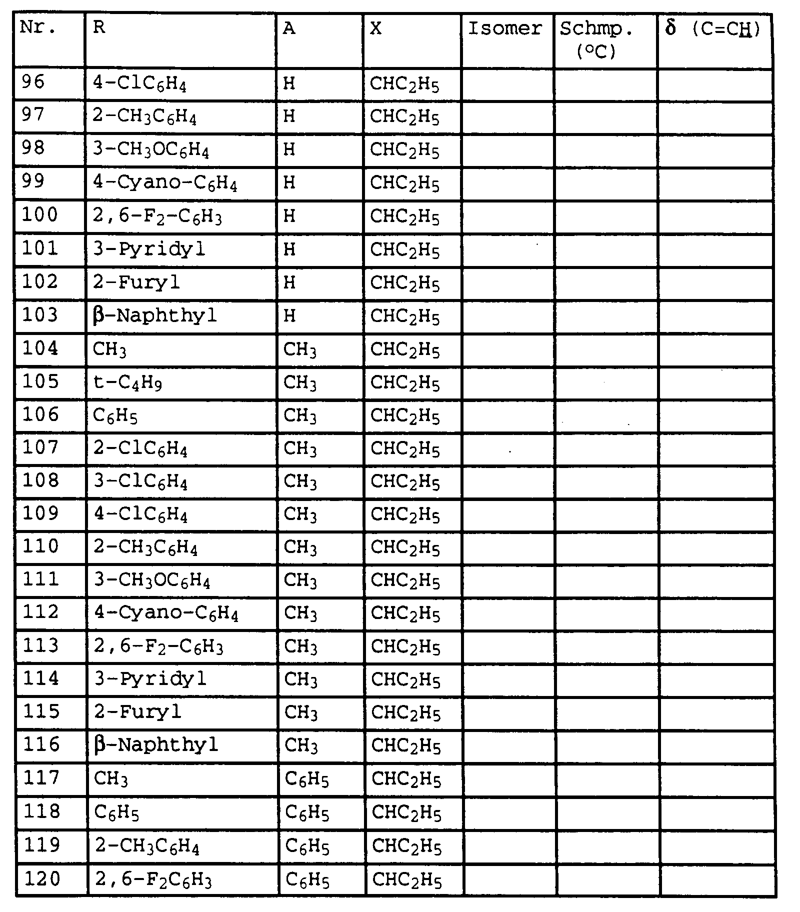

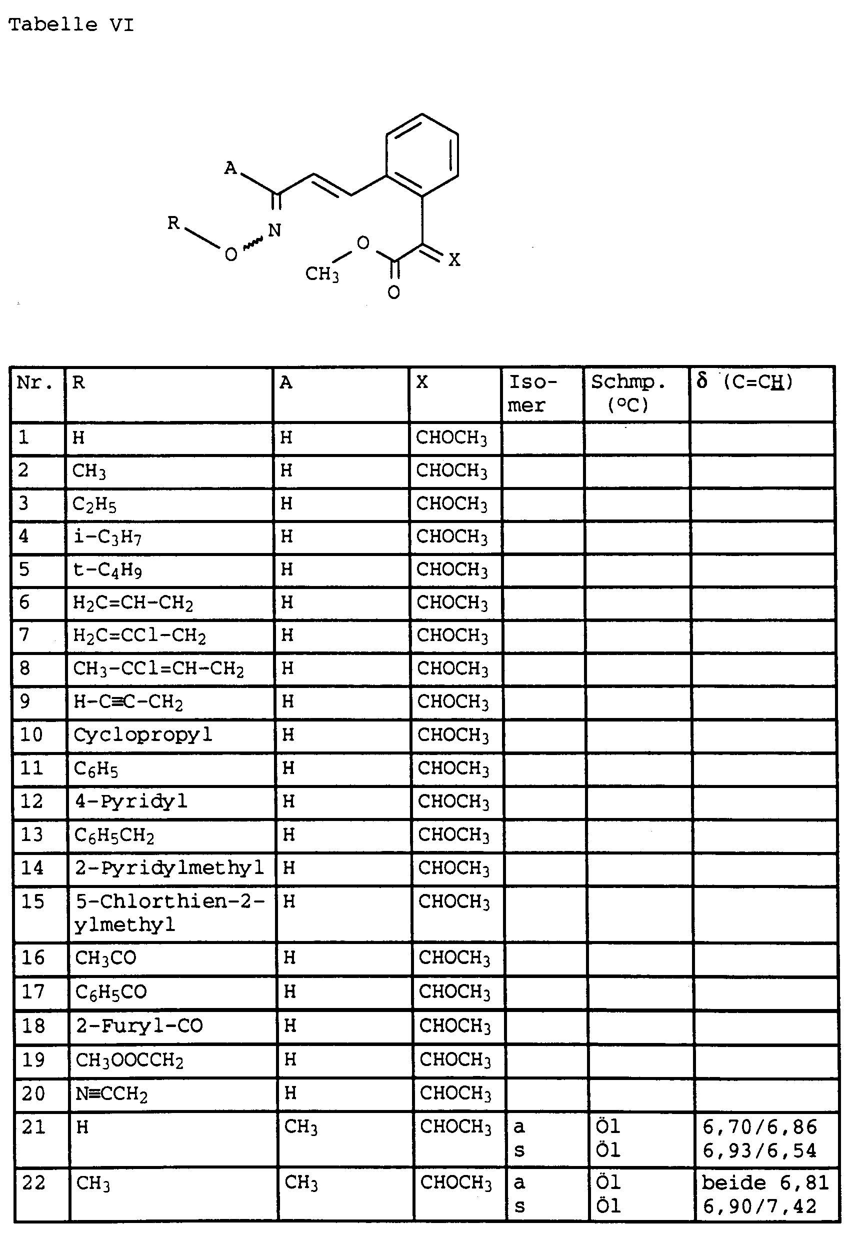

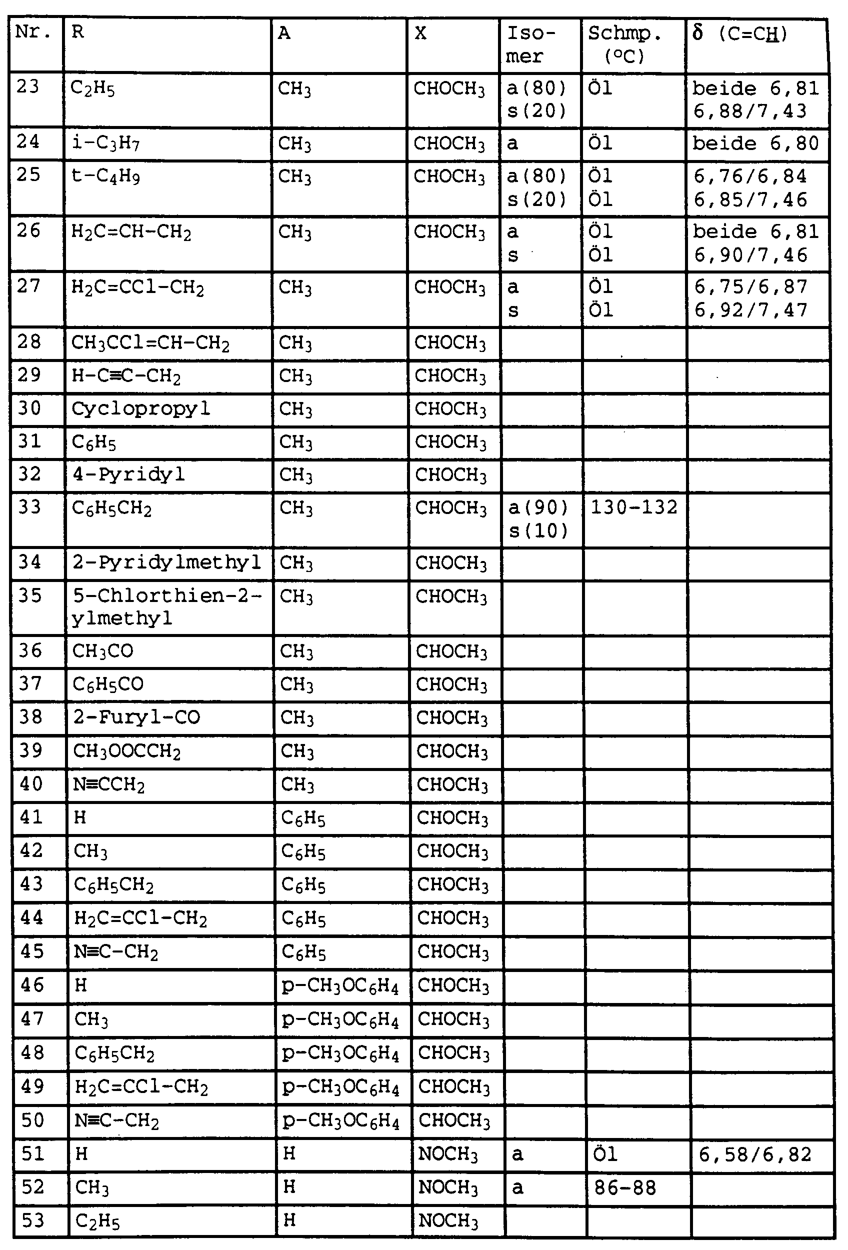

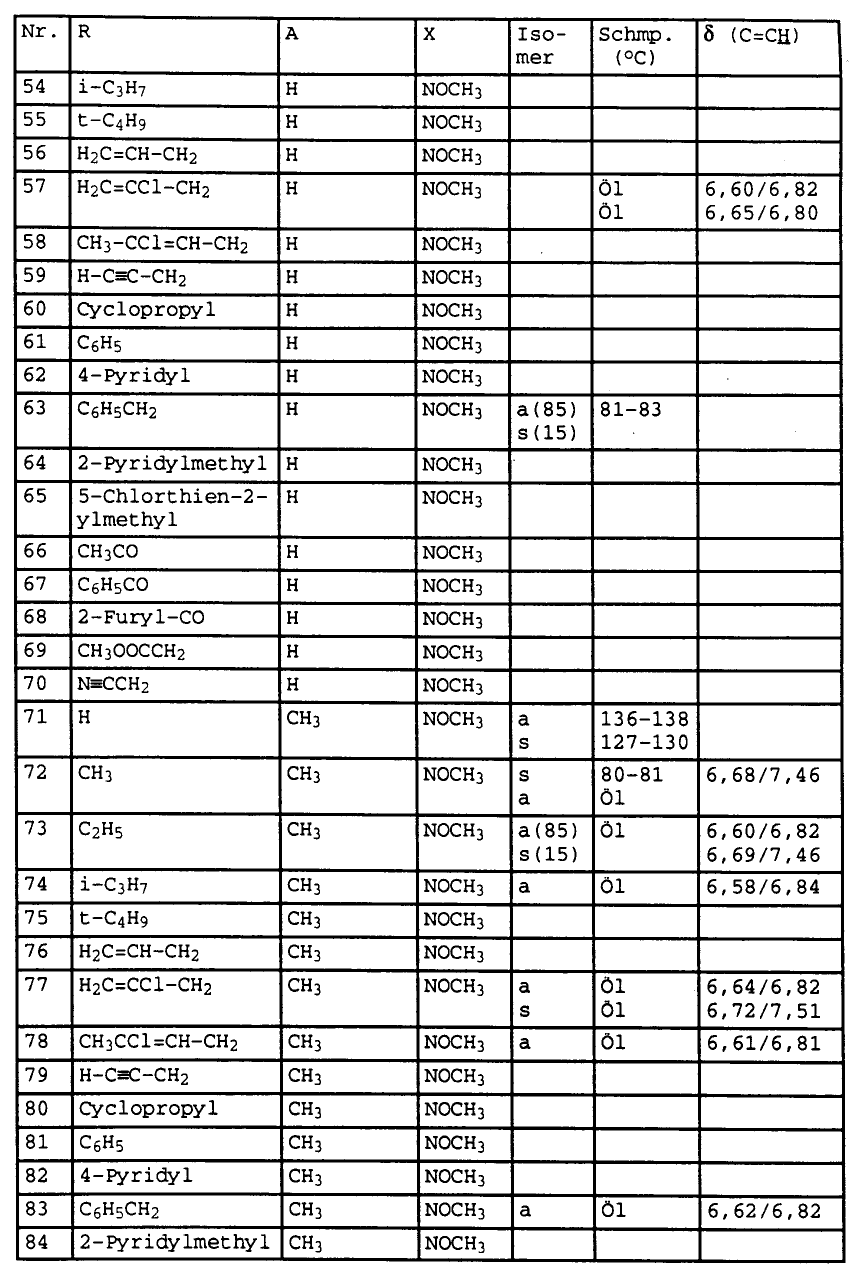

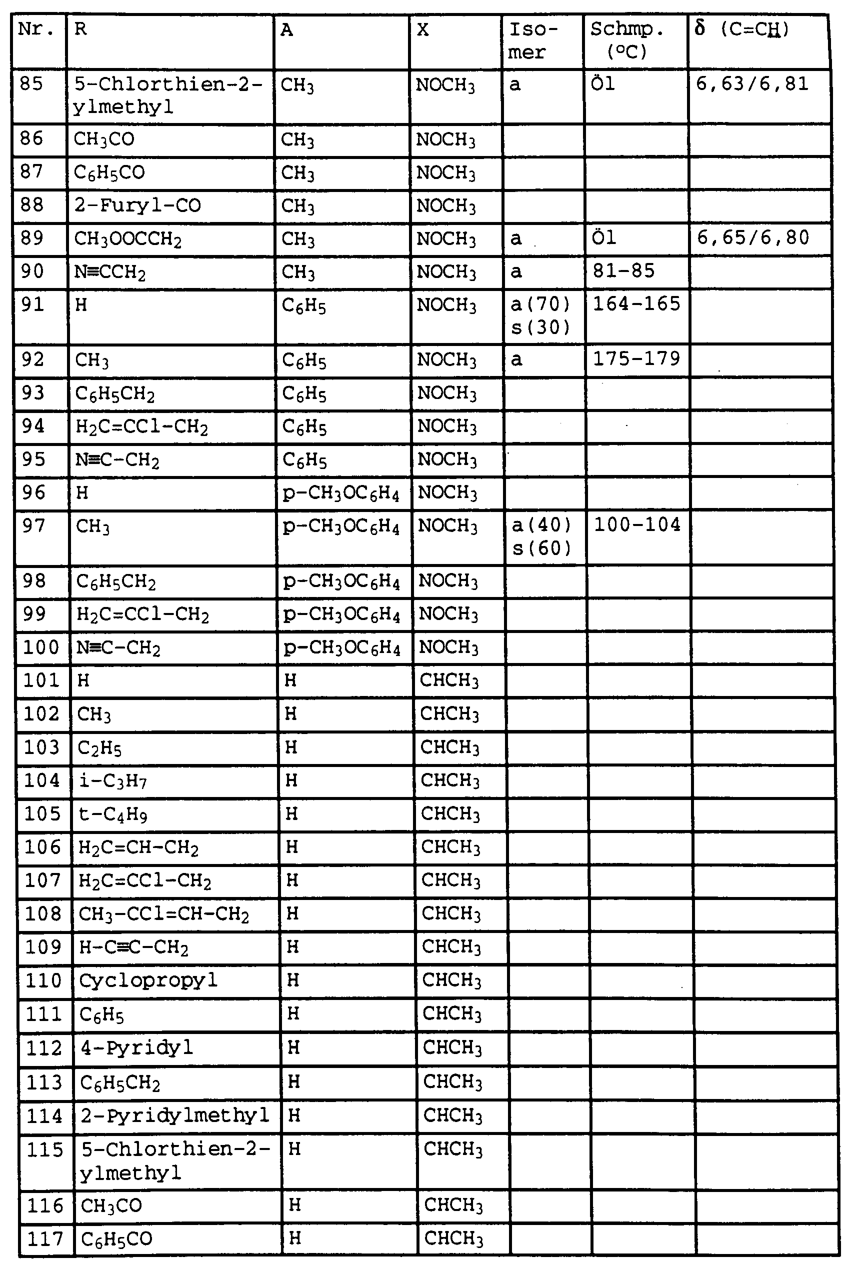

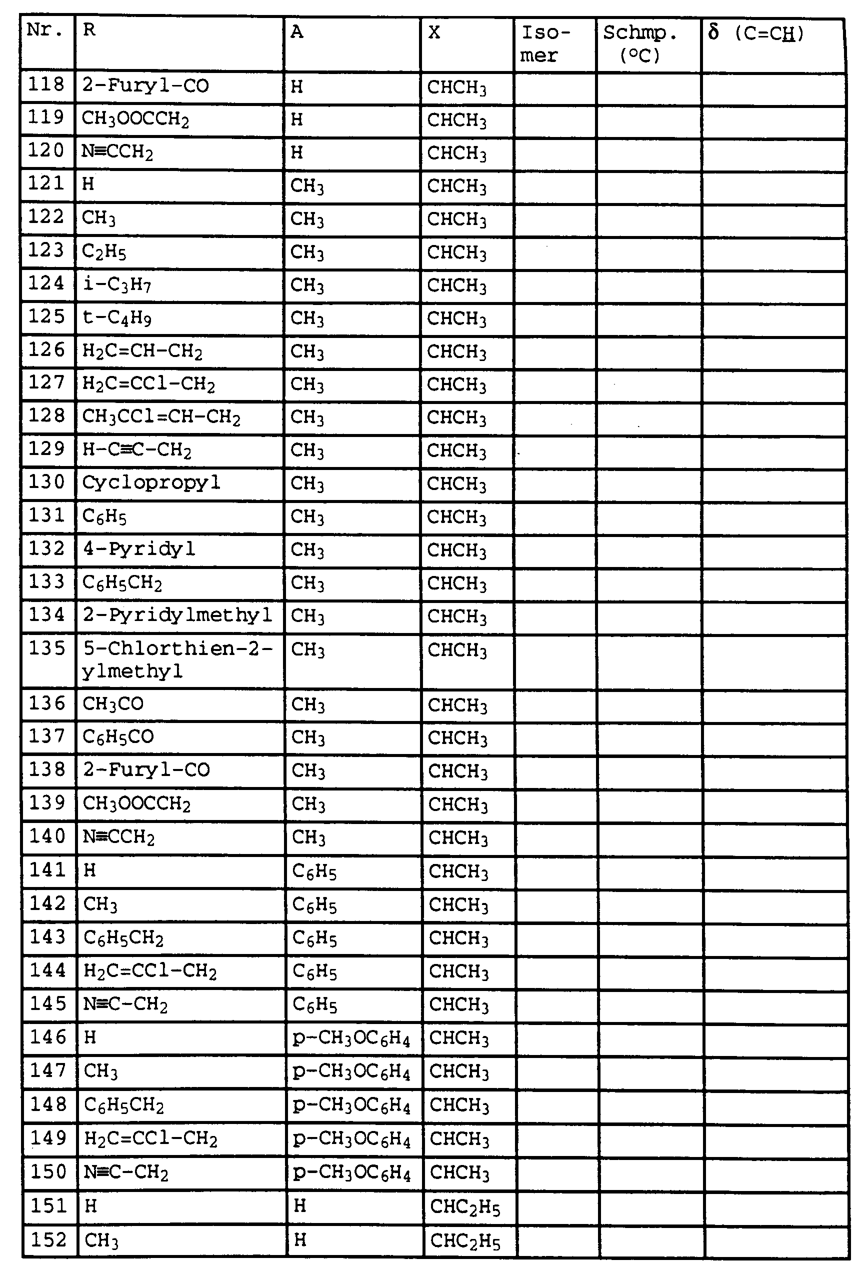

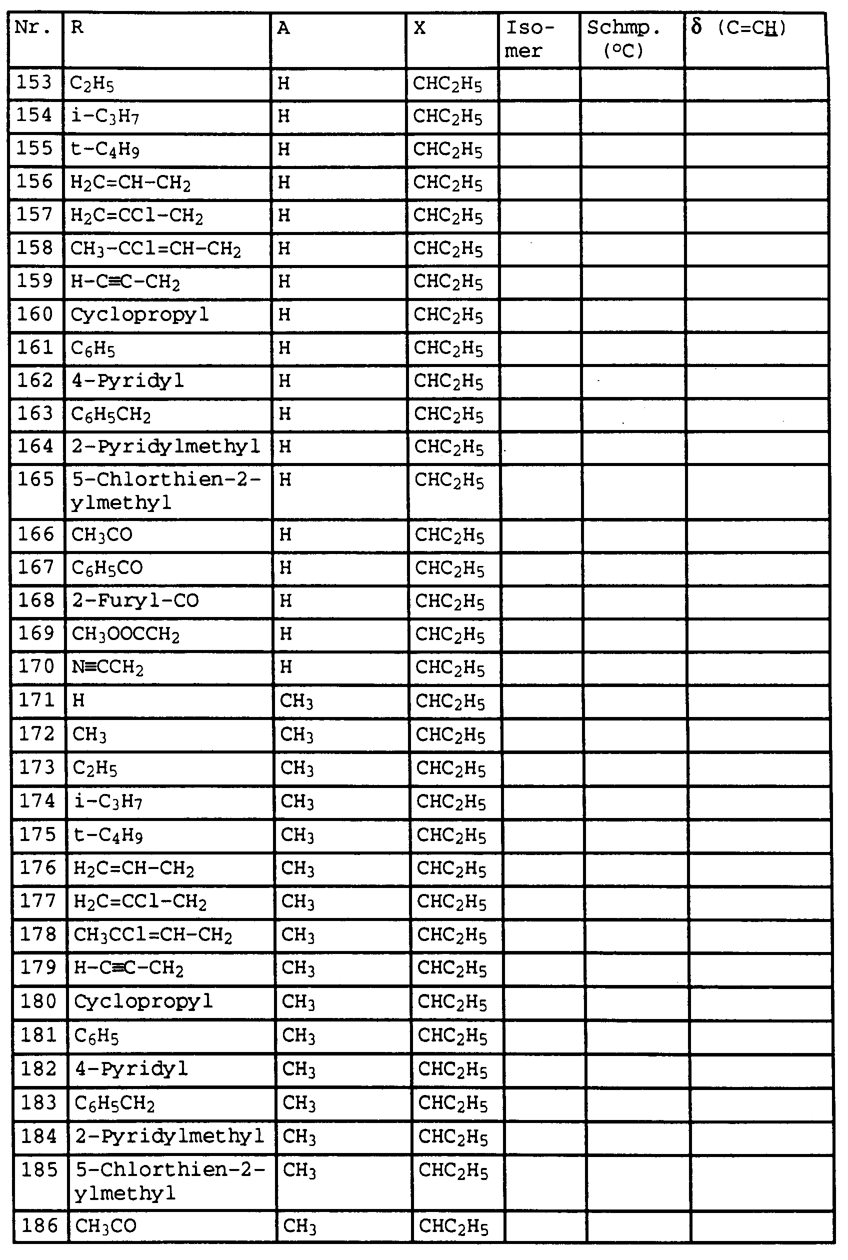

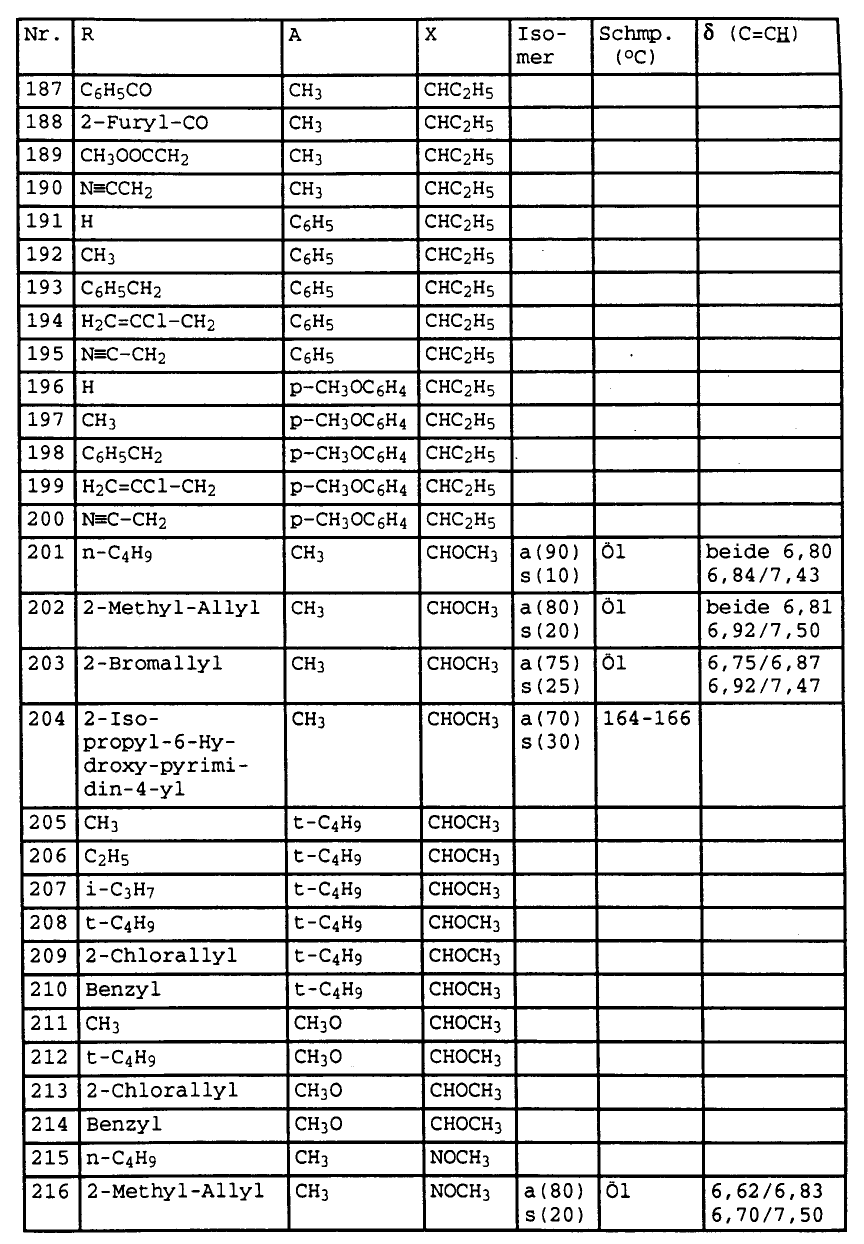

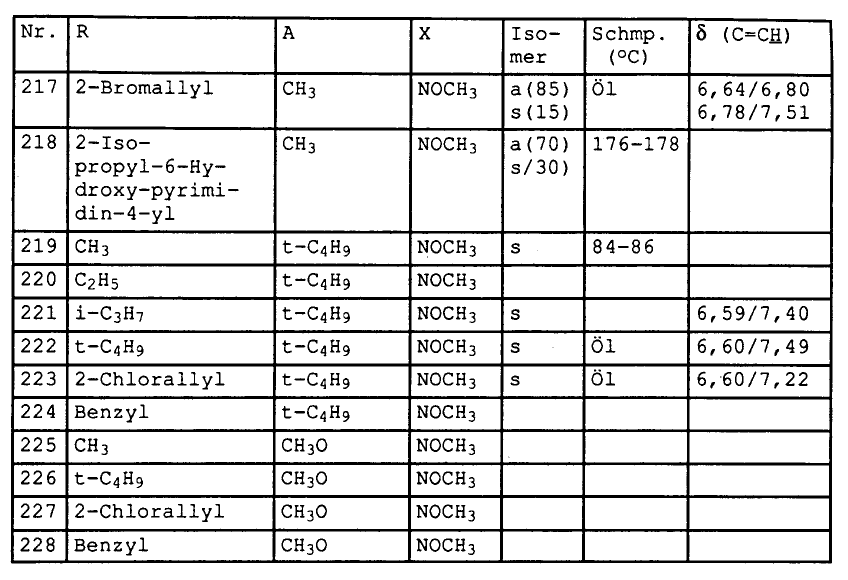

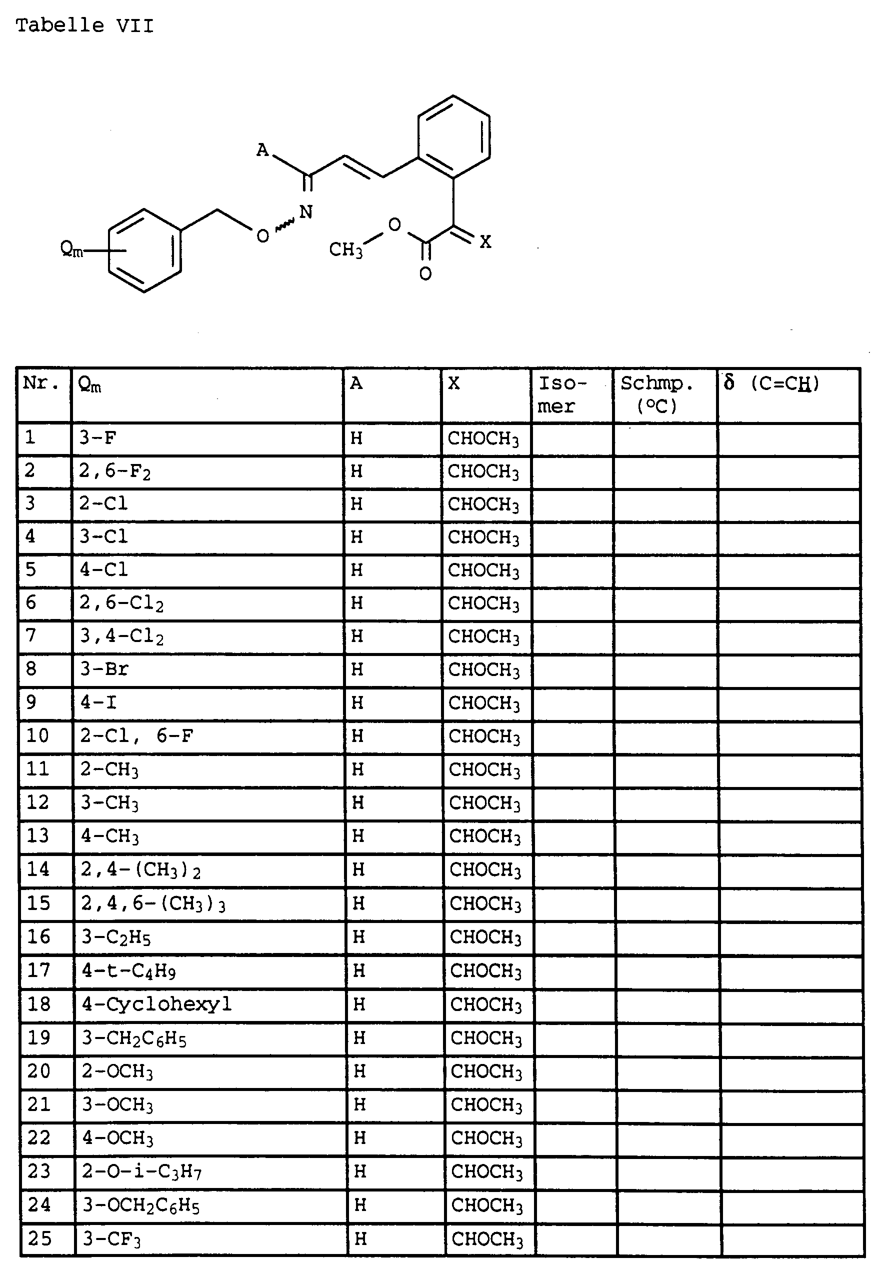

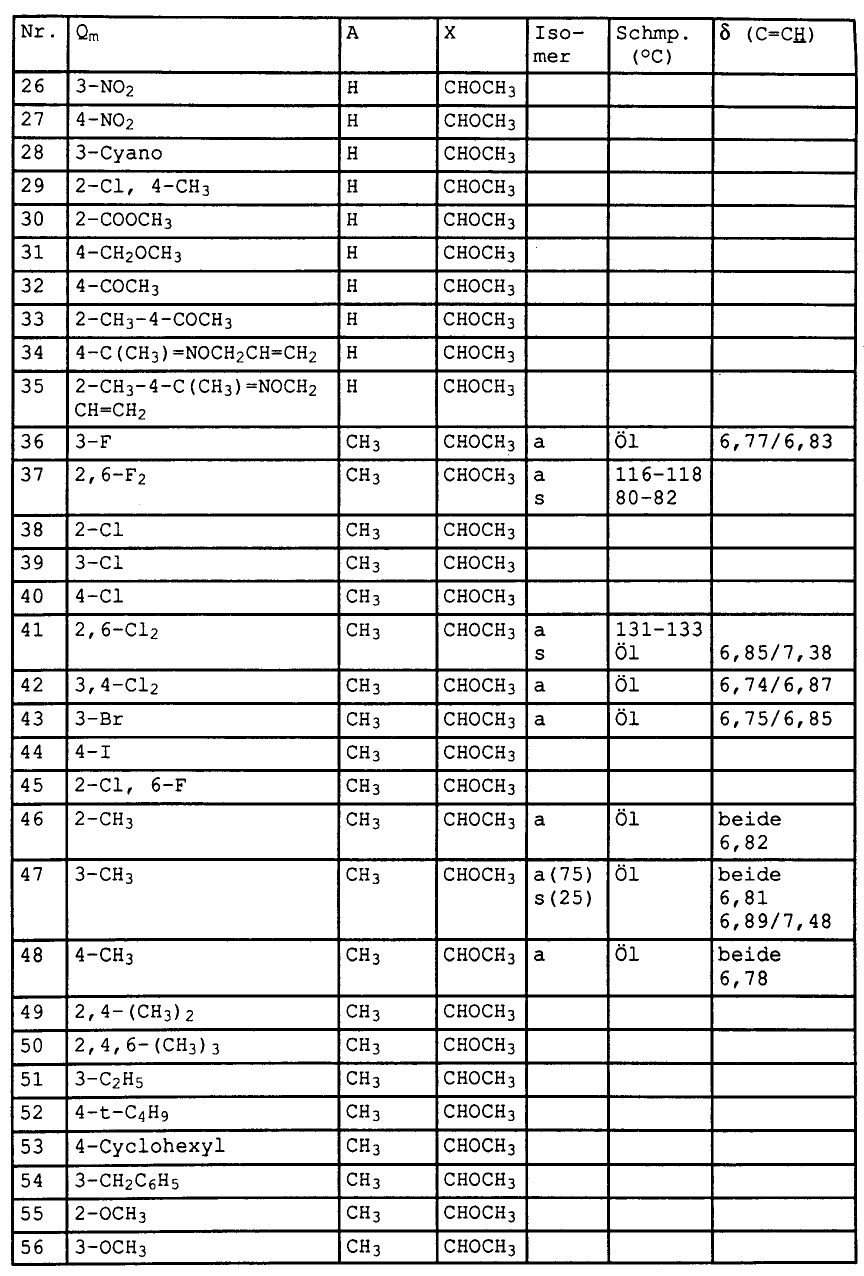

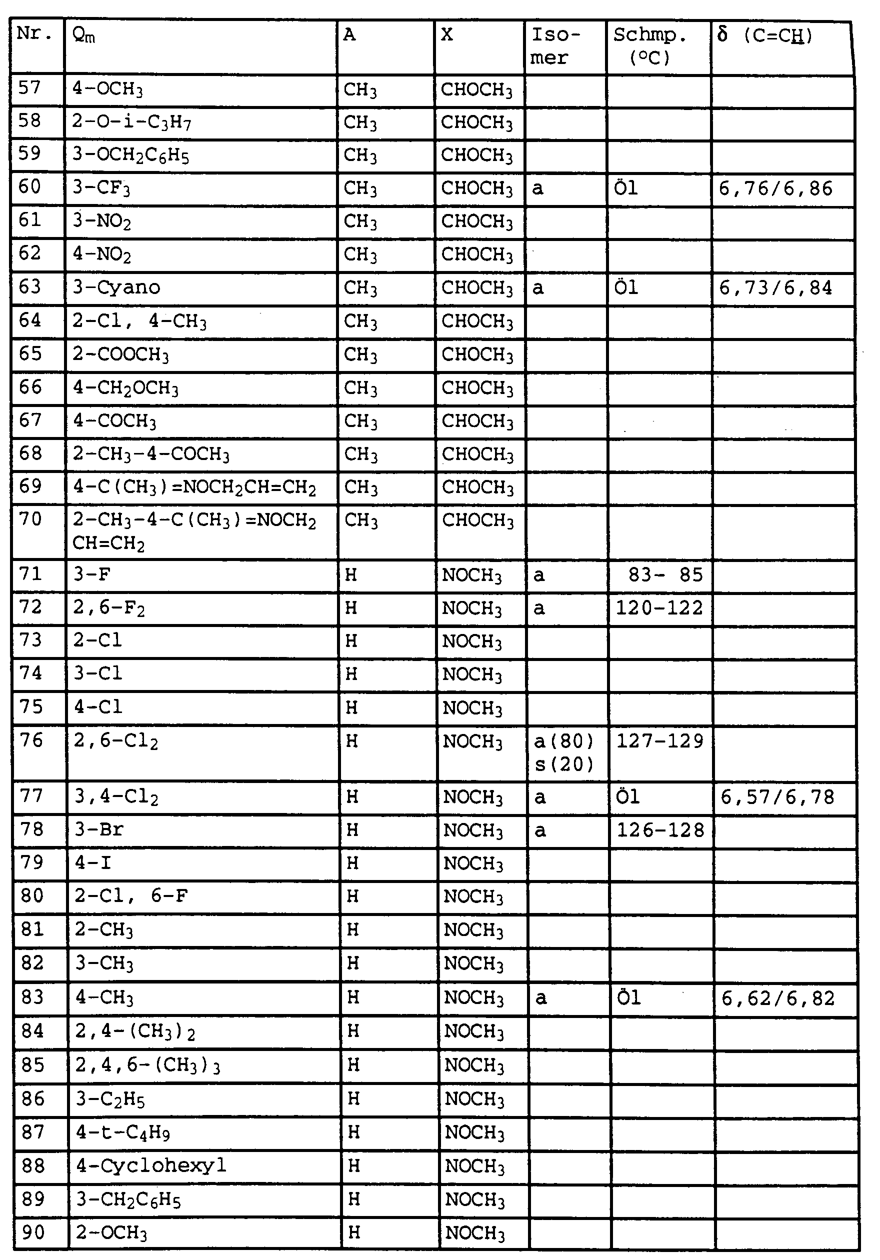

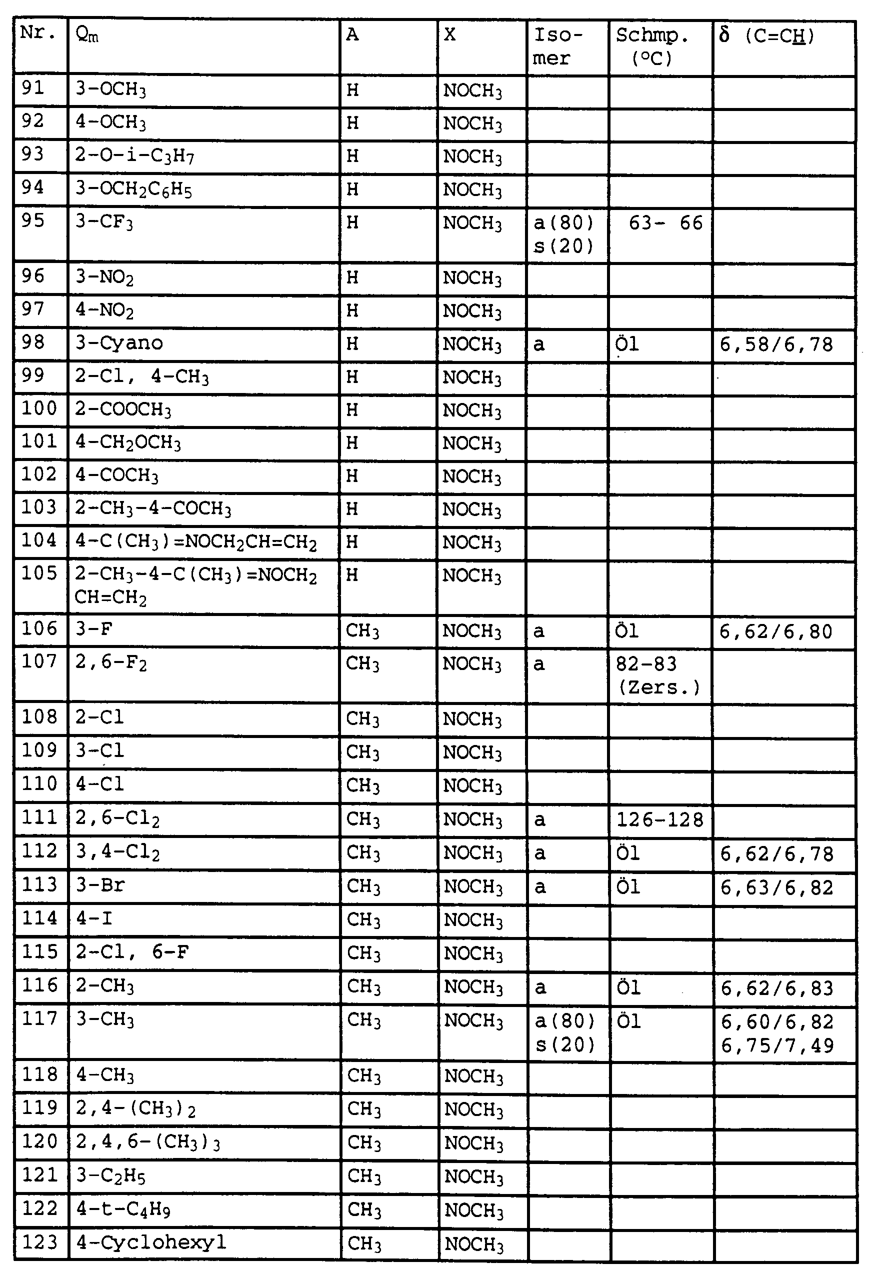

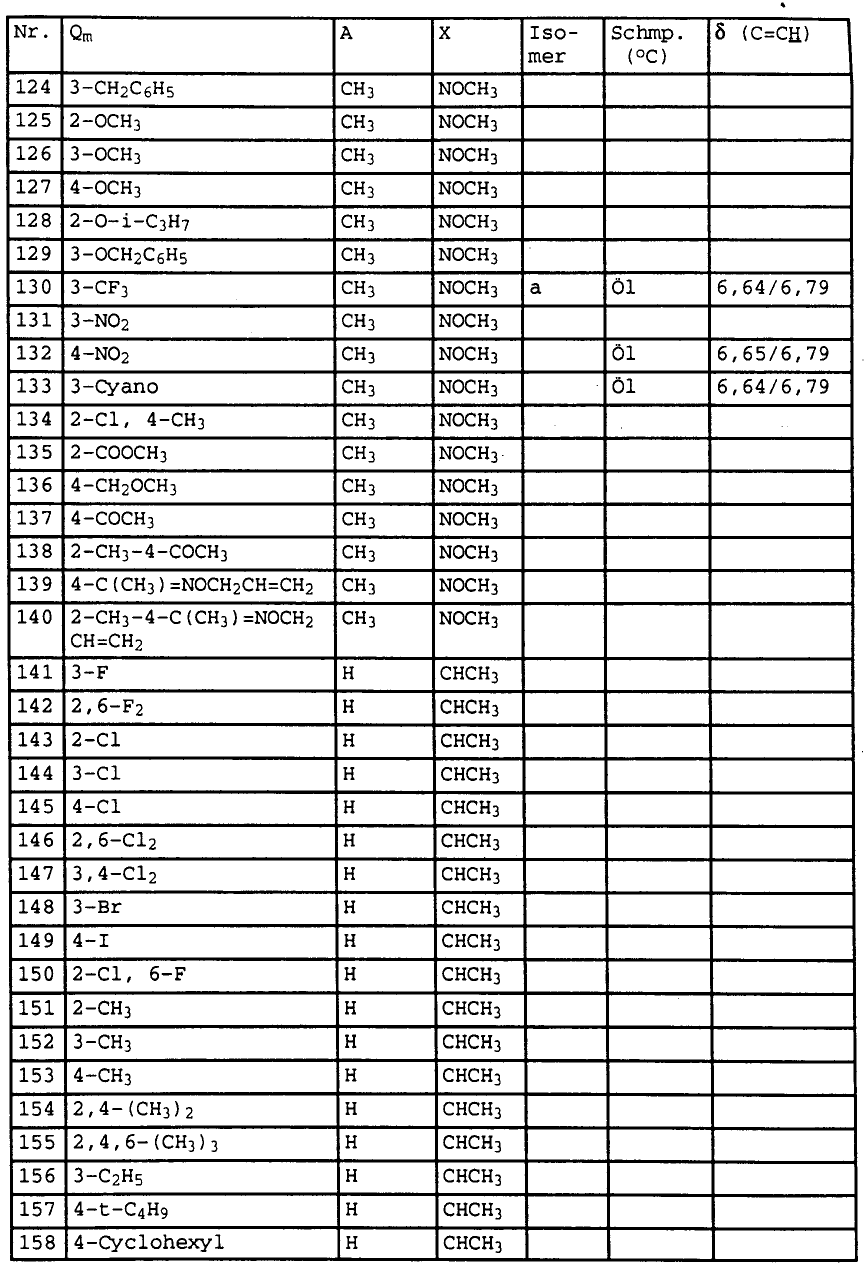

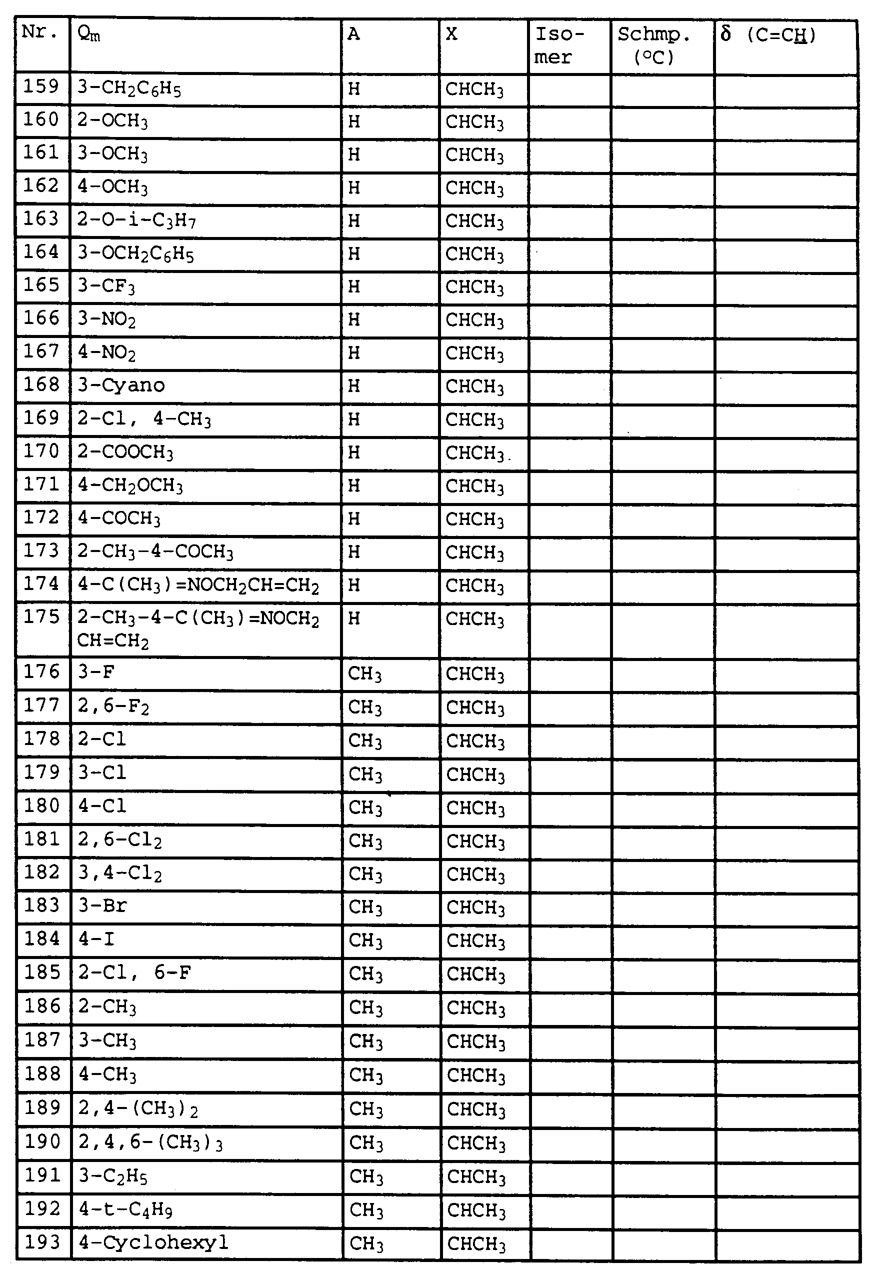

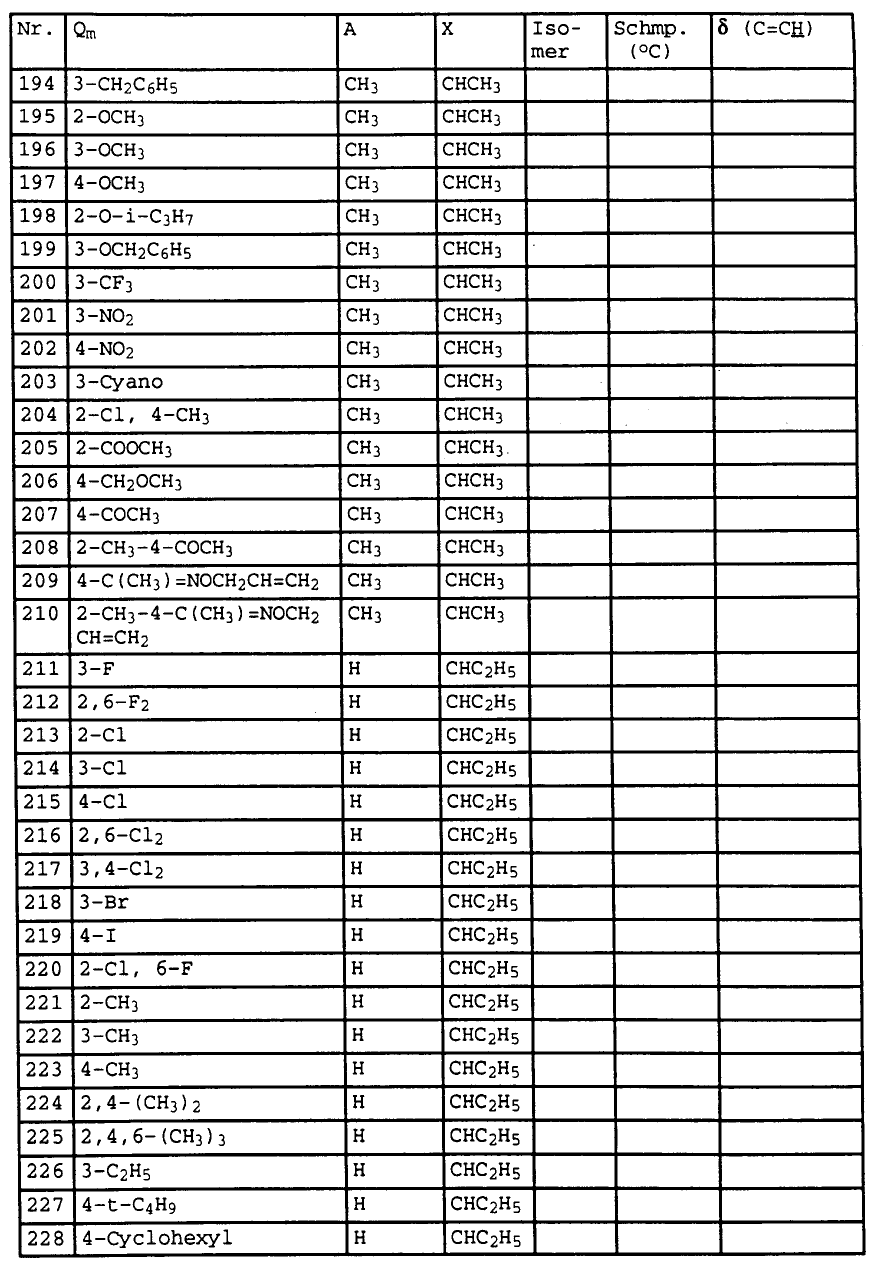

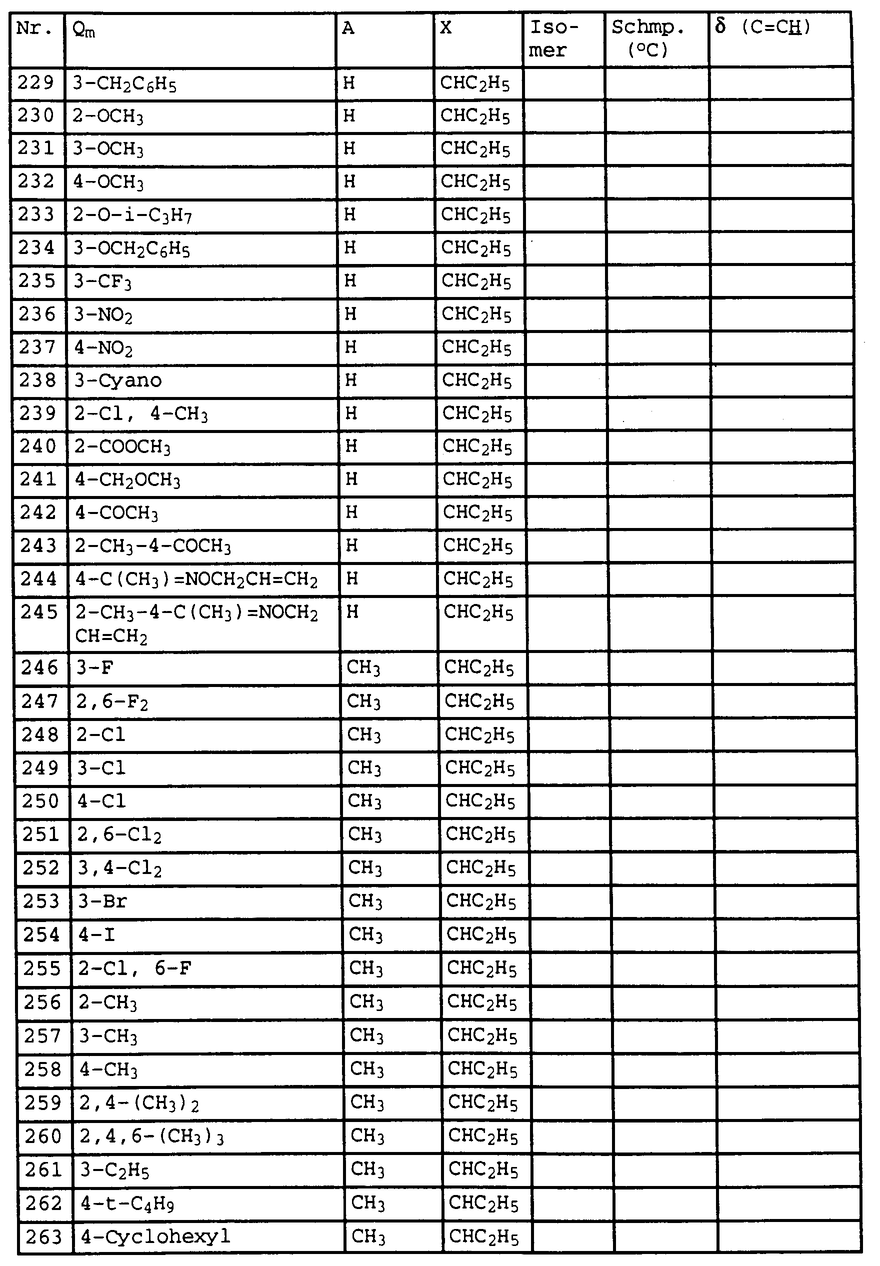

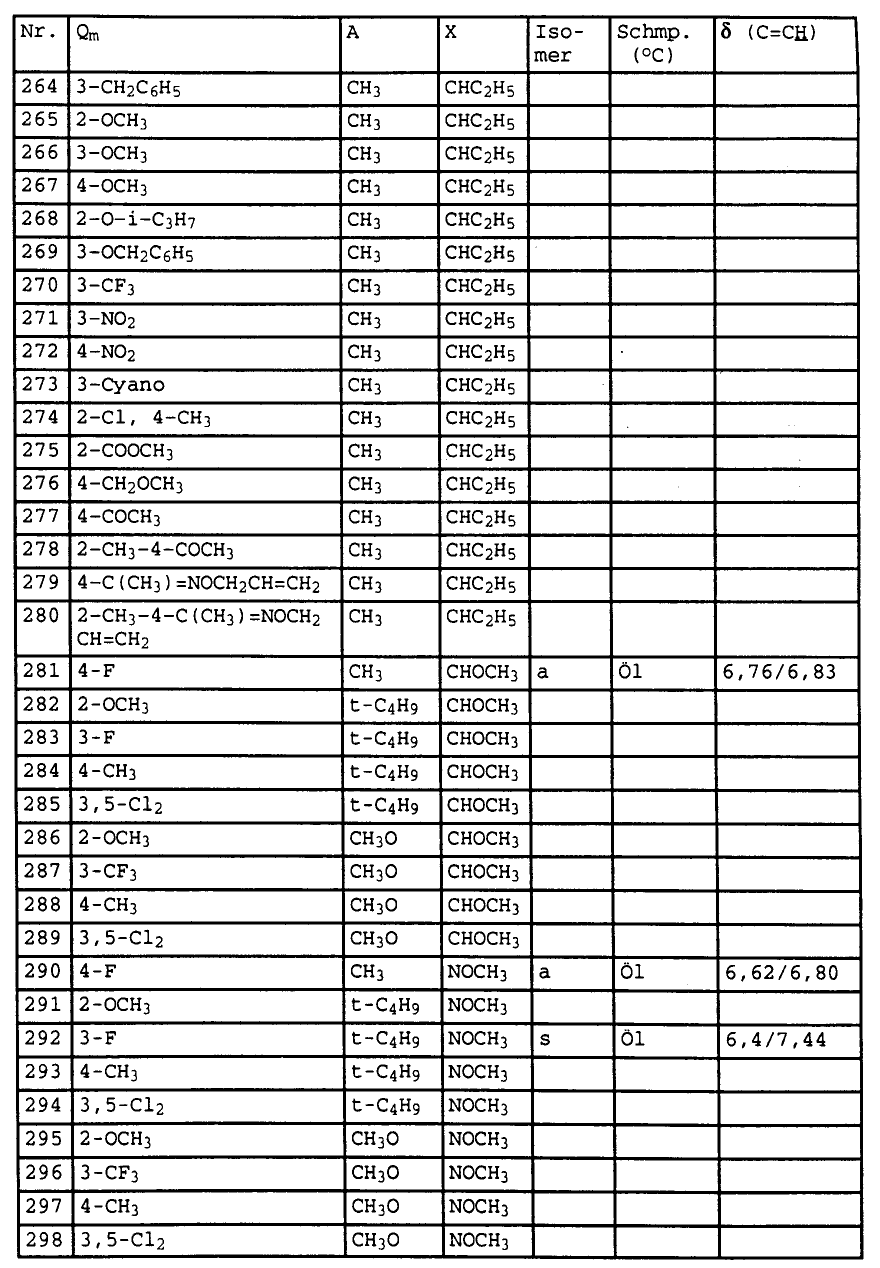

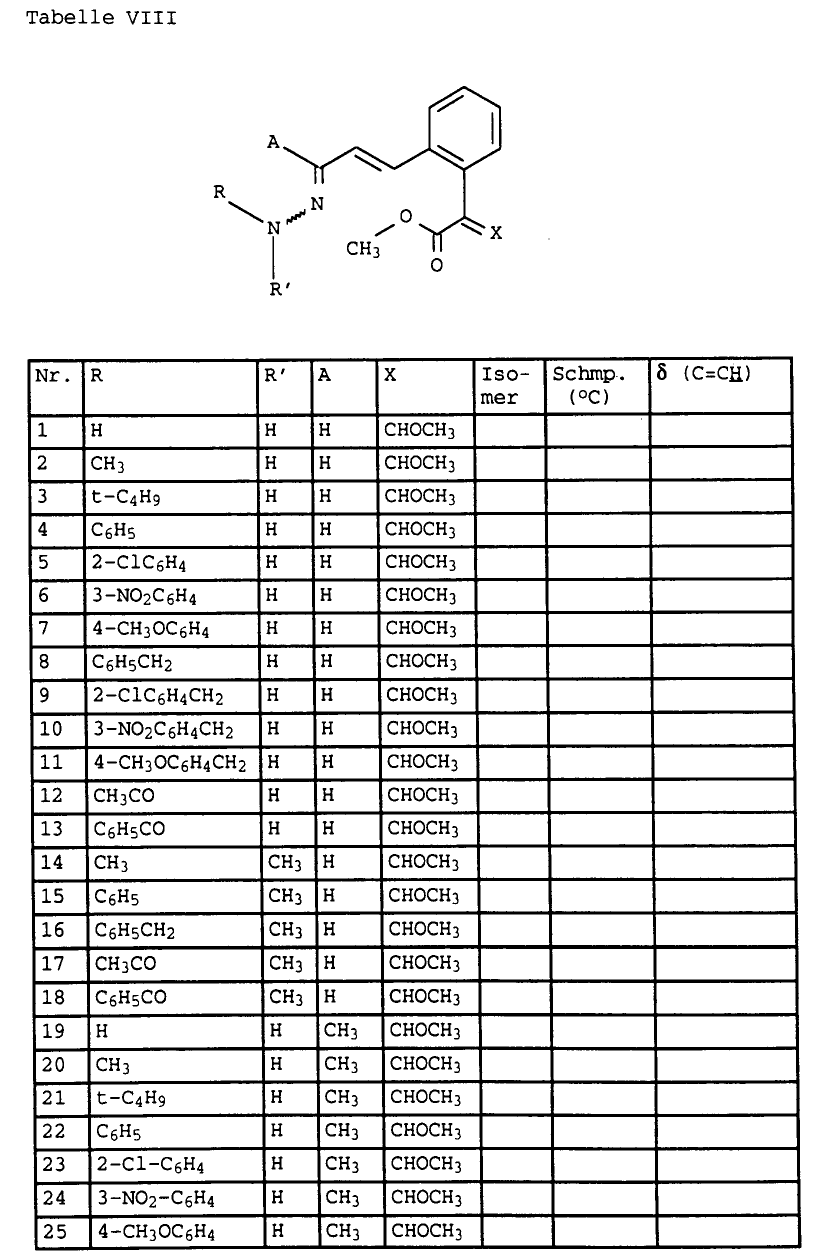

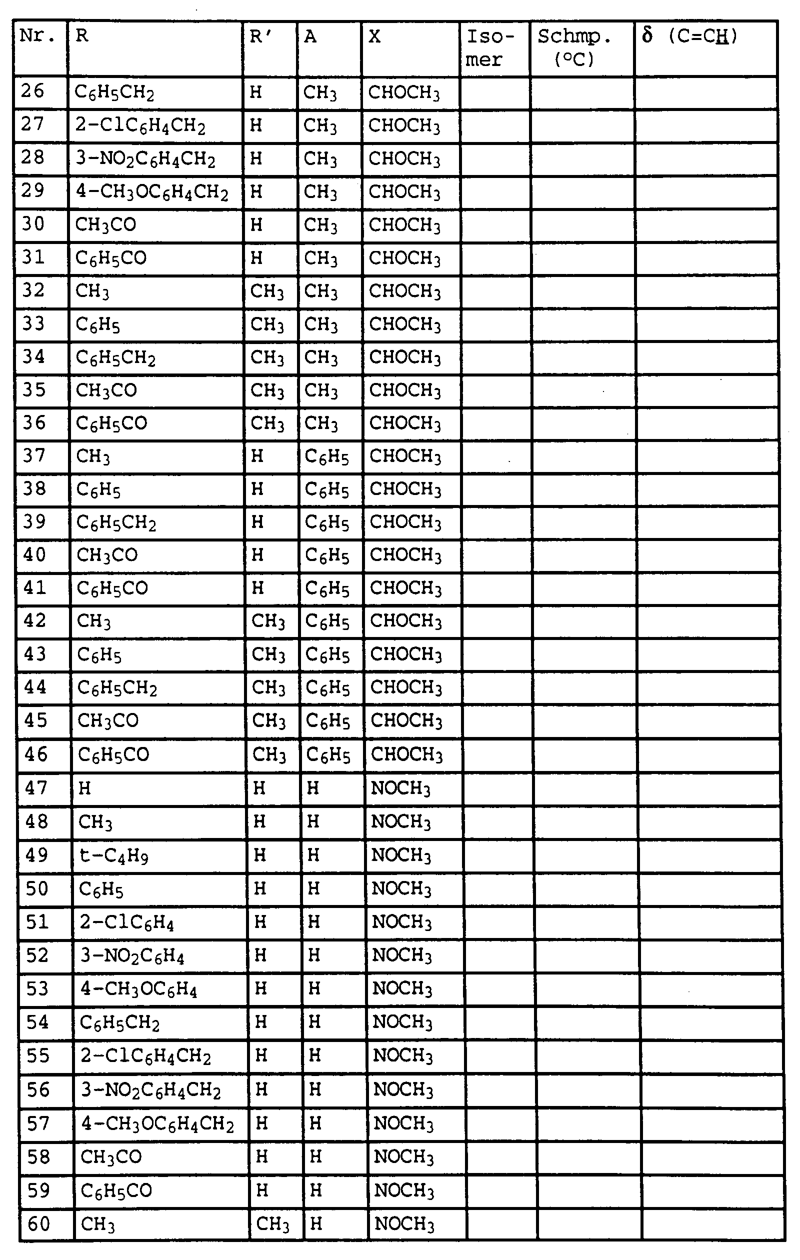

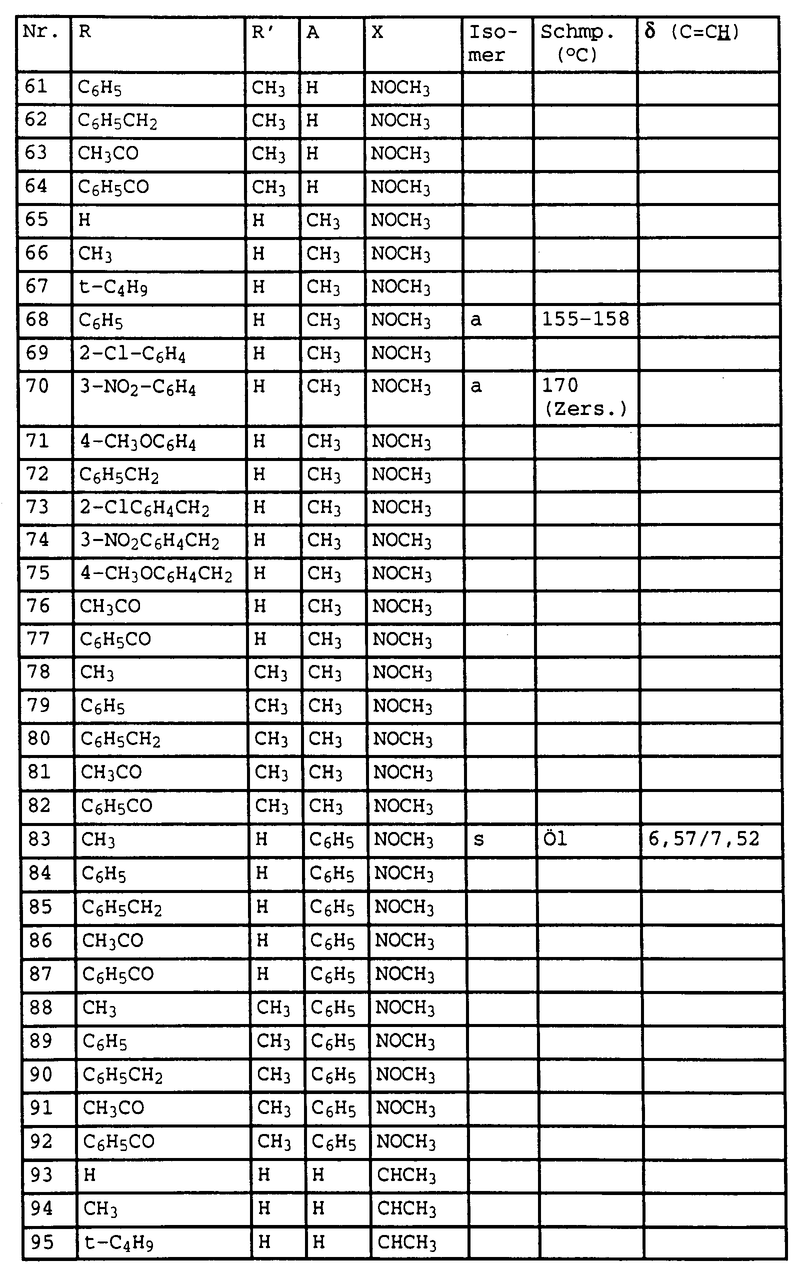

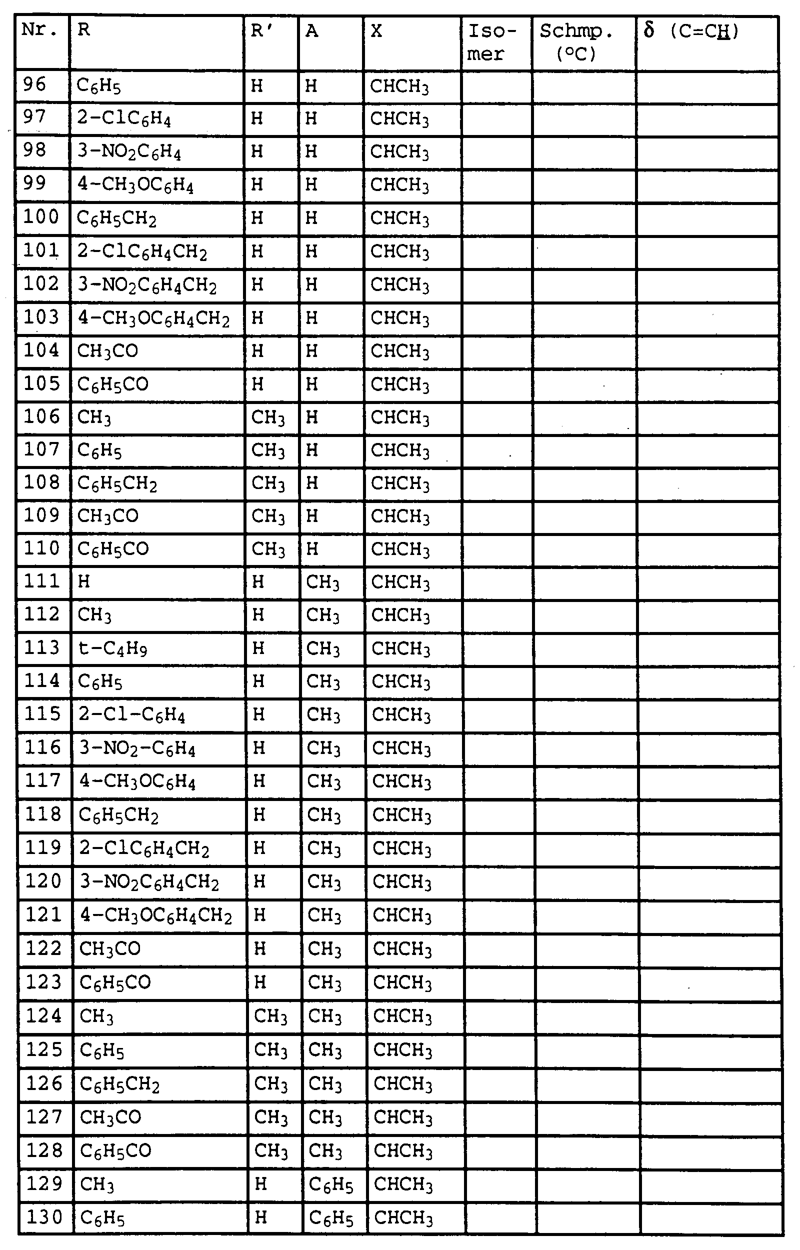

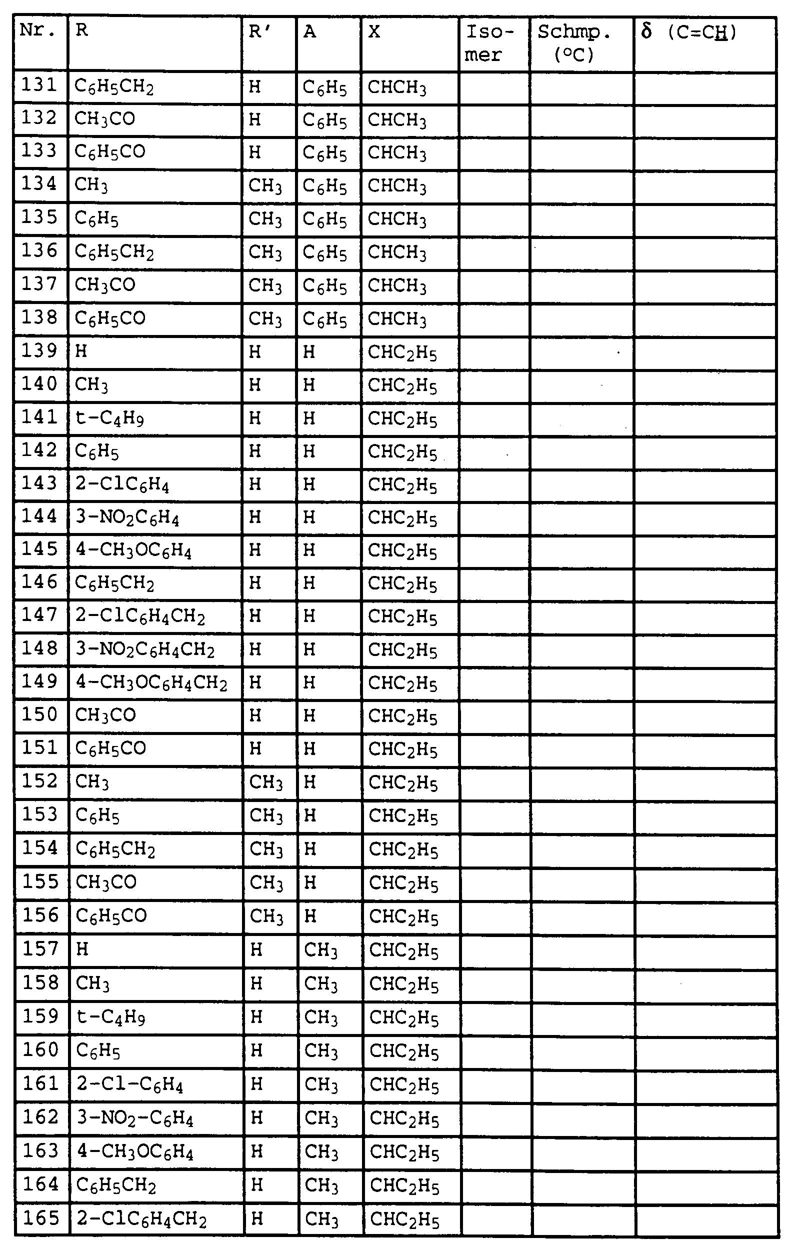

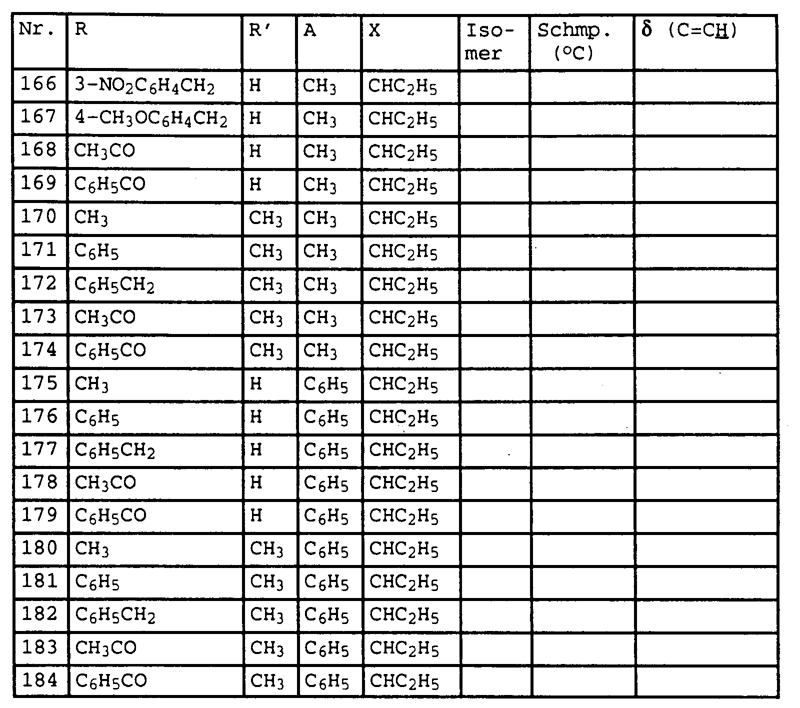

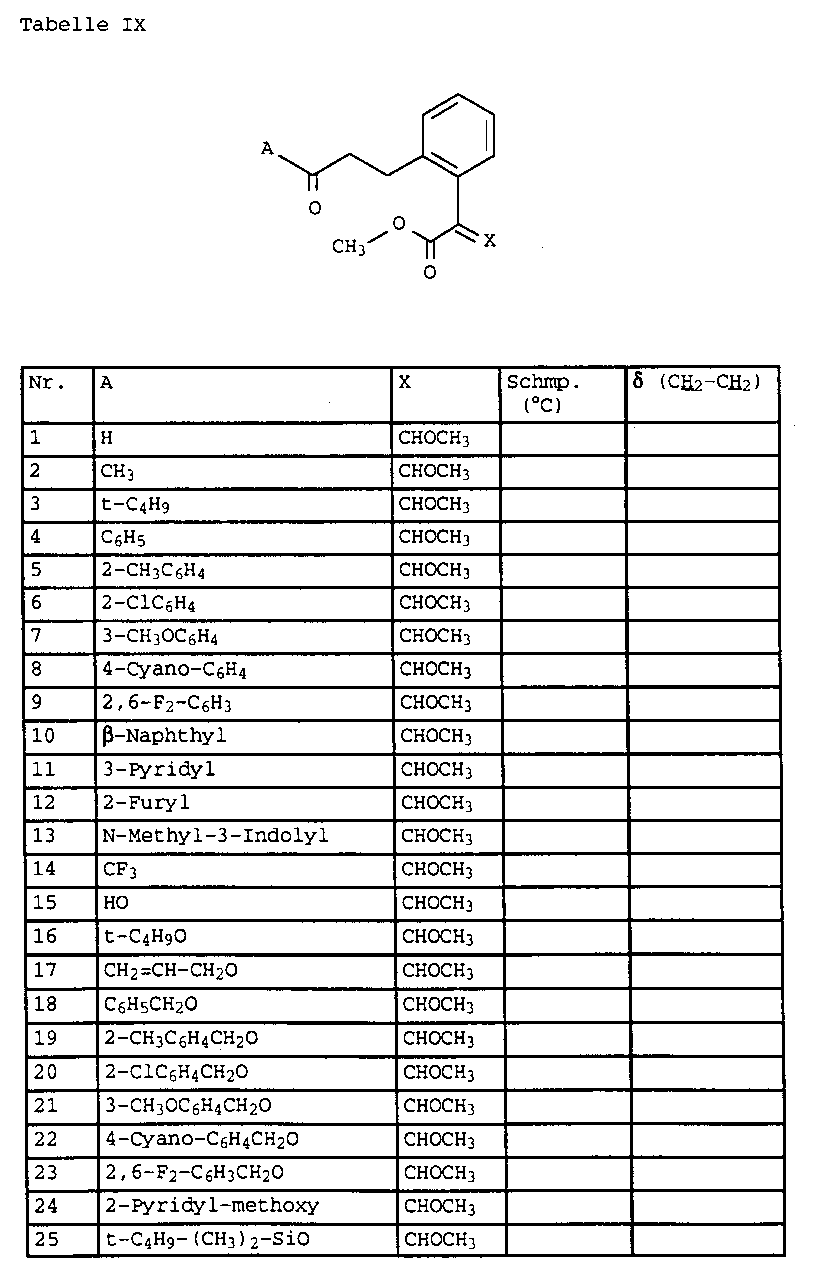

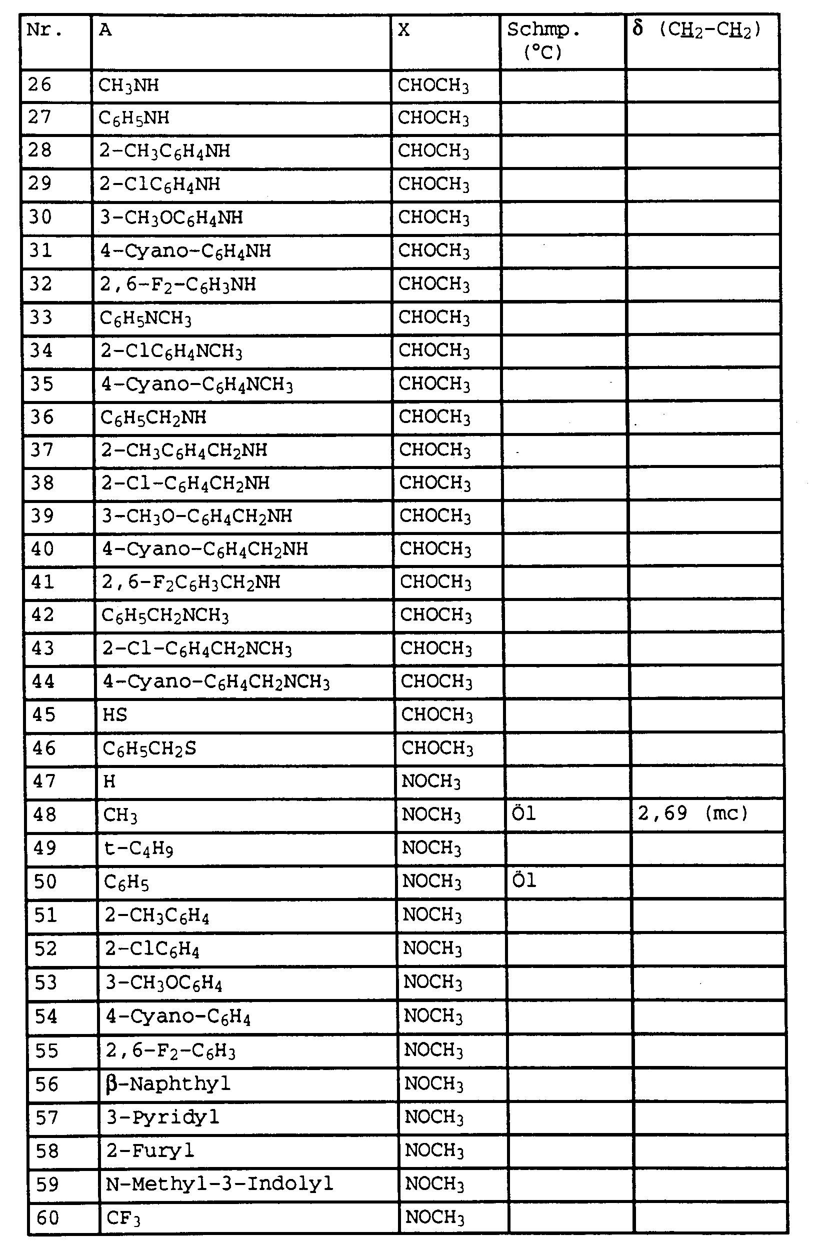

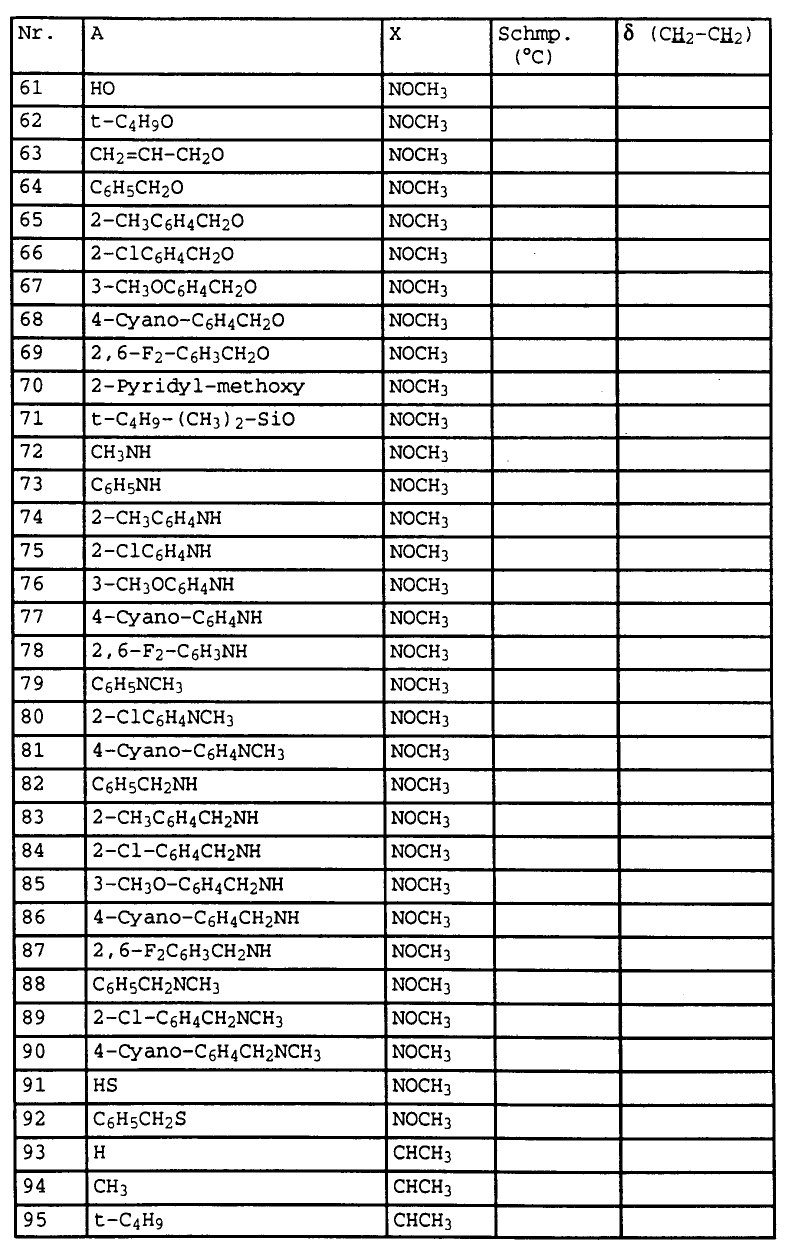

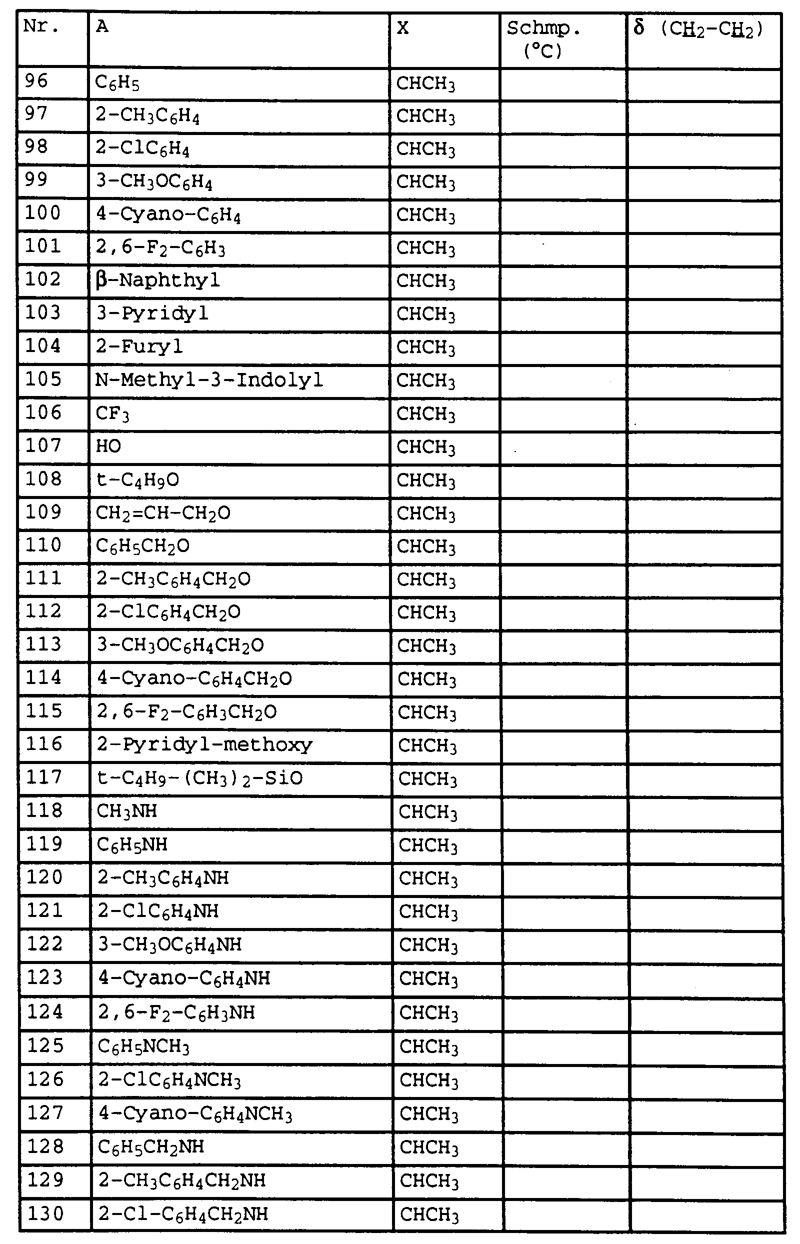

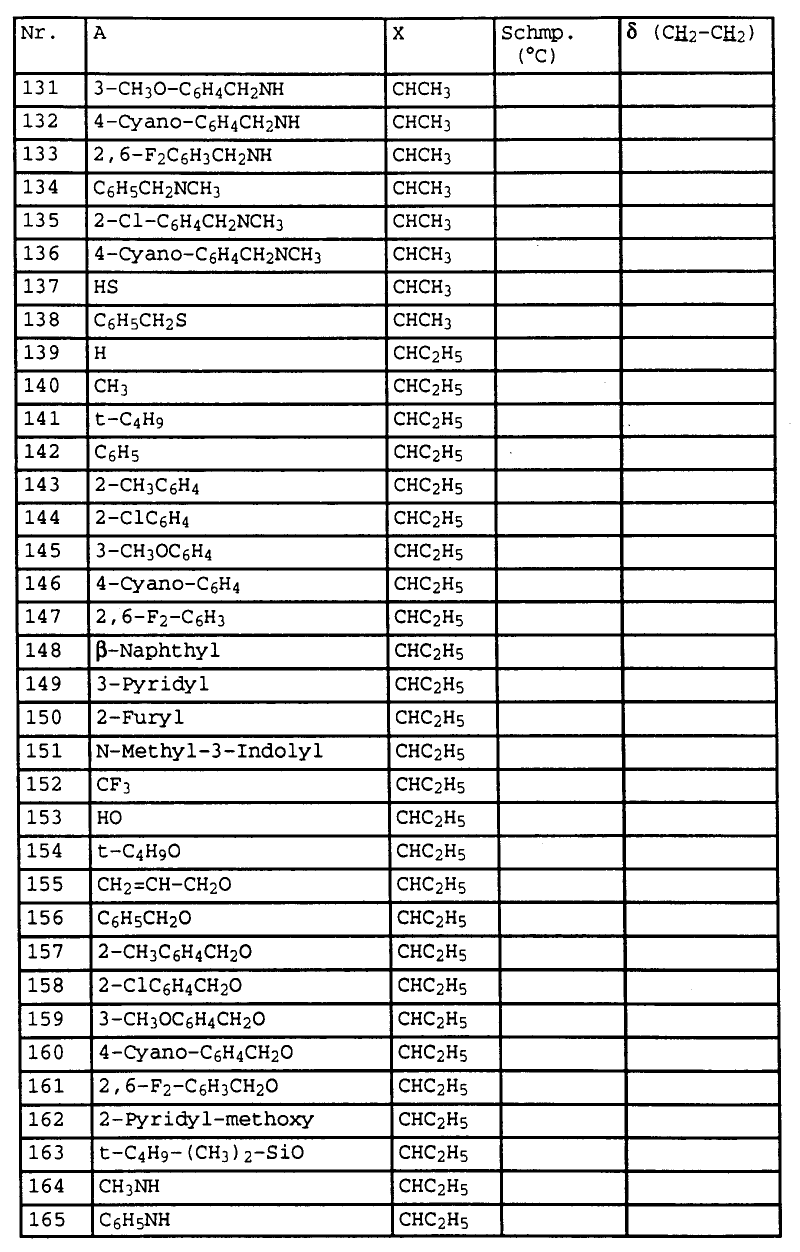

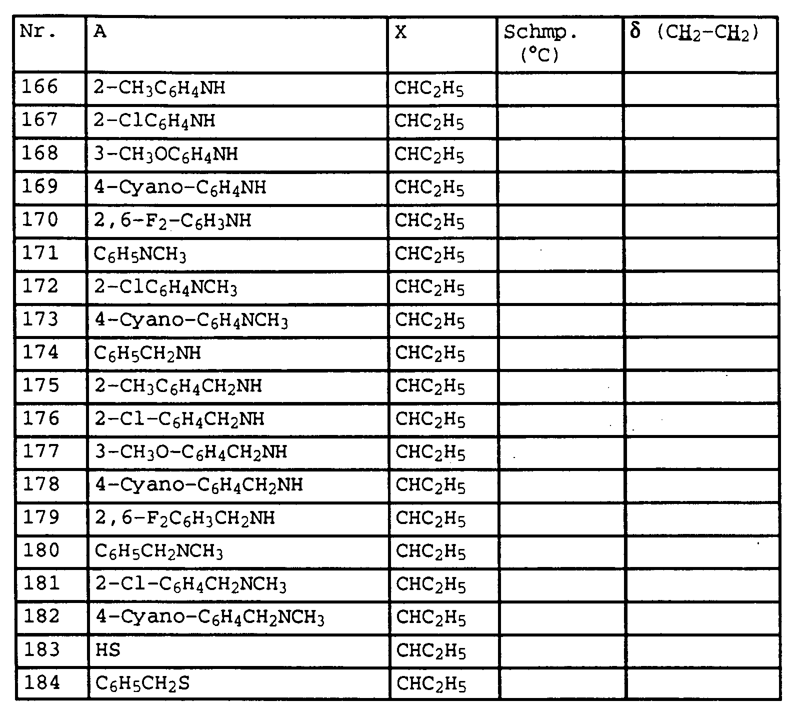

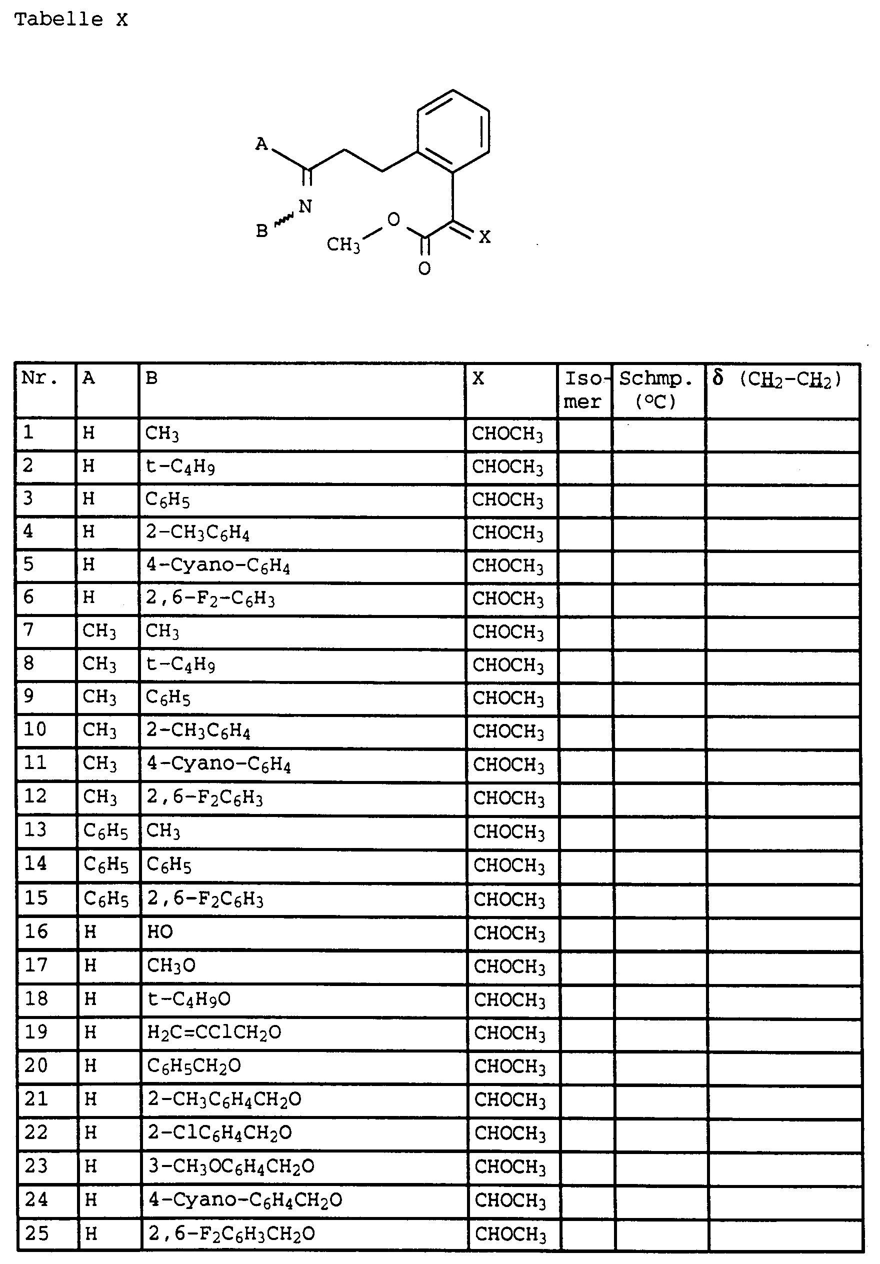

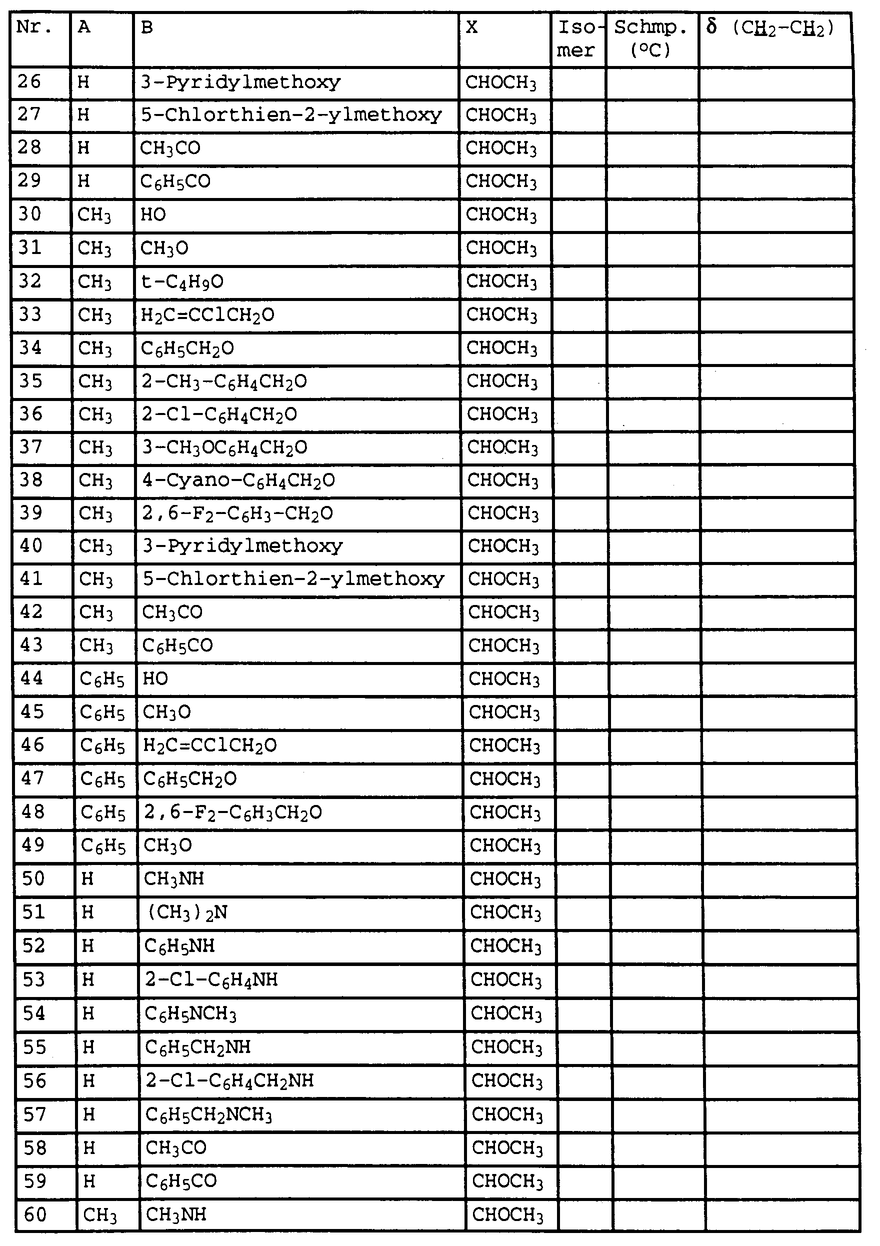

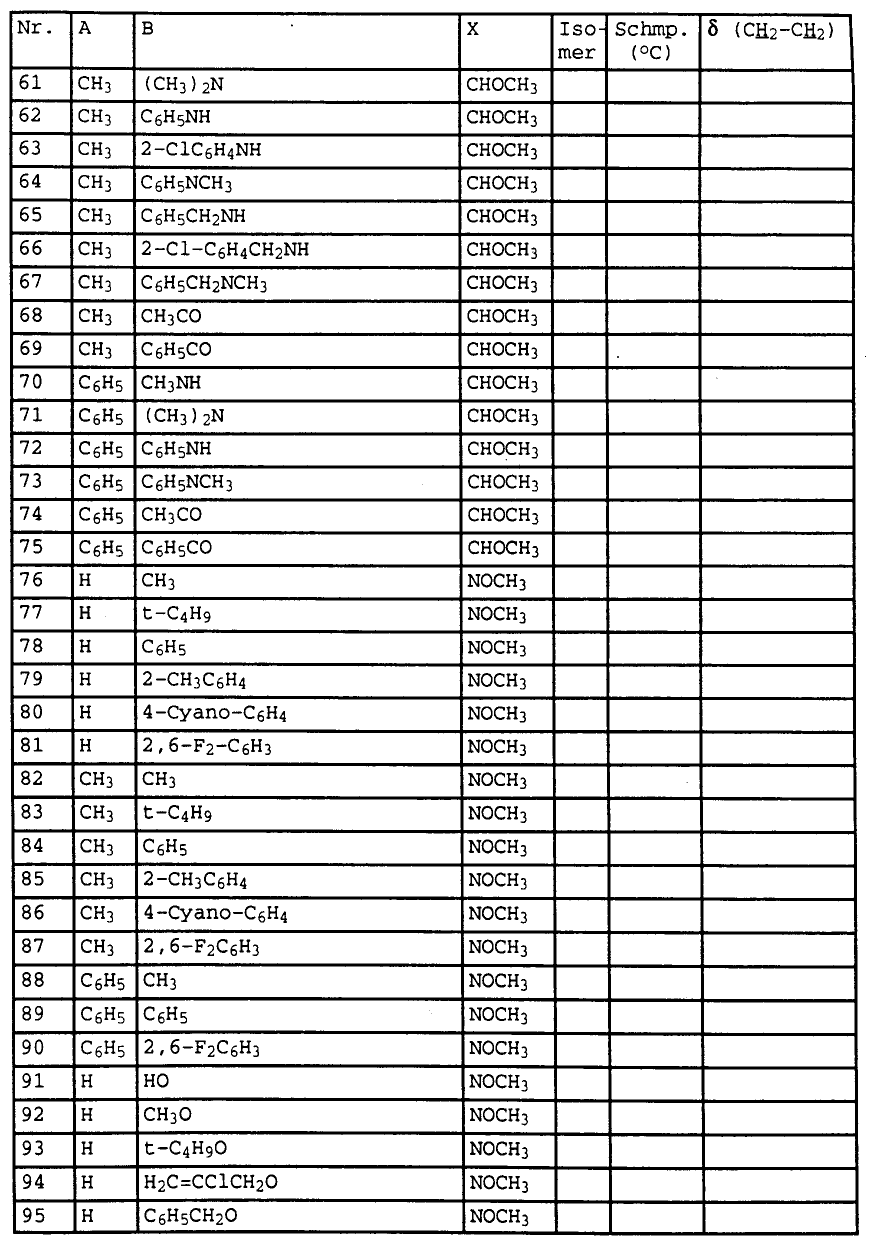

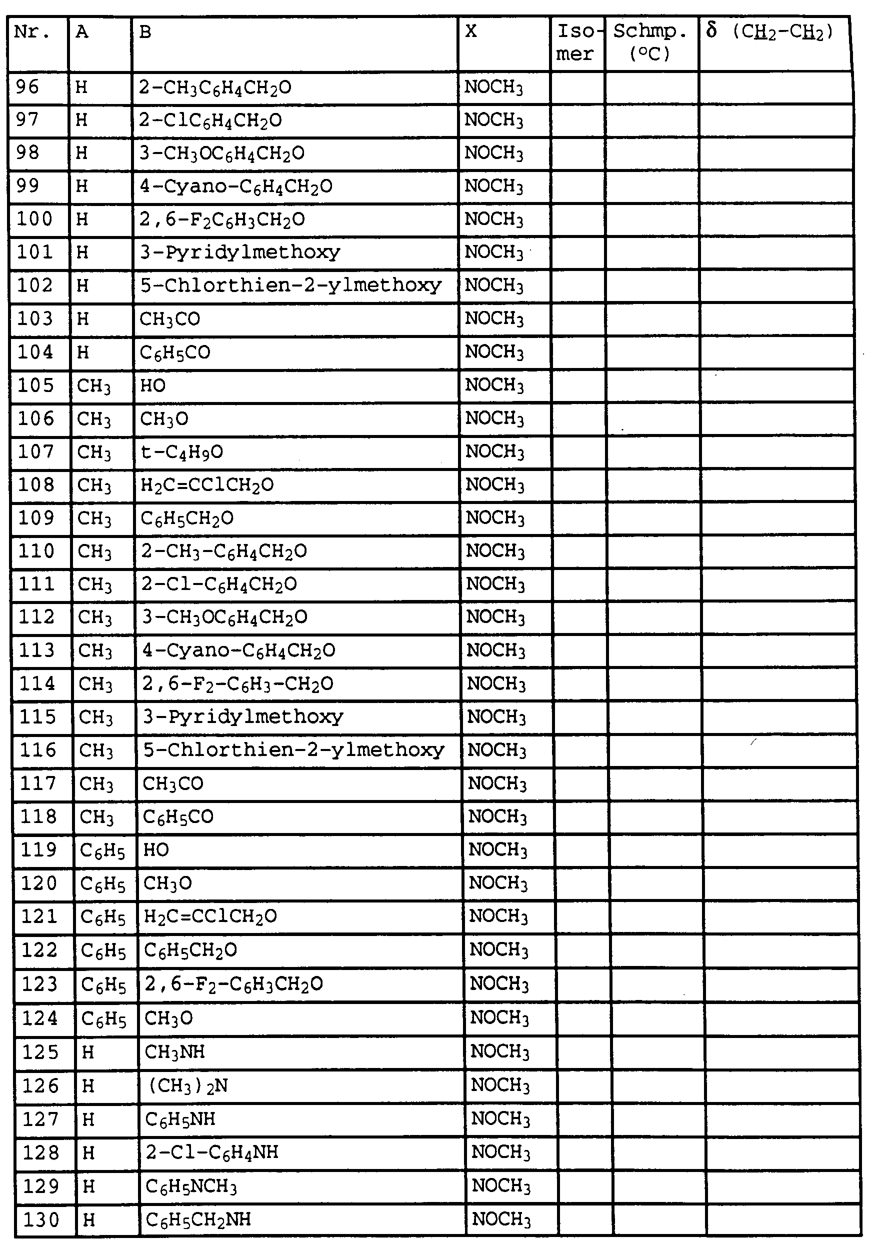

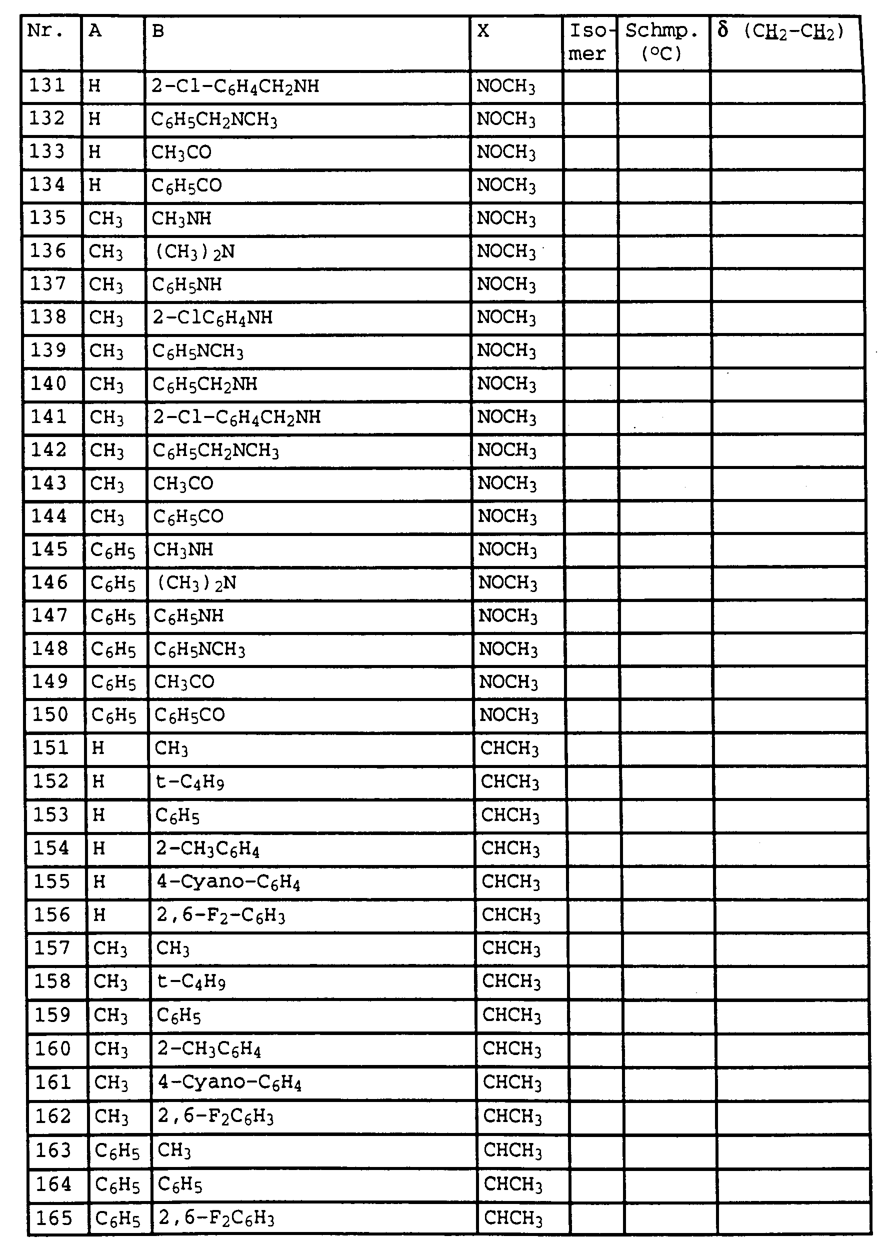

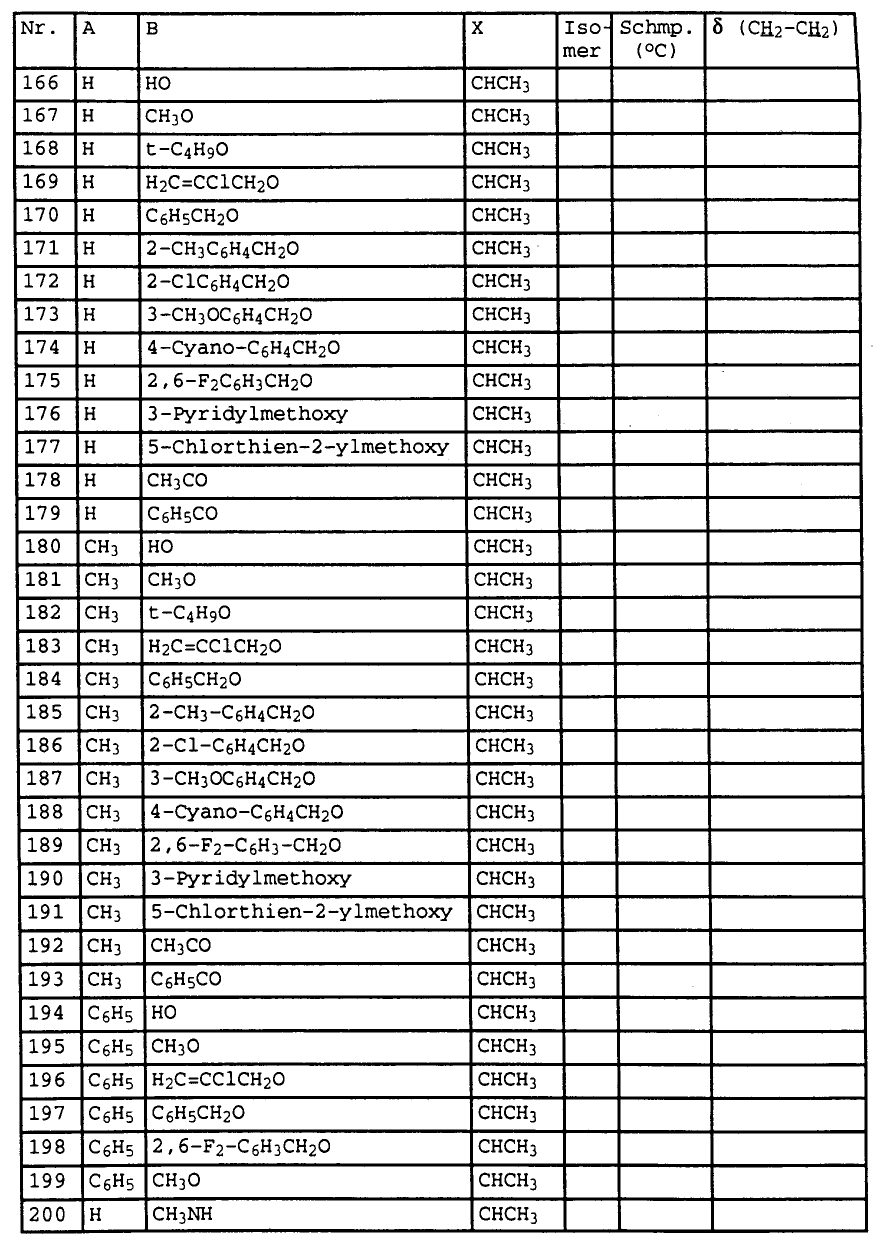

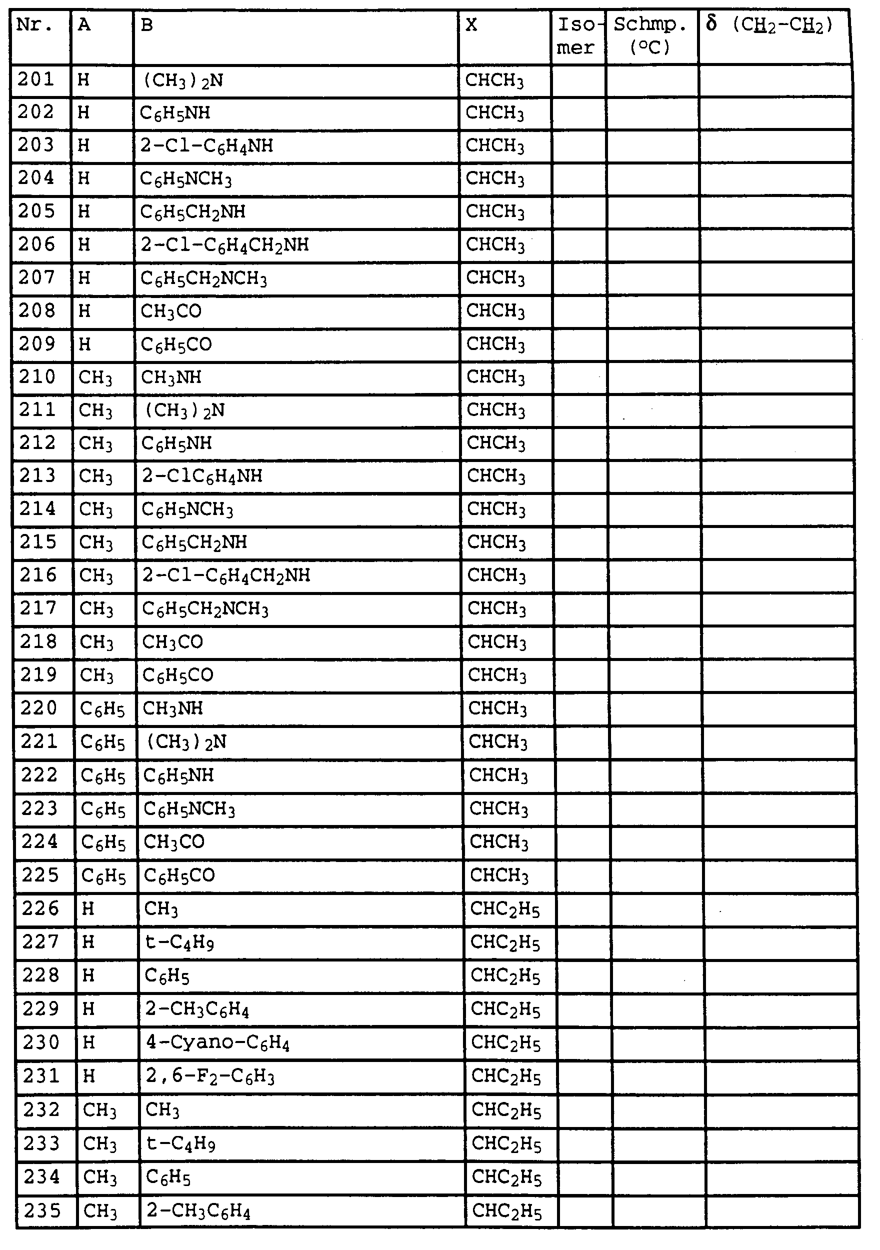

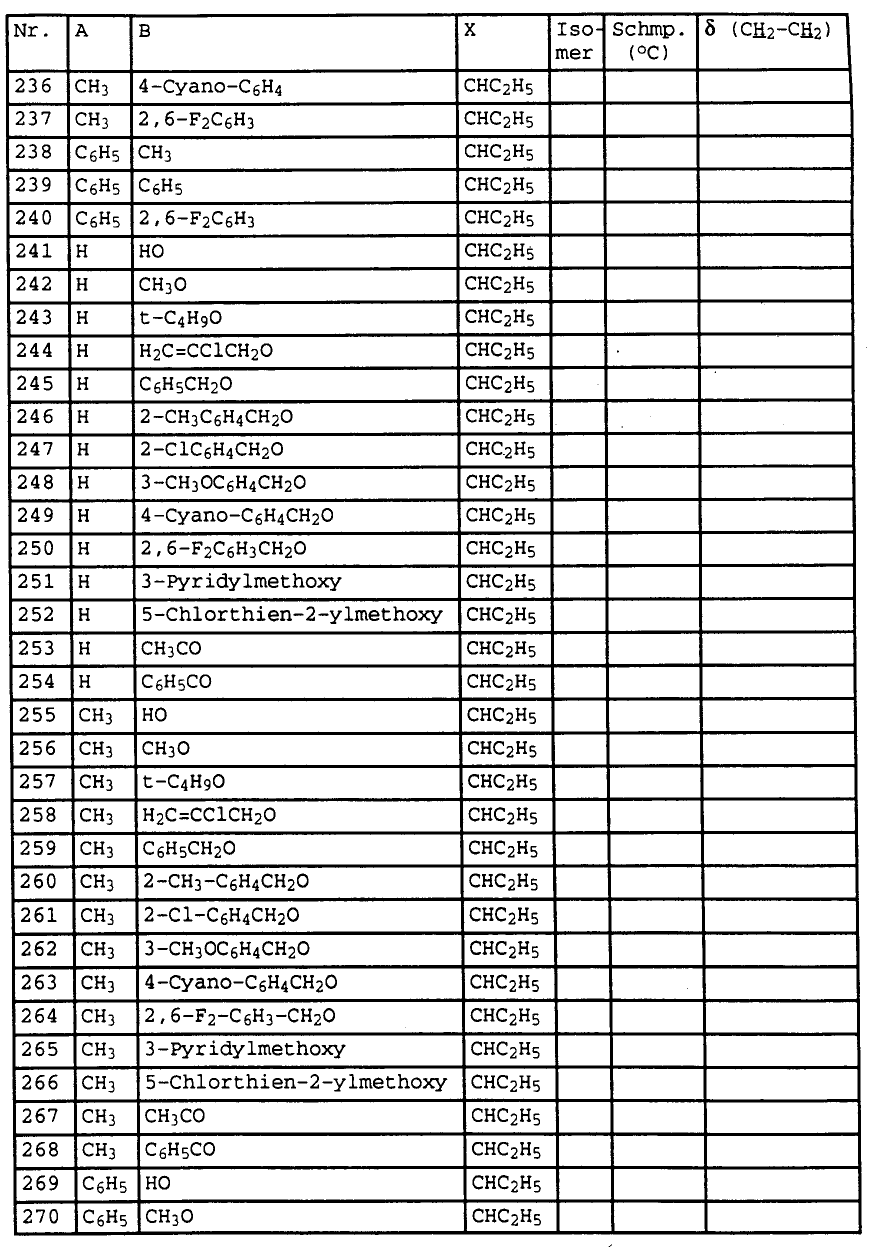

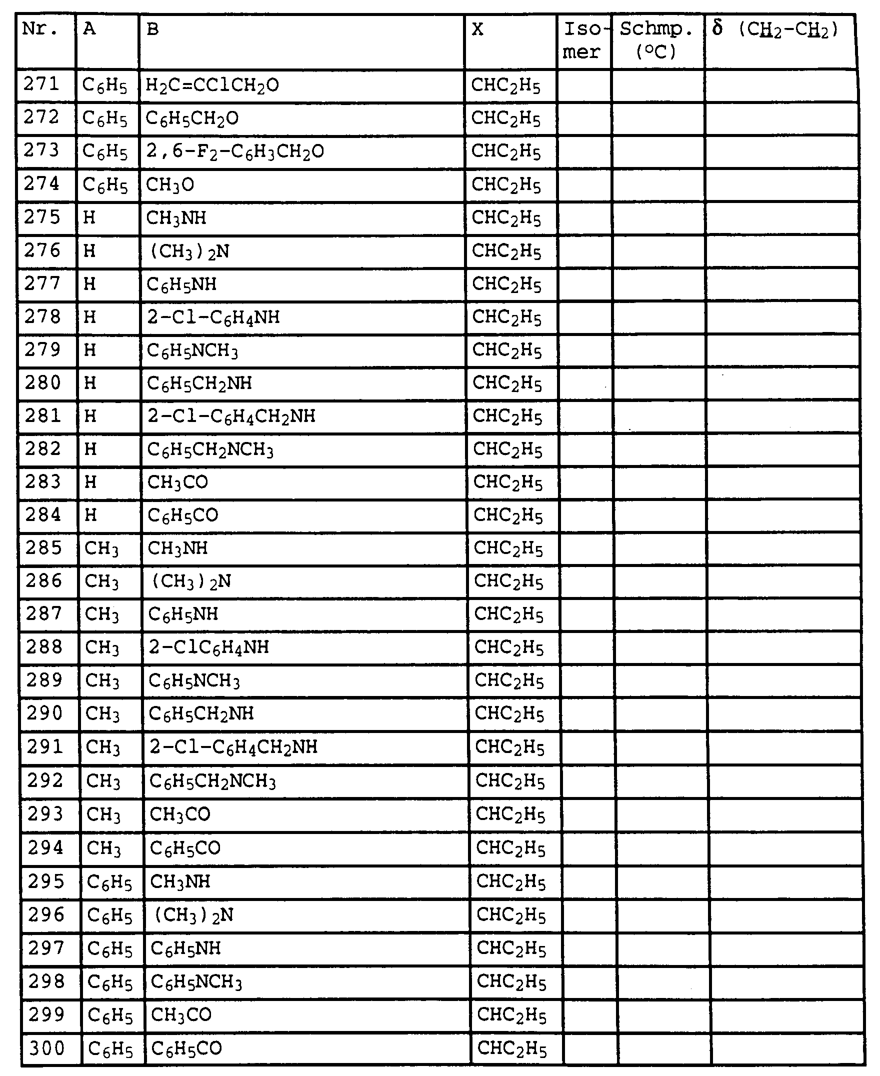

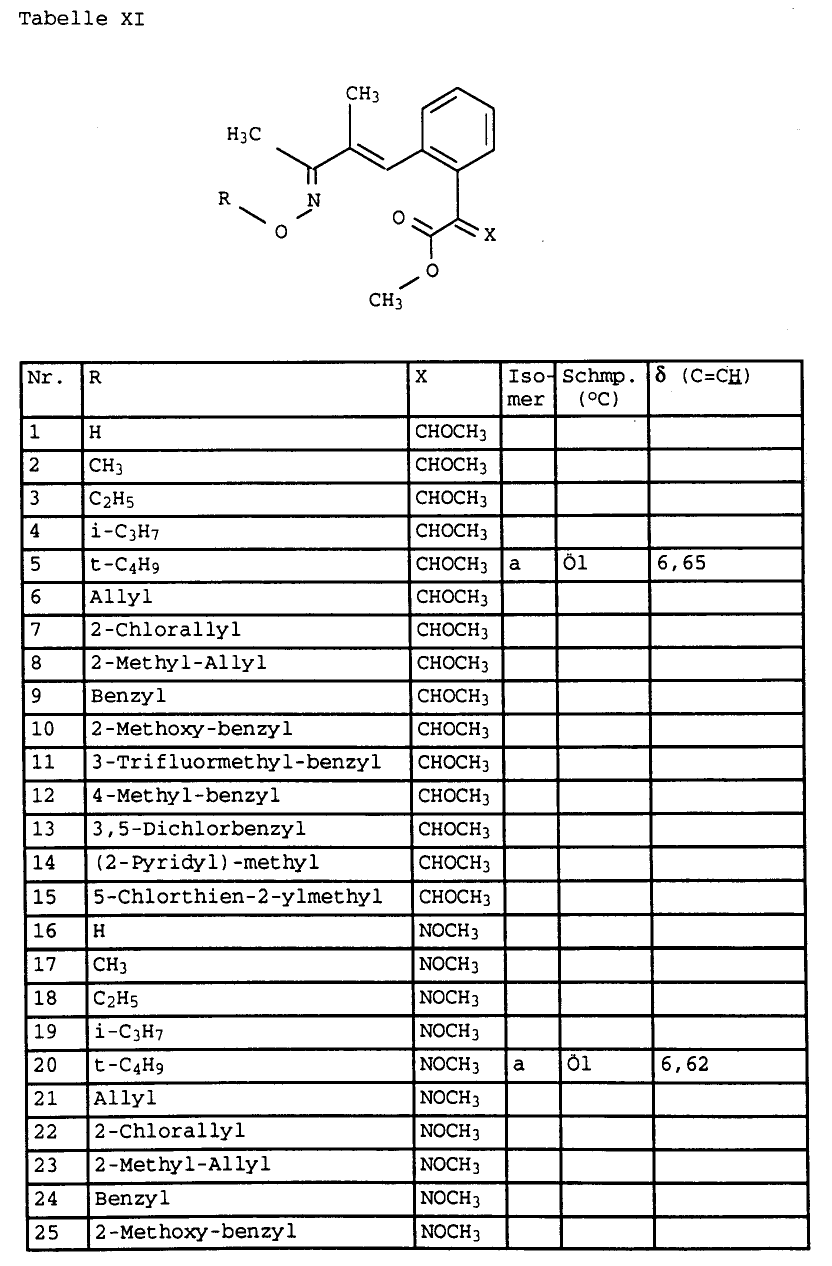

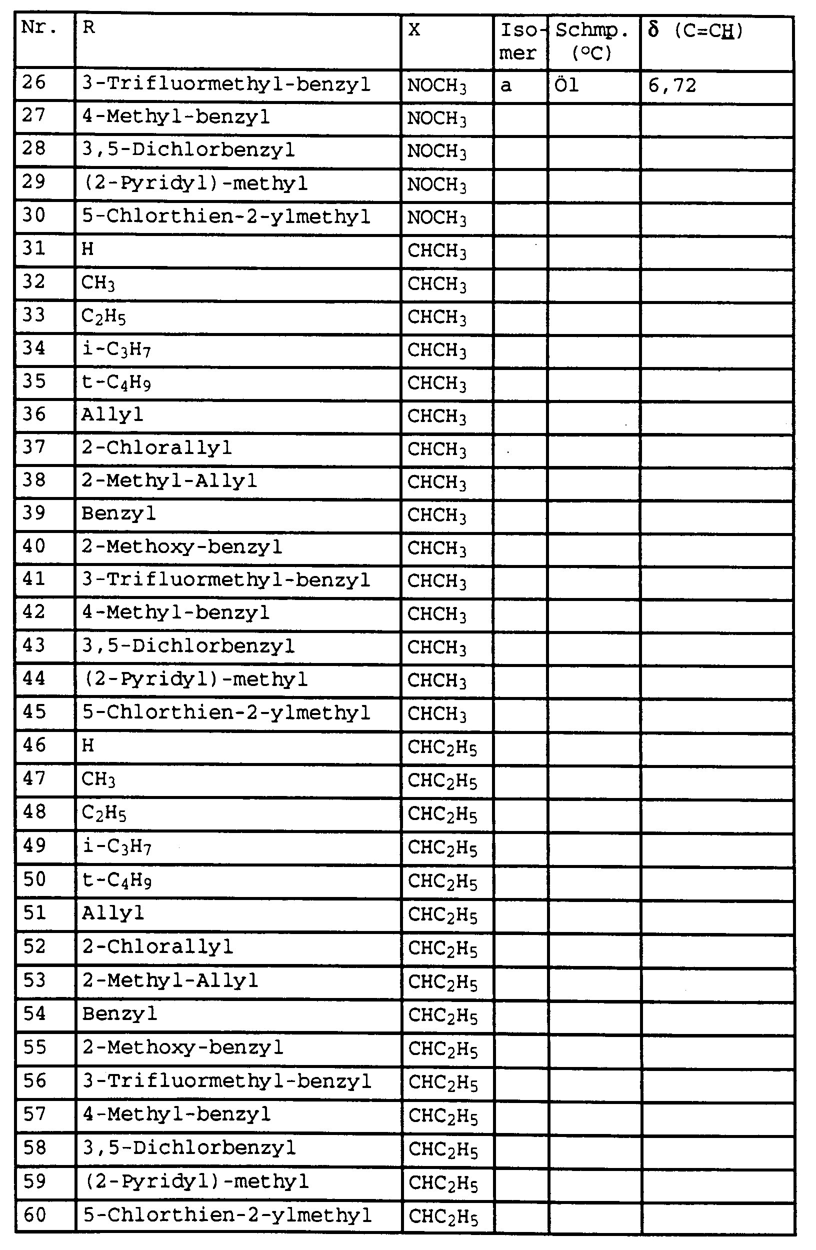

Die neuen Verbindungen eignen sich als Fungizide.

Die erfindungsgemäßen fungiziden Verbindungen bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwekken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zübereitungen sind:
I. eine Lösung aus 90 Gew.-Teilen der Verbindung Nr. 2 aus Tabelle 1 (1/2) und 10 Gew.-Teilen N-Methyl-a-pyrrolidon, die zur Anwendung in Form kleinster Tropfen geeignet ist;
II. eine Mischung aus 20 Gew.-Teilen der Verbindung Nr. 1/110, 80 Gew.Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.- Teilen des Anlagerungsproduktes von 40 Mol Ethylenoxid an 1 Mol Ricinusöl; durch feines Verteilen der Lösung in Wasser erhält man eine Dispersion.
III. eine wäßrige Dispersion aus 20 Gew.-Teilen der Verbindung Nr. 6/22, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;
IV. eine wäßrige Dispersion aus 20 Gew.-Teilen der Verbindung Nr. 6/27, 25 Gew.-Teilen Cyclohexanol, 65 Gew.-Teilen einer Mineralölfraktion vom Siedepunkt 210 bis 280'C und 10 Gew.-Teilen des Anlagerungsproduktes von 40 mol Ethylenoxid an 1 mol Ricinusöl;
V. eine in einer Hammermühle vermahlene Mischung aus 80 Gew.-Teilen der Verbindung Nr. 6/72, 3 Gew.-Teilen des Natriumsalzes der Diisobutylnaphtalin-asulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
VI. eine innige Mischung aus 3 Gew.-Teilen der Verbindung Nr. 6/77 und 97 Gew.-Teilen feinteiligem Kaolin; dieses Stäubemittel enthält 3 Gew.-% Wirkstoff;
VII. eine innige Mischung aus 30 Gew.-Teilen der Verbindung Nr. 6/78, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen der Verbindung Nr. 6/83, 10 Gew.-Teilen des Natriumsalzes eines Phenolsulfonsäure-harnstoff-formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen der Verbindung Nr. 6/85, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehydKondensates und 68 Gew.-Teilen eines paraffinischen Mineralöls.

Die neuen Verbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die Verbindungen I zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben, Cercospora arachidicola an Erdnüssen,
Pseudocercosporella herpotrichoides an Weizen, Gerste, Pyricularia oryzae an Reis,
Phytophthora infestans an Kartoffeln und Tomaten,
Fusarium- und Verticillium-Arten an verschiedenen Pflanzen, Plasmopara viticola an Reben,
Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

### Anwendungsbeispiele

Als Vergleichswirkstoff wurde 2-(2-Methylphenyl)-2-methoxyacrylsäuremethylester (A) - bekannt aus EP 178 826 - benutzt.

### Anwendungsbeispiel 1

### Wirksamkeit gegen Weizenmehltau

Blätter von in Töpfen gewachsenen Weizenkeimlingen der Sorte "Frühgold" wurden mit wäßriger Spritzbrühe, die 80 % Wirkstoff und 20 % Emulgiermittel in der Trockensubstanz enthielten, besprüht und 24 Stunden nach dem Antrocknen des Spritzbelages mit Oidien (Sporen) des Weizenmehltaus (Erysiphe graminis var. tritici) bestäubt. Die Versuchspflanzen wurden anschließend im Gewächshaus bei Temperaturen zwischen 20 und 22°C und 75 bis 80 % relativer Luftfeuchtigkeit aufgestellt. Nach 7 Tagen wurde das Ausmaß der Mehltauentwicklung ermittelt.

Das Ergebnis zeigt, daß die Wirkstoffe 2 aus Tabelle 1 (1/2), 1/110, 6/22, 6/27, 6/72, 6/77, 6/78, 6/83, 6/85, 6/89, 6/90, 7/36, 7/41a, 7/41s, 7/43, 7/60, 7/106, 7/107, 7/111, 7/112, 7/113, 7/116, 7/130, 7/133 bei der Anwendung als 250 ppm Wirkstoff enthaltende Spritzbrühe eine bessere fungizide Wirkung zeigen (90 %) als der bekannte Vergleichswirkstoff A (50 %).

### Anwendungsbeispiel 2

### Wirksamkeit gegen Plasmopara viticola

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus bei Temperaturen zwischen 20 und 30°C aufgestellt. Nach dieser Zeit wurden die Pflanzen zur Beschleunigung des Sporangienträgerausbruchs abermals für 16 Stunden in der feuchten Kammer aufgestellt. Dann erfolgte die Beurteilung des Ausmaßes des Pilzausbruchs auf den Blattunterseiten.

Das Ergebnis zeigt, daß die Wirkstoffe 1/2, 1/12, 1/88, 1(115, 2/57, 3/55, 6/22, 6/27, 6/72, 6/77, 6/78, 6/83, 6/85, 6/89, 6/90, 6/97, 7/36, 7/41a, 7/41s, 7/43, 7/60, 7/106, 7/112, 7/113, 7/116, 7/130, 7/133, 8/70, 9/50 bei der Anwendung als 250 ppm Wirkstoff enthaltende Spritzbrühe eine bessere fungizide Wirkung zeigen (90 %) als der bekannte Vergleichswirkstoff A (0 %).

## Patentansprüche

1. Verbindungen der allgemeinen Formel I, 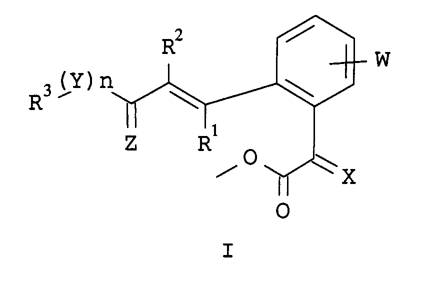 in der der Index und die Substituenten die folgende Bedeutung haben:
n 0 oder 1,
W Wasserstoff, Halogen, gegebenenfalls substituiertes C₁-C₄-Alkyl, oder gegebenenfalls substituiertes C₁-C₄-Alkoxy,
X CHOCH₃, NOCH₃, CHCH₃ oder CHC₂H₅,
Y Sauerstoff, Schwefel, NR⁴,-O-NR⁴-, -NR⁴-O- oder -NR⁴-NR⁵-,
Z Sauerstoff, NR⁶, NOR⁷ oder N-NR⁸R⁹, sowie für n = 1 auch Schwefel,
R¹ Wasserstoff oder gegebenenfalls substituiertes C₁-C₄-Alkyl,
R² Wasserstoff,
gegebenenfalls substituiertes C₁-C₄-Alkyl, gegebenenfalls substituiertes C₃-C₆-Cycloalkyl, gegebenenfalls substituiertes Aryl, gegebenenfalls substituiertes Heteroaryl, C₁-C₄-Perfluoroalkyl, oder CO-V,
R³ Wasserstoff,
gegebenenfalls substituiertes C₁-C₄-Alkyl, gegebenenfalls substituiertes C₂-C₄-Alkenyl, gegebenenfalls substituiertes C₂-C₄-Alkinyl, gegebenenfalls substituiertes C₃-C₆-Cycloalkyl, gegebenenfalls substituiertes 3- bis 6-gliedriges Heterocyclyl, gegebenenfalls substituiertes und/oder kondensiertes Aryl, gegebenenfalls substituiertes und/oder kondensiertes Heteroaryl, gegebenenfalls substituiertes Aryl-C₁-C₄-Alkyl, oder gegebenenfalls substituiertes Heteroaryl-C₁-C₄-Alkyl gegebenenfalls substituiertes Aryl-C₂-C₄-Alkenyl,
für n = 0 auch Cyano, C₁-C₄-Perfluoralkyl und
für n = 1, falls Y Sauerstoff bedeutet, gegebenenfalls substituiertes Trialkylsilyl,
R⁴, R⁵ und R⁹ Wasserstoff,
gegebenenfalls substituiertes C₁-C₄-Alkyl, oder gegebenenfalls substituiertes C₃-C₆-Cycloalkyl,
R⁶ gegebenenfalls substituiertes C₁-C₄-Alkyl, gegebenenfalls substituiertes oder kondensiertes Aryl oder gegebenenfalls substituiertes oder kondensiertes Heteroaryl,
R⁷, R⁸ Wasserstoff,
gegebenenfalls substituiertes C₁-C₄-Alkyl, gegebenenfalls substituiertes C₂-C₄-Alkenyl, gegebenenfalls substituiertes C₂-C₄-Alkinyl, gegebenenfalls substituiertes C₃-C₆-Cycloalkyl, gegebenenfalls substituiertes oder kondensiertes Aryl, gegebenenfalls substituiertes und/oder kondensiertes Heteroaryl, gegebenenfalls substituiertes Aryl-C₁-C₄-Alkyl, gegebenenfalls substituiertes Heteroaryl-C₁-C₄-Alkyl,
oder eine Gruppe COR¹⁰
R¹⁰ gegebenenfalls substituiertes C₁-C₄-Alkyl, gegebenenfalls substituiertes C₃-C₆-Cycloalkyl, gegebenenfalls substituiertes Aryl, oder gegebenenfalls substituiertes Heteroaryl,
V gegebenenfalls substituiertes C₁-C₄-Alkyl, gegebenenfalls substituiertes Aryl, gegebenenfalls substituiertes Heteroaryl, Hydroxyl, gegebenenfalls substituiertes C₁-C₄-Alkoxy, gegebenenfalls substituiertes Allyloxy, gegebenenfalls substituiertes Benzyloxy, odergegebenenfalls substituiertes Trialkylsilyloxy.

2. Verbindungen I gemäß Anspruch 1, dadurch gekennzeichnet, daß X CHOCH₃ bedeutet.

3. Verbindungen I gemäß Anspruch 1, dadurch gekennzeichnet, daß X NOCH₃ bedeutet.

4. Verbindungen I gemäß Anspruch 1, dadurch gekennzeichnet, daß X CHCH₃ oder CHC₂H₅ bedeutet.

5. Verbindungen I gemäß Anspruch 1, dadurch gekennzeichnet, daß n = 0 ist und Z O, NR⁶, NOR⁷ oder N-NR⁸R⁹ bedeutet.

6. Verbindungen I gemäß Anspruch 1, dadurch gekennzeichnet, daß n = 1 ist, Z für Sauerstoff steht und Y Sauerstoff, Schwefel, NR⁴ oder NR⁴NR⁵ bedeutet.

7. Verbindungen I gemäß Anspruch 1 in der n 0 bedeutet und in denen die Substituenten die folgende Bedeutung haben:
W Wasserstoff, Chlor, Methyl oder Methoxy,
Z Sauerstoff, NR⁶, NOR⁷ oder N-NR⁸R⁹,
R¹, R² Wasserstoff oder C₁-C₄-Alkyl,
R³ Wasserstoff,
gegebenenfalls substituiertes C₁-C₄-Alkyl, gegebenenfalls substituiertes C₂-C₄-Alkenyl, gegebenenfalls substituiertes C₂-C₄-Alkinyl, gegebenenfalls substituiertes C₃-C₆-Cycloalkyl, gegebenenfalls substituiertes 3- bis 6-gliedriges Heterocyclyl, gegebenenfalls substituiertes oder kondensiertes Aryl, gegebenenfalls substituiertes oder kondensiertes Heteroaryl, gegebenenfalls substituiertes Aryl-C₁-C₄-Alkyl, gegebenenfalls substituiertes Heteroaryl-C₁-C₄-Alkyl, gegebenenfalls substituiertes Aryl-C₂-C₄-Alkenyl oder gegebenenfalls substituiertes Heteroaryl-C₂-C₄-Alkenyl

8. Verbindungen I gemäß Anspruch 1, in denen n für 1 und Z für O steht und in denen die Substituenten die folgende Bedeutung haben:
W Wasserstoff, Chlor, Methyl oder Methoxy,
Y Sauerstoff, Schwefel oder eine Gruppe NR⁴,
R¹ Wasserstoff oder C₁-C₄-Alkyl,
R² Wasserstoff, C₁-C₄-Alkyl oder eine Gruppe CO-V,
R³ Wasserstoff,
gegebenenfalls substituiertes C₁-C₄-Alkyl, gegebenenfalls substituiertes oder kondensiertes Aryl, gegebenenfalls substituiertes oder kondensiertes Heteroaryl, gegebenenfalls substituiertes Aryl-C₁-C₄-Alkyl, oder gegebenenfalls substituiertes Heteroaryl-C₁-C₄-Alkyl,
V Hydroxyl, C₁-C₄-Alkoxy, Allyloxy, Trialkylsilyloxy oder gegebenenfalls substituiertes Benzyloxy.

9. Verbindungen I gemäß Anspruch 1, in denen W Wasserstoff und n 0 bedeutet und in denen die Substituenten die folgende Bedeutung haben:
Z Sauerstoff oder NOR⁷,
R³ Wasserstoff, gegebenenfalls substituiertes C₁-C₄-Alkyl, gegebenenfalls substituiertes C₂-C₄-Alkenyl, gegebenenfalls substituiertes oder kondensiertes Aryl, oder gegebenenfalls substituiertes oder kondensiertes Heteroaryl,
R⁷ Wasserstoff,
gegebenenfalls substituiertes C₁-C₄-Alkyl, gegebenenfalls substituiertes C₂-C₄-Alkenyl, gegebenenfalls substituiertes Aryl-C₁-C₄-Alkyl, gegebenenfalls substituiertes Heteroaryl-C₁-C₄-Alkyl, oder eine Gruppe COR¹⁰,
R¹⁰ gegebenenfalls substituiertes C₁-C₄-Alkyl,
gegebenenfalls substituiertes Aryl, oder gegebenenfalls substituiertes Heteroaryl.

10. Verbindungen I gemäß Anspruch 1, in denen W Wasserstoff, Z Sauerstoff und n 1 bedeutet und in denen die Substituenten die folgende Bedeutung haben:
Y Sauerstoff oder NR⁴,
R³ gegebenenfalls substituiertes C₁-C₄-Alkyl, gegebenenfalls substituiertes oder kondensiertes Aryl, gegebenenfalls substituiertes oder kondensiertes Heteroaryl, gegebenenfalls substituiertes Aryl-C₁-C₄-Alkyl, oder gegebenenfalls substituiertes Heteroaryl-C₁-C₄-Alkyl,
R⁴ Wasserstoff oder C₁-C₄-Alkyl.

11. Fungizid, dadurch gekennzeichnet, daß es einen inerten Trägerstoff und eine fungizid wirksame Menge einer Verbindung der Formel I gemäß Anspruch 1 enthält.

12. Verfahren zur Bekämpfung von Pilzen, dadurch gekennzeichnet, daß man auf die Pilze oder durch Pilzbefall bedrohte Pflanzen, Saatgüter, Materialien oder den Boden eine fungizid wirksame Menge einer Verbindung der allgemeinen Formel I gemäß Anspruch 1 einwirken läßt.

13. Insektizid, dadurch gekennzeichnet, daß es einen inerten Trägerstoff und eine insektizid wirksame Menge einer Verbindung der Formel I gemäß Anspruch 1 enthält.

14. Akarizid, dadurch gekennzeichnet, daß es einen inerten Trägerstoff und eine akarizid wirksame Menge einer Verbindung der Formel I gemäß Anspruch 1 enthält.

## Claims

1. A compound of the formula I 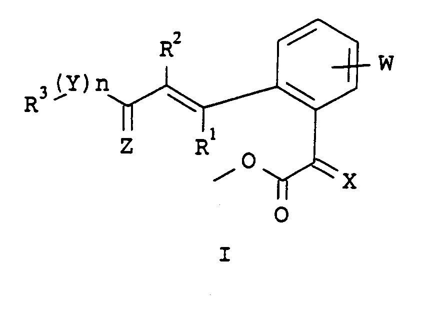 where
n is 0 or 1,
W is hydrogen, halogen, unsubstituted or substituted C₁-C₄-alkyl or unsubstituted or substituted C₁-C₄-alkoxy,
X is CHOCH₃, NOCH₃, CHCH₃ or CHC₂H₅,
Y is oxygen, sulfur, NR⁴, -ONR⁴-, -NR⁴-O- or -NR⁴-NR⁵-,
Z is oxygen, NR⁶, NOR⁷ or N-NR⁸R⁹ or, where n = 1, furthermore sulfur,
R¹ is hydrogen or unsubstituted or substituted C₁-C₄-alkyl,
R² is hydrogen,
unsubstituted or substituted C₁-C₄-alkyl, unsubstituted or substituted C₃-C₆-cycloalkyl, unsubstituted or substituted aryl, unsubstituted or substituted hetaryl, C₁-C₄-perfluoroalkyl or CO-V,
R³ is hydrogen,
unsubstituted or substituted C₁-C₄-alkyl, unsubstituted or substituted C₂-C₄-alkenyl, unsubstituted or substituted C₂-C₄-alkynyl, unsubstituted or substituted C₃-C₆-cycloalkyl, unsubstituted or substituted 3-membered to 6-membered heterocyclyl, unsubstituted or substituted and/or fused aryl, unsubstituted or substituted and/or fused hetaryl, unsubstituted or substituted aryl-C₁-C₄-alkyl, unsubstituted or substituted hetaryl-C₁-C₄-alkyl, unsubstituted or substituted aryl-C₂-C₄-alkenyl or unsubstituted or substituted hetaryl-C₂-C₄-alkenyl,
or,
where n = 0, furthermore cyano or C₁-C₄-perfluoroalkyl, or,
where n = 1, if Y is oxygen, furthermore unsubstituted or substituted trialkylsilyl,
R⁴, R⁵ and R⁹ are hydrogen,
unsubstituted or substituted C₁-C₄-alkyl or unsubstituted or substituted C₃-C₆-cycloalkyl,
R⁶ is unsubstituted or substituted C₁-C₄-alkyl, unsubstituted or substituted or fused aryl or unsubstituted or substituted or fused hetaryl,
R⁷ and R⁸ are hydrogen,
unsubstituted or substituted C₁-C₄-alkyl, unsubstituted or substituted C₂-C₄-alkenyl, unsubstituted or substituted C₂-C₄-alkynyl, unsubstituted or substituted C₃-C₆-cycloalkyl, unsubstituted or substituted or fused aryl, unsubstituted or substituted and/or fused hetaryl, unsubsti-tuted or substituted aryl-C₁-C₄-alkyl, unsubstituted or substituted hetaryl-C₁-C₄-alkyl
or a group COR¹⁰,
R¹⁰ is unsubstituted or substituted C₁-C₄-alkyl, unsubstituted or substituted C₃-C₆-cycloalkyl, unsubstituted or substituted aryl or unsubstituted or substituted hetaryl, and
V is unsubstituted or substituted C₁-C₄-alkyl, unsubstituted or substituted aryl, unsubstituted or substituted hetaryl, hydroxyl, unsubstituted or substituted C₁-C₄-alkoxy, unsubstituted or substituted allyloxy, unsubstituted or substituted benzyloxy or unsubstituted or substituted trialkylsilyloxy.

2. A compound I as claimed in claim 1, wherein X is CHOCH₃.

3. A compound I as claimed in claim 1, wherein X is NOCH₃.

4. A compound I as claimed in claim 1, wherein X is CHCH₃ or CHC₂H₅.

5. A compound I as claimed in claim 1, wherein n is 0 and Z is O, NR⁶, NOR⁷ or N-NR⁸R⁹.

6. A compound I as claimed in claim 1, wherein n is 1, Z is oxygen and Y is oxygen, sulfur, NR⁴ or NR⁴NR⁵

7. A compound I as claimed in claim 1, where
n is 0,
W is hydrogen, chlorine, methyl or methoxy,
Z is oxygen, NR⁶, NOR⁷ or N-NR⁸R⁹,
R¹ and R² are hydrogen or C₁-C₄-alkyl,
R³ is hydrogen,
unsubstituted or substituted C₁-C₄-alkyl, unsubstituted or substituted C₂-C₄-alkenyl, unsubstituted or substituted C₂-C₄-alkynyl, unsubstituted or substituted C₃-C₆-cycloalkyl, unsubstituted or substituted 3-membered to 6-membered heterocyclyl, unsubstituted or substituted or fused aryl, unsubstituted or substituted or fused hetaryl, unsubstituted or substituted aryl-C₁-C₄-alkyl, unsubstituted or substituted hetaryl-C₁-C₄-alkyl, unsubstituted or substituted aryl-C₂-C₄-alkenyl or unsubstituted or substituted hetaryl-C₂-C₄-alkenyl.

8. A compound I as claimed in claim 1, where
n is 1,
Z is O,
W is hydrogen, chlorine, methyl or methoxy,
Y is oxygen, sulfur or NR⁴,
R¹ is hydrogen or C₁-C₄-alkyl,
R² is hydrogen, C₁-C₄-alkyl or a group CO-V,
R³ is hydrogen,
unsubstituted or substituted C₁-C₄-alkyl, unsubstituted or substituted or fused aryl, unsubstituted or substituted or fused hetaryl, unsubstituted or substituted aryl-C₁-C₄-alkyl or unsubstituted or substituted hetaryl-C₁-C₄-alkyl, and
V is hydroxyl, C₁-C₄-alkoxy, allyloxy, trialkylsilyloxy or unsubstituted or substituted benzyloxy.

9. A compound I as claimed in claim 1, where
W is hydrogen,
n is 0,
Z is oxygen or NOR⁷,
R³ is hydrogen,
unsubstituted or substituted C₁-C₄-alkyl, unsubstituted or substituted C₂-C₄-alkenyl, unsubstituted or substituted or fused aryl or unsubstituted or substituted or fused hetaryl,
R⁷ is hydrogen,
unsubstituted or substituted C₁-C₄-alkyl, unsubstituted or substituted C₂-C₄-alkenyl, unsubstituted or substituted aryl-C₁-C₄-alkyl, unsubstituted or substituted hetaryl-C₁-C₄-alkyl or a group COR¹⁰, and
R¹⁰ is unsubstituted or substituted C₁-C₄-alkyl, unsubstituted or substituted aryl or unsubstituted or substituted hetaryl.

10. A compound I as claimed in claim 1, where
W is hydrogen
Z is oxygen,
n is 1,
Y is oxygen or NR⁴,
R³ is unsubstituted or substituted C₁-C₄-alkyl, unsubstituted or substituted or fused aryl, unsubstituted or substituted or fused hetaryl, unsubstituted or substituted aryl-C₁-C₄-alkyl or unsubstituted or substituted hetaryl-C₁-C₄-alkyl, and
R⁴ is hydrogen or C₁-C₄-alkyl.

11. A fungicide which contains an inert carrier and a fungicidal amount of a compound of the formula I as claimed in claim 1

12. A method for controlling fungi, wherein a fungicidal amount of a compound of the formula I as claimed in claim 1 is allowed to act on the fungi or plants, seeds materials or the soil threatened by fungal attack.

13. An insecticide which contains an inert carrier and an insecticidal amount of a compound of the formula I as claimed in claim 1.

14. An acaricide which contains an inert carrier and an acaricidal amount of a compound of the formula I as claimed in claim 1.

## Revendications

1. Composés de formule générale I, 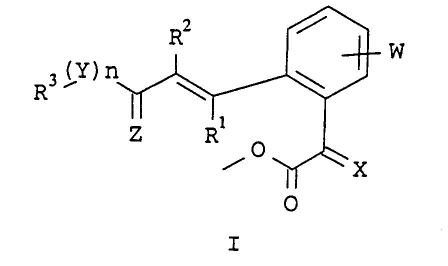 où l'indice et les substituants ont la signification suivante:
n 0 ou 1,
W hydrogène, halogène, groupe alkyle en C₁-C₄ éventuellement substitué ou groupe alcoxy en C₁-C₄ éventuellement substitué,
X CHOCH₃, NOCH₃, CHCH₃ ou CHC₂H₅,
Y oxygène, soufre, NR⁴, -O-NR⁴-, -NR⁴-O- ou -NR⁴-NR⁵-,
Z oxygène, NR⁶, NOR⁷ ou N-NR⁸R⁹, ainsi que pour n = 1 également le soufre,
R¹ hydrogène ou groupe alkyle en C₁-C₄ éventuellement substitué,
R² hydrogène,
groupe alkyle en C₁-C₄ éventuellement substitué, groupe cycloalkyle en C₃-C₆ éventuellement substitué, groupe aryle éventuellement substitué, groupe hétéroaryle éventuellement substitué, groupe perfluoroalkyle en C₁-C₄, ou CO-V,
R³ hydrogène,
groupe alkyle en C₁-C₄ éventuellement substitué, groupe alcényle en C₂-C₄ éventuellement substitué, groupe alcynyle en C₂-C₄ éventuellement substitué, groupe cycloalkyle en C₃-C₆ éventuellement substitué, groupe hétérocyclyle à 3 à 6 chaînons éventuellement substitué, groupe aryle éventuellement substitué et/ou condensé, groupe hétéroaryle éventuellement substitué et/ou condensé, groupe aryl-alkyle(C₁-C₄) éventuellement substitué, ou groupe hétéroarylalkyle(C₁-C₄) éventuellement substitué, ou arylalcényle(C₂-C₄) éventuellement substitué,
pour n = 0 également cyano, perfluoroalkyle en C₁-C₄ et
pour n = 1, dans le cas où Y désigne l'oxygène, un groupe trialkylsilyle éventuellement substitué,
R⁴, R⁵ et R⁹ hydrogène,
groupe alkyle en C₁-C₄ éventuellement substitué, ou groupe cycloalkyle en C₃-C₆ éventuellement substitué,
R⁶ groupe alkyle en C₁-C₄ éventuellement substitué, groupe aryle éventuellement substitué ou condensé ou groupe hétéroaryle éventuellement substitué ou condensé,
R⁷, R⁸ hydrogène,
groupe alkyle en C₁-C₄ éventuellement substitué, groupe alcényle en C₂-C₄ éventuellement substitué, groupe alcynyle en C₂-C₄ éventuellement substitué, groupe cycloalkyle en C₃-C₆ éventuellement substitué, groupe aryle éventuellement substitué ou condensé, groupe hétéroaryle éventuellement substitué et/ou condensé, groupe aryl-alkyle(C₁-C₄) éventuellement substitué, groupe hétéroaryl-alkyle (C₁-C₄) éventuellement substitué,
ou groupe COR¹⁰
R¹⁰ groupe alkyle en C₁-C₄ éventuellement substitué, groupe cycloalkyle en C₃-C₆ éventuellement substitué, groupe aryle éventuellement substitué, ou groupe hétéroaryle éventuellement substitué,
V groupe alkyle en C₁-C₄ éventuellement substitué, groupe aryle éventuellement substitué, groupe hétéroaryle éventuellement substitué, groupe hydroxyle, groupe alcoxy en C₁-C₄ éventuellement substitué, groupe allyloxy éventuellement substitué, groupe benzyloxy éventuellement substitué, ou groupe trialkylsilyloxy éventuellement substitué.

2. Composés I selon la revendication 1, caractérisés par le fait que X représente CHOCH₃.

3. Composés I selon la revendication 1, caractérisés par le fait que X représente NOCH₃.

4. Composés I selon la revendication 1, caractérisés par le fait que X représente CHCH₃ ou CHC₂H₅.

5. Composés I selon la revendication 1, caractérisés par le fait que n = 0 et Z représente O, NR⁶, NOR⁷ ou N-NR⁸R⁹.

6. Composés I selon la revendication 1, caractérisés par le fait que n = 1, Z représente l'oxygène et Y représente l'oxygène, le soufre, NR⁴ ou NR⁴NR⁵.

7. Composés I selon la revendication 1, où n = 0 et dans lesquels les substituants ont la signification suivante:
W hydrogène, chlore, groupe méthyle ou groupe méthoxy,
Z oxygène, NR⁶, NOR⁷ ou N-NR⁸R⁹,
R¹, R² hydrogène ou groupe alkyle en C₁-C₄,
R³ hydrogène,
groupe alkyle en C₁-C₄ éventuellement substitué, groupe alcényle en C₂-C₄ éventuellement substitué, groupe alcynyle en C₂-C₄ eventuellement substitué, groupe cycloalkyle en C₃-C₆ éventuellement substitué, groupe hétérocyclyle à 3 à 6 chaînons éventuellement substitué, groupe aryle éventuellement substitué ou condensé, groupe hétéroaryle éventuellement substitué ou condensé, groupe aryl-alkyle(C₁-C₄) éventuellement substitué, groupe hétéroaryl-alkyle(C₁-C₄) éventuellement substitué, groupe aryl-alcényle(C₂-C₄) éventuellement substitué ou groupe hétéroarylalcényle(C₂-C₄) éventuellement substitué.

8. Composés I selon la revendication 1, dans lesquels n vaut 1 et Z vaut 0 et dans lesquels les substituants ont la signification suivante:
W hydrogène, chlore, groupe méthyle ou groupe méthoxy,
Y oxygène, soufre ou groupe NR⁴,
R¹ hydrogène ou groupe alkyle en C₁-C₄,
R² hydrogène, groupe alkyle en C₁-C₄ ou groupe CO-V,
R³ hydrogène,
groupe alkyle en C₁-C₄ éventuellement substitué, groupe aryle éventuellement substitué ou condensé, groupe hétéroaryle éventuellement substitué ou condensé, groupe aryl-alkyle(C₁-C₄) éventuellement substitué, ou groupe hétéroaryl-alkyle(C₁-C₄) éventuellement substitué,
V groupe hydroxyle, groupe alcoxy en C₁-C₄, groupe allyloxy, groupe trialkylsilyloxy ou groupe benzyloxy éventuellement substitué.

9. Composés I selon la revendication 1, dans lesquels W représente l'hydrogène et n vaut 0 et dans lesquels les substituants ont la signification suivante:
Z oxygène ou NOR⁷,
R³ hydrogène,
groupe alkyle en C₁-C₄ éventuellement substitué, groupe alcényle en C₂-C₄ éventuellement substitué, groupe aryle éventuellement substitué ou condensé, ou groupe hétéroaryle éventuellement substitué ou condensé,
R⁷ hydrogène,
groupe alkyle en C₁-C₄ éventuellement substitué, groupe alcényle en C₂-C₄ éventuellement substitué, groupe aryl-alkyle(C₁-C₄) éventuellement substitué, groupe hétéroaryl-alkyle(C₁-C₄) éventuellement substitué ou groupe COR¹⁰,
R¹⁰ groupe alkyle en C₁-C₄ éventuellement substitué, groupe aryle éventuellement substitué, ou groupe hétéroaryle éventuellement substitué.

10. Composés I selon la revendication 1, dans lesquels W représente l'hydrogène, Z désigne l'oxygène et n vaut 1 et dans lesquels les substituants ont la signification suivante:
Y oxygène ou NR⁴,
R³ groupe alkyle en C₁-C₄ éventuellement substitué, groupe aryle éventuellement substitué ou condensé, groupe hétéroaryle éventuellement substitué ou condensé, groupe aryl-alkyle(C₁-C₄) éventuellement substitué, ou groupe hétéroaryl-alkyle(C₁-C₄) éventuellement substitué,
R⁴ hydrogène ou groupe alkyle en C₁-C₄.

11. Fongicide, caractérisé par le fait qu'il contient un support inerte et une quantité efficace du point de vue fongicide d'un composé de formule I selon la revendication 1.

12. Procédé pour lutter contre les champignons, caractérisé par le fait qu'on fait agir sur les champignons ou sur des plantes, des semences, des matériels ou le sol menacés par une attaque de champignons une quantité efficace du point de vue fongicide d'un composé de formule générale I selon la revendication 1.

13. Insecticide, caractérisé par le fait qu'il contient un support inerte et une quantité efficace du point de vue insecticide d'un composé de formule I selon la revendication 1.

14. Acaricide, caractérisé par le fait qu'il contient une quantité efficace du point de vus acaricide d'un composé de formule I selon la revendication 1.
